(19) Europäisches Patentamt
European Patent Office
Office européen des brevets

(11) EP 1 129 187 B1

(12) EUROPEAN PATENT SPECIFICATION

(45) Date of publication and mention of the grant of the patent:
**07.05.2008 Bulletin 2008/19**

(21) Application number: **99956260.6**

(22) Date of filing: **09.11.1999**

(51) Int Cl.:
*C12N 15/12* (2006.01) *C07K 14/47* (2006.01)
*C12N 1/19* (2006.01) *C12N 1/21* (2006.01)
*C12N 5/14* (2006.01) *C12N 5/16* (2006.01)
*C12P 21/02* (2006.01) *A61K 38/17* (2006.01)
*C12Q 1/68* (2006.01)

(86) International application number:
**PCT/IB1999/001886**

(87) International publication number:
**WO 2000/027871 (18.05.2000 Gazette 2000/20)**

(54) **NEW FAMILY OF MAMMALIAN POTASSIUM CHANNELS, THEIR CLONING AND THEIR USE, ESPECIALLY FOR THE SCREENING OF DRUGS**

NEUE FAMILIE VON SÄUGETIER-KALIUMKANÄLEN, IHRE KLONIERUNG UND IHRE VERWENDUNG, BESONDERS FÜR DAS DROGENSCREENING

NOUVELLE FAMILLE DE CANAUX POTASSIQUES DE MAMMIFERES, LEURS CLONAGE ET LEUR APPLICATION, SPECIALEMENT DANS LE CRIBLAGE DE MEDICAMENTS

(84) Designated Contracting States:
**AT BE CH CY DE DK ES FI FR GB GR IE IT LI LU MC NL PT SE**

(30) Priority: **09.11.1998 US 107692 P**
**08.11.1999 US 436265**

(43) Date of publication of application:
**05.09.2001 Bulletin 2001/36**

(73) Proprietor: **CENTRE NATIONAL DE LA RECHERCHE SCIENTIFIQUE (CNRS)**
**75794 Paris Cedex 16 (FR)**

(72) Inventors:
- **DUPRAT, Fabrice,**
**Centre Nat.de la Rech.Scient.**
**75794 Paris Cedex (FR)**
- **LESAGE, Florian**
**06000 Nice (FR)**
- **LAZDUNSKI, Michel**
**06000 Nice (FR)**

(74) Representative: **Breese, Pierre**
**BREDEMA**
**38, avenue de l'Opéra**
**75002 Paris (FR)**

(56) References cited:
**EP-A- 0 799 889** **WO-A-00/05367**

- **DUPRAT F. ET AL: "TASK, a *human* background K+ channel to sense external pH variations near physiological pH" EMBO JOURNAL(EMBO J.), 16/17 (5464-5471), 1997, XP000919218 United Kingdom**
- **REYES ROBERTO ET AL: "Cloning and expression of a novel pH-sensitive two pore domain K+ channel from human kidney." JOURNAL OF BIOLOGICAL CHEMISTRY, vol. 273, no. 47, 20 November 1998 (1998-11-20), pages 30863-30869, XP000919229 ISSN: 0021-9258**
- **LESAGE F ET AL: "TWIK-1, A UBIQUITOUS HUMAN WEAKLY INWARD RECTIFYING K+ CHANNEL WITH A NOVEL STRUCTURE" EMBO JOURNAL,GB,OXFORD UNIVERSITY PRESS, SURREY, vol. 15, no. 5, 1996, pages 1004-1011, XP002017569 ISSN: 0261-4189**
- **FINK M ET AL: "Cloning, functional expression an brain localization of a novel unconventional outward rectifier K+ channel" EMBO JOURNAL, GB,OXFORD UNIVERSITY PRESS, SURREY, vol. 15, no. 24, 16 December 1996 (1996-12-16), pages 6854-6862, XP002085602 ISSN: 0261-4189**
- **FINK M ET AL: "A neuronal two P domain K+ channel stimulated by arachidonic acid and polyunsaturated fatty acids" EMBO JOURNAL, GB,OXFORD UNIVERSITY PRESS, SURREY, vol. 17, no. 12, 15 June 1998 (1998-06-15), pages 3297-3308, XP002085600 ISSN: 0261-4189**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

• PATEL A J ET AL: "A mammalian two pore domain mechano-gated S-like K+ channel" EMBO JOURNAL,GB,OXFORD UNIVERSITY PRESS, SURREY, vol. 17, no. 15, 3 August 1998 (1998-08-03), pages 4283-4290, XP002085601 ISSN: 0261-4189

**Description**

## BACKGROUND OF THE INVENTION

Cross-Reference to Related Applications

This application claims the benefit of U.S. Serial No. 08/749,816, filed November 15, 1996 and entitled *New Family of Mammalian Potassium Channels. Their Cloning And Their Use, Especially For The Screening of Drugs,* U.S. Provisional Application Serial No. 60/095,234, filed August 4, 1998, entitled Task *a Human Background K+Channel to Sense External pH Variations Near Physiological pH,* U.S. patent serial no.09/144,914 entitled *Family of Mammalian Potassium Channels. Their Cloning and Their Use. Especially for the Screening of Drugs,* filed August 5, 1998 and provisional application serial no. 60/107,692, entitled *Cloning and Expression of a Novel Ph-sensitive Two Pore Domain K+channel from Human Kidney,* filed on November 9,1998. This application claims the benefit of the filing dates of these applications.

Field Of Invention

The present invention relates to a new family of potassium channels. More specifically, the invention relates to the cloning of new human potassium channels that constitute the members ofanew functional and structural group of potassium channels. The abundance of these channels and their presence in a large number of tissues are such as to confer on them a fundamental role in the transport of potassium in a large number of types of calls.

## SUMMARY OF THE INVENTION

Potassium channels are ubiquitous in eukaryote and prokaryote cells. Their exceptional functional diversity make them ideal candidates for a large number of biological processes in living cells (Rudy, B., 1988, Neurosciences 25, 729-749; Hille, B., 1992, "Ionic Channels of Excitable Membrane", 2nd edition, Sinauer, Sunderland, Massachusetts). In excitable cells, the K+ channels define the form of the action potentials and the frequency of the electric activity, and play a major role in neuronal integration, muscle contraction or hormonal secretion. In nonexcitable cells, their expression appears to be correlated with specific stages of the development of the cell (Barrel, B. A. et al., 1990, Annul Rev. Neurosci., 13, 441-474). In most cells, specific types of K+ channels play a vital role in determining the electrical potential of the membrane at rest by regulating the membrane permeability to K+ ions. These channels exhibit the characteristic of being instantaneous and open in a large range of membrane potentials.

Recent cloning studies have resulted in the identification of a large number of subunits capable of forming potassium channels (Betz, H., 1990, Biochemistry, 29, 3591-3599; Pongs, 0., 1992, Physiol. Rev., 72, S69 88; Sallcoff, L. et al., 1992, Trends Neurosci., 15, 161-166; Jan. L. Y. and Y. N. Jan, 1994, Nature, 371, 199-122; Doupnik. C. A. et al., 1995, Curr. Opin. Neurobiol., 5, 268-277) which could be regulated by other types of subunits (Aldrich, R. W., 1994, Curr. Biol., 4, 839-840; Isom, L. L. et al., 1994, Neuron, 12,1183-1194; Rettig, J. et al., 1994, Nature, 369, 289-294; Attali, B. at al., 1995. Proc. Nafl. Acad. Sci. USA, 92.6092- 6096).

The subunits of the voltage-dependent K+ channels activated by depolarization (Kv families) and the calcium-dependent K+ channels exhibit six hydrophobic transmembranal domains, one of which (S4) contains repeated positive charges which confer on these channels their sensitivity to voltage and, consequently, in their functional outward rectification (Logothetis, D. E. et al., 1992, Neuron, 8,531- 540; Bezanilla, F. and Stefani, E., 1994, Annul Rev. Biophys. Biomol. Struct., 23, 819- 846).

The K+ channels with inward rectification (Kir families) have only two transmembranal domains. They do not have the S4 segment and the inward rectification results from a voltage-dependent blockade by cytoplasmic magnesium (Matsuda, H., 1991, Annul Rev. Physiol., 53, 289-298; Lu, Z. and Mackinnon, R., 1994, Nature, 371, 243 246; Nichols, C. G. et al., 1994, J. Physiol. London, 476, 399- 409).

A common structural unit, called the P domain, is found in both groups, and constitutes an essential element of the structure of the K+-permeable porc. The presence of this unit in a membrane protein is considered to be the signature of the structure of a K+ channel (Pongs, O., 1993,J.Membrane Biol., 136, 1-8; Heginbotham, L. et al., 1994, Biophys. J., 66, 1061-1067; Mackinnon, R., 1995, Neuron, 14, 889-892; Pascual, J. M. et al., 1995, Neuron, 14, 1055-1063).

Duprat, F et al. ("TASK, a human background K+ channel to sense external pH variation near physiological pH" EMBO JOURNAL, 16/17 (5464-5471), 1997, XP000919218 United Kingdom) described an isolated nucleic acid encoding a mammalian (human) protein (TASK) which is competent to transport potassium across a membrane, said transport being regulated by external pH, which protein is highly expressed in kidney and comprises two P domains and four transmembrane segments.

Lesage F et al. ("TWIK-1, a ubiquitous human weakly inward rectifying K+ channel with a novel structure" EMBO JOURNAL, GB, OXFORD UNIVERSITY PRESS, SURREY, vol. 15, no. 5, 1996, pages 1004-1011, XP002017569 ISSN: 0261-4189) and Fink M et al. ("Cloning, functional expression and brain localization of a novel unconventional outward rectifier K+ channel" EMBO JOURNAL, GB, OXFORD UNIVERSITY PRESS, SURREY, vol. 15, no. 24, 16 December 1996 (1996-12-16), pages 6854-6862, XP002085602 ISSN: 0261-4189) describe nucleic acids encoding mammalian proteins (TWIK-1 and TREK-1) which are competent to transport potassium across a membrane, said transport being regulated by external pH, which proteins are expressed in most tissue (including kidney) and which comprise two P domains and four transmembrane segments.

PCT patent application WO00/05367 discloses human proteins having hydrophobic domains, DNAs coding for these proteins, and expression vectors for these DNAs as well as eucaryotic cells expressing these DNAs.

The present invention is defined in the claims. It relates to the use of an isolated nucleic acid encoding a mammalian protein which is competent to transport potassium across a membrane, said transport being regulated by external pH, which protein is highly expressed in kidney and comprises two P domains and four transmembrane segments, said protein having the amino acid sequence represented on figure 14a, for the manufacture of a composition for diagnosing or for treating hypertension or dysfunctions of the kidney, liver or pancreas.

The present invention is based on the cloning of K+ channels which are the first members of a new structural and functional group of potassium channels. These new K+ channels have a novel molecular architecture with four trans-membranal segments and two P domains. From a functional point of view, these channels are remarkable in that they exhibit different electrophysical properties. For example, one of these exhibits weak inward rectification properties. This new channel is referred to below as TWIK-1 following the English-language term "Tandem of P domains in a Weak Inward rectifying K+ channel". Its abundance and its presence in a large number of tissues are such as to confer on it a fundamental role in the transport of potassium in a large number of types of cells. In contrast, TASK1 and TASK2 (TWIK-related Acid-Sensitive K+ channel) exhibit an outward rectification under low concentrations of potassium.

The discovery of this new family of potassium channels and the cloning ofnew members of this family provides, notably, new means for screening drugs capable of modulating the activity of these new potassium channels and thus of preventing or treating the diseases in which these channels are involved.

The research activities that led to the cloning of the TWIK-1. TASK1 and TASK2 channels was carried out in the manner described below with reference to the attached sequences and drawings in which:

- SEQ ID NO: 1 represents the nucleotide sequence of the cDNA of TWIK1 and its deduced amino acid sequence.
- SEQ ID NO: 2 represents the amino acid sequence of the TWIK- protein.
- SEQ ID NO: 3 represents the nucleotide sequence of the cDNA of TASK1 and its deduced amino acid sequence.
- SEQ ID NO: 4 represents the amino acid sequence of the TASK1 protein.
- SEQ ID NO: 5 represents the nucleotide sequence of the cDNA of TASK2 and its deduced amino acid sequence.
- SEQ ID NO: 6 represents the amino acid sequence of the TASK2 protein.

**DETAILED DESCRIPTION OF THE DRAWINGS**

**Figure 1** represents the Northern blot analysis, the nucleotide sequences and the deduced amino acid sequence, as well as the hydrophobicity profile of TWIK-1 (SEQ ID No. 1). (a): expression of TWIK-1 mRNA in human tissues; each track contains 5 μg of poly(A)+ RNA; the autoradiograph was exposed for 24 hours. (b) cDNA sequence of TWIK-1 and the amino acid sequences of the coding sequence. The supposed transmembranal segments are circled and the P domains are underlined; □ represents a potential glycosylation site and ■ represents the threonine residue in the consensus recognition site of protein kinase C. (c): the hydrophobicity analysis and the topology of TWIK-1 deduced from it; the hydrophobicity values were calculated according to the method of Kyte and Doolittle (window size of 11 amino acids) and are presented in relation to the position of the amino acid; the shaded hydrophobic peaks correspond to the transmembranal segments.

**Figure 2** represents the sequence alignments. (a): alignment of the P domains of TWIK-1, TOC/YORK and other representative K+ channel families (SEQ ID No. 9 through SEQ ID No.23); the identical and conserved residues are circled in black and in gray, respectively. (b): alignment of TWIK-1 (SEQ ID No.2) with potential homologs of;

the sequences M110.2 (SEQ ID No. 7) and F17C8.5 (SEQ ID No. 6) were deduced from the gene sequences (respective access numbers Z49968 and Z35719); the computerized splicing of the other genomic sequences of *C. elegans* (respective access numbers Z49889, P34411 and Z22180) is not sufficiently precise to allow their perfect alignment and is therefore not shown.

**Figure 3** shows the biophysical and pharmacological properties of K+ currents recorded by the imposed voltage technique on *Xenopus* oocytes that had received an injection of TWIK-1 cRNA; (a): the oocyte was maintained at a holding potential (HP) of -80 mV and the currents were recorded at the end of 1-s voltage jumps from -120 to +60 mV in 20 mV increments. (b): regular current-voltage relationship using the same technique as in (a). (c): potential reversal of the TWIK-1 1 currents ($E_{rew}$) as a function of the external K+ concentration. (d) current tracings linked to +30 mV depolarizations starting at a holding potential (HP) of -80 mV in the absence (top tracing) and in the presence (bottom tracing) of 1 mM of $Ba^{2+}$, (e): blocking effect of 100 $\mu$M of quinine, same protocol as in (d). (f) dose-response relationship of the blocking of the TWIK-1 currents by quinine.

**Figure 4** shows the influence of the expression of TW1K-1 on the membrane potential. (a): dose-response relationships of the cRNA; top row = equilibrium state of the outward currents measured at +30 mV; bottom row = membrane potentials associated with the resting state. (b): effect of 100 $\mu$M of quinine on the membrane potential of an oocyte which did not receive an injection (left tracing) and that of an oocyte that received 20 ng of TWIK-1 cRNA. (c): statistical evaluation of the depolarizing effects of 100 $\mu$M of quinine on oocytes that did not receive injections (left bars) and on oocytes that received injections of 20 ng of TWIK-1 cRNA (right bars); control (unfilled bar), + quinine (solid bars); each bar represents the mean +/- SD of 5 oocytes.

**Figure 5** shows the properties of the single TWIK-1 channel, (a): current tracings recording in the input-output configuration to the membrane potentials indicated in the absence (m) or in the presence (.) of internal $M^{2+}$ (3 mM) and in symmetry with 140 mM of K+. (b): mean of curves I-V (n =10). (c and d): open time of distribution obtained at +80 mV (top histograms) and at -80 mV (bottom histograms) in the presence of 3 mM $Mg^{2+}$ (c) or in the absence of $Mg^{2+}$ (d).

**Figure 6** shows the blocking of the TWIK-1 channels by the internal pH. (a and b): blocking effect of the internal acidification on the TWIK-1 currents, induced by perfusion of $CO_2$; (a) tracings of superimposed currents induced by a depolarization phase at -30 mV starting at HP = -80 mV, control (top tracing), effect when equilibrium is reached in the presence of $CO_2$ (bottom tracing); (b): graph (n = 5) showing the almost complete blockade of the TWIK=1 currents induced by CO2; (c and d): internal acidification induced by the application of DNP (1 mM). (c): same protocol as in (a), control (top tracing) and after 5 minutes of application ofDNP (bottom tracing); (d): graph (n = 4) indicating the percentage of TWIK-1 current remaining after treatment with DNP. (e and f): imposed voltage (method: attached patch) under symmetrical conditions of K+ concentration (140 mM) maintained at +80 mV. (e) course over time of the effect of 1 niM ofDNP (marked with arrow) on the activities of the single TWIK-1 channel. (f): graph (n = 4) showing the effect of DNP on the mean probability of opening $NP_o$ calculated during 1 minute of recording starting at the equilibrium state. (g): activities measured in the "inside-out patch" state at 80 mV at different internal pH values. Bar graph (n =10) of $NP_o$ in relation to the internal pH.

**Figure 7** shows the activation of the TWIK-1 channels by PMA, activator of protein kinase C. (a): perfusion of PMA (30 nM) for 10 minutes increases the TWIK-1 current (top tracing) induced by a depolarization phase at +30 mV starting at HP = -80 mV, control current (top tracing). (b): graph (n = 5) showing the activation effect ofPMA on the TWIK-1 currents, (c and d): attached patch configuration under symmetrical K+ concentration conditions maintained at +50 mV; (c): course over time of the effect of 30 nM ofPMA on the single channel activities; the recordings of the channel activity were performed with a rapid scanning before and after the application of PMA; (d): bar graph (n = 5) showing the activation effect of PMA on $NP_o$.

**Figure 8** shows the nucleotide and deduced amino acid sequences of human TASK1 (SEQ ID No. 3) and partial amino acid sequence of mouse TASK1 (SEQ ID No. 5). Consensus sites for N-linked glycosylation (*) and phosphorylation by protein kinase C (n), protein kinase A (s) and tyrosine kinase (1) in human TASK1. These sites have been identified by using the prosite server (European Bioinformatics Institute) with the ppsearch software (EMBL Data library) based on the MacPattern program. The sequence of human and mouse TASK1 have been deposited in the GenBank/EMBL database under the accession numbers AF006823 and AF006824, respectively.

**Figure 9** shows the sequence comparison and membrane topology of TWIK-related channels. A: Alignment of human TWIK-1 (SEQ ID No. 2), mouse TREK-1 (SEQ ID No. 8) and human TASK1 (SEQ ID No.4) sequences.

Identical and conserved residues are shown in black and grey, respectively. Dashes indicate gaps introduced for a better alignment. Relative positions of putative transmembrane segments (M1 to M4) and P domains (P1 and P2) of human TASK are also indicated. The M1-M4 domains were deduced from a hydropathy profile computed with a window size of 11 amino acids according to the Kyte and Doolittle method (Kyte and Doolittle, 1982). B: Putative membrane topology of TWIK-1, TREK-1 and TASK1 channels.

**Figure 10** shows the northern blot analysis of TASK1 distribution in adult human tissues. Human multiple tissues Northern blots from Clontech were probed at high stringency with a TASK1 cDNA probe. Each lane contains 2 μg of poly(A)$^+$RNA. Autoradiograms were exposed for 48 h at -70 °C. The blots were re-probed with a β-actin cDNA probe for control. *sk muscle:* skeletal muscle, *sm. intestine:* small intestine, *PBL:* peripheral blood leukocytes.

**Figure 11** shows the distribution of TASK1 mRNA in adult mouse. A: Northern blot analysis. Each lane contains 2 μg of poly(A)$^+$RNA. Autoradiograms were exposed for 72 h at -70 °C. The blots were re-probed with a B-actin cDNA probe for control. B, C, D:*In situ* hybridization analysis from a coronal section at the level of the forebrain (B), the cerebellum (C), and the heart (D). Warmer colors represent higher levels of expression. *CA1-CA3:* fields CA1-3 of Ammon's horn, *Cx:* cerebral cortex, DG: dentate gyrus, *Gl:* granular layer, *Hb:* habenula, *SN:* substantia nigra, *PLCo:* postero lateral cortical amygdaloid nuclei, *PVP:* paraventricular thalamic nucleus, *A:* atrium, *V.* ventricule.

**Figure 12** shows the biophysical properties of TASK1 in *Xenopus* oocytes and COS cells. A: TASK1 currents recorded from a *Xenopus* oocyte injected with TASK cRNA and elicited by voltage pulses from -150 mV to +50 mV in 40 mV steps, 500 ms in duration, from a holding potential of -80 mV in low (2 mM $K^+$) or high solutions (98 mM $K^+$). The zero current level is indicated by an arrow. B: Current voltage relationships. Mean currents were measured over the last 50 ms at the end of voltage pulses from -150 to +50 mV in 10 mV steps as in A. Modified ND96 solutions containing 2 mM $K^+$ and 96 mM TMA were used, TMA was then substituted by $K^+$ to obtain solutions ranging from 2 mM to 98 mM $K^+$. TASK1 currents are not sensitive to external TMA, no changes were observed upon substitution of NaCl by TMA (data not shown). C: Upper panel: reversal potentials of TASK1 currents as a function of external $K^+$ concentration (mean ±SEM, n = 3). Lower panel: slope conductance measured between +10 and +50 mV on current-voltage relations as in A, plotted as a function of the external $K^+$ concentration (mean ±SEM, n = 3). The mean values were fitted with an hyperbola function. D: Theoretical current-voltage relation in the same conditions as in A calculated according to the following modified Goldman-Hodgkin-Katz (GHK) current relation:

$$I_{K+} = P_{K-} \frac{[K^+]_{out}}{K_{0.5} + [K^+]_{out}} \cdot \frac{V_m F^2}{RT} \cdot \frac{[K^+]_{in} - [K^+]_{out}\, e^{-VF/RT}}{1 - e^{-VF/RT}}$$

where is the potassium current, is the apparent permeability for $K^+$,$K_{0.5}$ the half maximum activation by $K^+$, $[K^+]_{out}$ and $[K^+]_{in}$ are the external and internal $K^+$ concentrations, $V_m$ the membrane potential, F, R and T have their usual meanings. The classical GHK relation has been modified with to take into account the sensitivity of the conductance to external $K^+$. E: TASK1 currents recorded from a transfected COS cell and elicited by voltage pulses from . -150 mV to +50 mV in 40 mV steps, 500 ms in duration, from a holding potential of-80 mV, in low (5 mM $K^+$) or high $K^+$ solutions (155 mM $K^+$): The zero current level is indicated by an arrow. F: Current-voltage relationships. Mean currents were measured over the last 50 ms at the end of voltage pulses ranging from-150 to +50 mV in 10 mV steps as in E. Solutions containing 5 mM $K^+$ and 150 mM TMA were used, TMA was then substituted by $K^+$ to obtain solutions ranging from 5 mM to 155 mM $K^+$.

**Figure 13** shows the pH dependent regulation of TASK1 in *Xenopus* oocytes and COS cells. A: Current-voltage relationships recorded from a TASKI-expressing oocyte with a ramp ranging from -150 mV to +50 mV, 500 ms in duration, from a holding potential of-80 mV, in ND96 solution at pH 6.5, 7.4 or 8.4. Inset; Currents elicited by voltage pulses to +50 mV, 500 ms in duration, in the same conditions as above. The zero current level is indicated by an arrow. B: pH-dependence of TASK1 activity in *Xenopus* oocyte recorded at -50, 0 and +50 mV (mean ±SEM, n = 3) as in A. Data were fitted with a Boltzman relation. C: Current-voltage relation recorded from a TASKI-expressing COS cell with a ramp ranging from -150 mV to +50 mV, 500 ms in duration, from a holding potential of -80 mV, in 5 mM $K^+$ solution at pH 6.1, 7.4 and 8.4. Inset: Currents elicited by voltage pulses to +50 mV, 500 ms in duration, in the same conditions as above. The zero current level is indicated by an arrow. D: pH-dependence of TASK1 activity recorded in COS cell at -50,0 and +50 mV (mean ±SEM, n = 3) as in C. Data were fitted with a Boltzman relation.

**Figure 14** shows the comparison of the TASK2 sequence with the cloned TWIK-related potassium channels. A) Nucleotide and deduced amino acid sequences of TASK2. The four potential transmembrane segments are boxed and the two P domains are underlined. A consensus site for N-linked glycosylation (*) in indicated. B) Dendrogram of the five 2P potassium channels cloned from mammals.

**Figure 15** shows the tissue distribution of TASK2 in human and mouse. A) multiple tissue Northen (MTN) blots were probed at high stringency with a TASK2 cDNA probe and reprobed with a β-actin probe as a control. sk, skeletal muscle; sm intestine, small intestine; PBL, peripheral blod lymphocytes. B) RT-PCR analysis from mouse tissues. The amplified products were analyzed by Southern blot using a specific TASK2 probe. To check the integrity of the cDNAs, a GAPDH fragment was amplified and separated by electrophoresis before staining with ethidium bromide.

**Figure 16** shows the TASK2 mRNA distribution in human adult kidney. *In situ* hybridization was performed with antisense (A) and sense (B) probes. Specific signal (A was observed over distal tubules and collecting ducts (indicated by stars in the tubular lumen), while a low non-specific labeling was apparent over the glomerulus (G) and proximal tubule (PT). Sense probe (B) gave a unifor signal. Magnification: X520.

**Figure 17** shows the chromosomal mapping of the TASK2 gene. Idiogram of human G-banded chromosome 6p and localization of the TASK2 gene relative to markers mapped in the Genebridge 4 Radiation Hybrid DNA panel from Research Genetics.

**Figure 18** shows the expression of TASK2 in COS cells and *Xenopus* oocytes. A) TASK2 whole cell currents recorded in transfected COS cells in 5mM or 155mM external K+, during voltage pulses ranging from -150 to +50 in 50mV steps. The holding potential is -80 mV, the dotted lines indicate the zero current level. B) current voltage relationship recorded as in A, with voltage ramps ranging from -150 to +50 mV, 500 ms in duration. C) relationship between the reversal potential measured in COS cells and the external K+ concentration, data (mean ±SEM, n = 4) are shown with the linear regression. D) current voltage relationship recorded in a TASK2 expressing oocyte in 2mM external K+, with voltage ramps ranging from -150 to +50 mV, 500 ms in duration. Inset: currents recorded in 2mM external K+ during voltage pulses ranging from -150 to +50 in 50mV steps. The holding potential is -80 mV. E) single channel currents recorded in transfected COS cells in outside-out patch at various potentials ranging from -80mV to +80mV, in 40mV steps. The dotted lines indicate the zero current level. F) single channel current potential relationships recorded as in (E) in 5mM (n =22) and in 155mM (n=19) exte4rnal K+.

Figure 19 shows the sensitivity of TASK2 currents to external pH. A) current voltage relationships deduced from currents elicited by voltage pulses ranging from -150 to +50 in 50mV steps, 500 ms duration starting from a holding potential of -80mV, measured at three different external pH (6.0, 7.4, and 8.6). B) whole cell currents elicited as in (A). C) relationship between the currents measured at -50, 0, and +50mV and the external pH. Data (mean± SEM) were fitted with a Bolzmann relation (pHm = 7.8 ± 0.1, n = 17, at 50mV). D) effect of pH6.5, 7.3, and 9.1 on N.Po calculated from the mean single channel currents recorded in the outside-out patches at 0mV during 30 s and from slope conductance between -20 and +20mV (n=5). F) effect of pH 6.5, 7.3 and 9.1 on single channel currents recorded at 0mV (n=13).

## Cloning of TWIK-1.

The P domains of K+ channels were used to determine the corresponding sequences in the GenBank data base by means of the BLAST sequence alignment program (Altschul, S. F. et al., 1990, J. Mol. Biol., 215,403-410). There was thus identified a 298 bp human Tag expressed sequence (EST, HSC3AH031), the deduced amino acid sequence of which includes a nonconventional "P-like" domain sequence: GLG in place of GrYG as shown in Figure 2a. It was then envisaged that this EST sequence was a partial copy of a mRNA coding a new type ofK+ channel subunit. A DNA probe was prepared from this sequence in order to carry out hybridization with a Northern blot (Clontech) of multiple human tissues. A 1.9 kb transcript was thereby found in abundance, as shown in Figure 1a, in the heart and the brain and, at lower levels, in the placenta, the lung, the liver and the kidney. The DNA probe was used to screen a bank of kidney cDNA and four independent clones were obtained. The cDNA inserts of 1.8 to 1.9 kb of these clones all have the same open reading frame (ORE) containing a region identical to the 298 bp sequence of HSC3A.H031 and differing solely in the length of their noncoding 5' sequences.

The TWIK-1 coding sequence was amplified using a low-error rate DNA polymerase (Pwo DNA pal, Boehringer) and subcloned in the plasmid pEXO so as to yield pEXO-TWIK-1. Mutations were performed using the whole plasmid pEXO-TWIK-1 with a highly reliable PCR extension kit (Boehringer) and two adjacent primers. One of these introduced a punctiform mutation in the IINIK-1 coding sequence, changing the 161 Thr codon into a codon for alanine. The product of the PCR was linearized by the enzyme BamHI and the cRNA were synthesized using a T7 RNA polymerase (Stratagene). Preparation of the X. larvis oocytes and cRNA injection were carried out in accordance with the literature (Guillemare, E. et al., 1992, Biochemistry. 31, 1246312468,

## Primary Structure of TWIK-1

The following characteristics were demonstrated:

- The sequences of the cDNA clones contain an ORF of 1011 nucleotides (SEQ ID No. 1) coding for a polypeptide of 336 amino acids (SEQ ID No. 2) shown in Figure 1b.
- This protein has two P domains.
- Other than the P domains, no significant alignment was seen between TWIK-1 and a K+ channel recently cloned in yeast and which also has two P domains (Ketchum, K. A. et al., 1995, Nature, 376, 690-695).
- Analysis of the hydrophobicity of TWIK-1, shown in Figure 1c, reveals the presence of four transmembranal domains, designated T1 to T4
- By placing the NH2 end on the cytoplasmic surface, in accordance with the absence of signal peptide, one obtains the topology model shown in Figure 1c.
- In this model, the two P domains are inserted in the membrane from the exterior in accordance with the known orientation of these loops in the K+ channels.
- In addition, the general structural unit of TWIK-1 is similar to the unit that one would obtain by making a tandem of two classical subunits rectifying the entry of a potassium channel. Like a classical inward rectifier, TWIK-1 does not exhibit the highly conserved segment S4 which is responsible for the sensitivity to the membrane potential of the inward rectification of the K+ channels of the Kv family.
- A unusual large loop of 59 amino acids is present between M1 and P1, such as to extend the length of the linker M1-P1 of the extracellular side of the membrane.
- A potential site of N-glycosylation is present in this loop.
- Three consensus sites of phosphorylation are present at the N-terminal (Ser 19 for calcium calmodulin kinase II) and C-terminal (Ser 303 for casein kinase II) ends of the cytoplasmic domains, and in the M2-M3 linker (Thrl61 for protein kinase II).
- The alignment of the P domains of an important group of K+ channels is presented in Figure 2a. It shows that the regions constituting the pore selective for K+ are well conserved including the G residues in position 16 and 18 and three other residues indicating practically exclusively conservative changes in positions 7,14 and 17, It is of interest to note that a leucine residue is present in the place of a tyrosine conserved in position 18 in the P2 domain of TWIK-1, or of a phenylalanine in position 17 of the P domain of the K+ channel of type eag.

## Functional Expression of Twik-1

For the functional study, the coding sequence of TWIK-1 was inserted between the noncoding sequences 5' and 3' of *Xenopus* globin in the vector pEXO (Lingueglia, E. et al., 1993, J. Biol. Chem., 269, 13736-13739). A complementary RNA (cRNA) was transcribed of this construction and injected in the oocytes of *X laevis.* In a 0.3ml perfusion chamber, a single oocyte was impaled on two standard glass microelectrodes (0.5 - 2.0 MW) charged with 3 M KCI and maintained under voltage-clamp with a Dagan TEV200 amplifier. The bath solution contained 98 mM KCl,1.8 raM $CaCl_2$ mM $MgCl_2$ and 5 mM HEPES at pH 7.4 with KOH. Stimulation of the preparation, data acquisition and analyses were carried out with the pClamp program (Axon Instruments) USA3.

For the patch-clamp experiments, the vitelline membrane was removed from the oocytes as described in the literature (Duprat, F. et al., 1995, Biochem. Biophys. Res. Commun., 212, 657-663); the oocytes were then placed in a bath solution containing 140 mM KCl, 1.8 mM $CaCl_2$, 2 mM $MgCl_2$ and 5 mM HEPES at pH 7.4 with KOH. The pipettes were filled with a strong K+ solution (40 mM KCl, 100 mM of potassium methane sulfonate, 1.8 mM $CaCl_2$, 2 m M $MgCl_2$ and 5 mM HEPES adjusted to pH 7.4 with KOH). 100 μM of $GdCl_3$ was added to the pipette solution to inhibit the action of the activated channel. The inside-out patches were perfused with a solution containing 140 mM KC1,10 mM $CaCl_2$, 5 mM HEPES adjusted to pH 7.2 with KOH and 5 m M EGTA added daily. The single channel signals were filtered at 3.5 kHz and analyzed with the Biopatch program (Big-Logic, Grenoble, France).

A noninactivating current, free from noninjected cells, was measured by the imposed voltage technique, as shown in Figure 3a. Kinetic activation of the current is usually instantaneous and cannot be resolved because it is masked by the capacitive discharge of the current recorded at the beginning of the impulse. The current-voltage relationship is linear above 0 mV and then saturates for a stronger depolarization of the membrane, as shown in Figure 3b. TWIK-1 is therefore K+ selective. In the case of a replacement of the external K+ by Na+ or N-methyl-D-gluconate, the reversal of the potential of the currents follows the K+ equilibrium potential (EK), as shown in Figure 3c. In addition, a change by 10 in the concentration $[(K)]_o$ leads to a change of 56 +/- 2 mV in the inversion value of the potential, in accordance with Nernst's equation.

As shown in Figure 3, the K+ currents of TWIK-1 are inhibited by Ba $^{2+}$ (Figure 3d) with an $IC_{50}$ value of 100 μM, by quinine (Figure 3e and 3f) and by quinidine (not shown) with respective $IC_{50}$ values of 50 and 95 μM. The TWIK-1 currents are slightly sensitive to TEA and to the class III antiarrhythmic agent tedisamil (30% inhibition for each, at 20 mM and 100 μM, respectively). Less than 10% inhibition was seen after application of 4-aminopyridine (1 mM), apamin (0.3 μM), charybdotoxine (3 nM), dedrotoxine (0.1 μM), clofilium (30 μM), amiodarone (100 μM) and glibenclamide (30 μM). The TW1TC-1 channel is not sensitive to the Kit channel openers cromakaline (140 μM) and pinacidil (100 μM).

Figure 4 shows the effect of increasing the doses of injected TWIK-1 cRNA on the independent expression of the time of the K+ currents and on the resting state of the membrane potential ($E_m$). As soon as the current appears, the oocytes become increasingly polarized, reaching a value of $E_m$ close to $E_K$. The amplitude of the TWIK 1 current reaches values of 0.6 to 0.8 μM with the injection of 20 ng per oocyte. Higher doses of TWIK1 cRNA are toxic, leading to a reduction in expression. In oocytes that received 20 ng of cRNA, quinine is the best blocker of TWIK-1, inducing a noteworthy reversible depolarization (73 +/- 6 mV, n = 5) as shown in figures 4b and 4c.

**The Unitary Properties of the Twik-1 Channel**

Single channel current recordings, shown in Figure 5, in an inside-out patch configuration or in a whole cell configuration show that the TWIK-1 channels assure the passage of influx or exit currents as a function, respectively, of a depolarization or a hyperpolarization (Figure 5a). The current voltage relationship of the single channel, shown in Figure 5 b, shows a barely accentuated inward rectification in the presence of 3 mM (Figure 5) and 10 mM (not shown) of $Mg^{2+}$ on the cytoplasmic side. As shown in Figure 5b, this rectification disappears in the absence of internal $M^{2+}$. With 3 mM of internal $Mg^{2+}$, the mean duration of opening at +80 mV is 1.9 ms and the unitary conductance is 19 +/- 1 pS (Figure 5c). At -80 mV, the channels are oscillating with a mean duration of opening of 0.3 ms, and a conductance value in creasing to 34 pS. The withdrawal of the internal $Mg^{2+}$ ions does not influence the kinetic parameters under either polarized or depolarized conditions, but the unitary conductance measured at-80 mV reaches 35'+/- 4 pS. This apparent increase in conductance in the single channel suggests that it is the extremely rapid oscillation induced by Mg $^{2+}$ that results in an underestimation of the real value of conductance. The same properties were observed in the fixed cell configuration, showing that the channel behavior is not modified by the excision of the patch. The TWIK-1 channels in the excised patches do not discharge and do not appear to be deficient in intracellular constituents. In contrast to numerous channels which require the presence of ATP for their activity in the excised patch configuration, ATP is not required for the expression of TWIK-1. In addition, perfusion of the patch with a solution containing 10 mM of ATP does not induce any effect on the activity of the TWIK-1 channel.

**Regulation of the TWIK-1 channel.**

The intracellular pH ($pH_i$) is involved in the control of numerous cellular processes, and in cells such as the hepatic cells, the change in $pH_i$ regulates the membrane potential (Bear, C. E. et al., 1988, Biochim. Biophys. Acta, 944,113-120).

Intracellular acidification of the oocytes was produced using two methods:

- superfusion with a solution enriched in $CO_2$ which produces acidification by a mechanism involving the bicarbonate transport system (Guillemare, E. et al., 1995, Mol. Pharmacol., 47, 588-594);

- treatment with dinitrophenol (DNP), which is a metabolic inhibitor that decouples the H+ gradient in mitochondria and induces internal acidity (Pedersen, P. L. and Carafoli, E., 1997, Trends Biol. Sci., 12, 146-189).

Both of these experimental methods resulted in a significant reduction in the TWIK-1 currents, greater than 95% in the case of $CO_2$ and 80% in the case of DNP of the control amplitude values, as shown in Figures 6a to 6d. The inhibition induced by DNP on the activity of the single K+ channel was again observed under the attached patch conditions, as shown in Figures 6e to 6f. However, after excision of the patch, the activity of the channel became insensitive to the acidification of the internal solution produced either by modifying the $Na_2HPO_4/NaH_2PO_4$ buffer ratio (Figures 6g and 6h3 or by bubbling of $CO_2$ (not shown). Thus, the effect of the pH value on the activity of the TWIK-1 channel is probably indirect.

Phosphorylation or dephosphorylation of specific amino acid residues is an important mechanism of regulation of the ionic channels (Levitan, I. B., 1994, Annul Rev. Physiol., 56, 193-212). As shown in Figure 7, activation of protein kinase C by phorbol-12 myristate acetate (PMA, 30 nM) increases the TWIK-1 currents. The inactive phorbol ester 4α-phorbol-12, 13 didecanoate (PDA, 1μM) has no effect. In an attached patch which initially expressed solely a single channel, application ofPMA showed the presence of at least five channels (Figure 7c and 7d). This experiment shows that at least four channels are silently present in the patch before the application of PMA. Since the TWBC-1 sequence contains a consensus phosphorylation site for protein kinase C (PKC), located at the level of the threonine in position 161 (Figure 1b), the effect of PMA suggests regulation under the control of PKC. However, the mutation of the threonine 161 into alanine leads to a muted channel which remains functional and conserves the capacity to be activated by PMA.

Activation of protein kinase A by application of 8-Cl-AMPc (300 μM) or forskolin (10μM) does not affect the activity of TWIK-1. Elevation of the cytoplasmic $Ca^{2-}$ concentration by application of A23187 (1μM) which could be activated by Ca2+-calrnodulin kinase nand/or reveal the presence of a channel activated by the $Ca^{2+}$, is also without effect on the properties of the TWIK-1 channel.

## TASK1, a homolog of TWIK-1

TWIK- and TREK-1 sequences were used to search for homologs in gene databases by using the tBlastn sequence alignment program (Altschul *et al.,*1990). Translation of two overlapping EST sequences (GenBank accession numbers W36852 and W36914) in one frame exhibited significant sequence similarities with both TWIK-1 and TREK-1. A 560 bp DNA fragment was amplified by PCR from mouse brain poly(A)$^+$ cDNAs and subcloned into pBluescript (Stratagene) to give pBS-852/914. This fragment was $^{32}$P-labelled and used to screen mouse brain and heart cDNA libraries. Filters were hybridized and washed as previously described (Fink *et al.,* 1996b). Eight positive clones from brain and ten from heart were obtained. cDNA inserts were characterized by restriction analysis and by partial or complete sequencing on both strands by the dideoxy nucleotide chain termination method using an automatic sequencer (Applied Biosystems). All the clones were shown to only contain a partial ORF. The cDNA insert of the longer mouse clone (designated pBS-mTASK1) was $^{32}$P-labelled and used to screen a human kidney cDNA library. Two independent hybridizing clones were isolated and sequenced. Both clones (2.5 kb long) were shown to contain the full-length ORF. The longer one was designated pBS-hTASK1.

Both cDNAs contain an ORF of 1185 nucleotides(SEQ ID No. 3) encoding a polypeptide of 394 amino acids (SEQ ID No. 4) (Figure 8). The human protein sequence contains consensus sites for N-linked glycosylation (residue 53), and phosphorylation by protein kinase C (residues 358 and 383), tyrosine kinase (residue 323) and protein kinase A (residues 392 and 393). All these phosphorylation sites are located in the C-terminus part of the protein. Except for a 19 residues cluster (aa 276 to 294 in the human sequence), mouse and human proteins share a high overall sequence conservation (85% of identity) indicating that they probably are products of ortholog genes. Sequence alignments presented in Figure 9 clearly show that the cloned protein is a new member of the TW1K related $K^+$ channel family. Like TWIK-1 (SEQ ID No. 2) and TREK-1 (SEQ ID No. 8), TASK1 (SEQ ID No. 4) has four putative transmembrane segments (M1 to M4) and two P domains (P1 and P2) (Figures 9A and 9B). TASK1 is 58 amino acids longer than TWIK-1 and 24 amino acids longer than TREK-1 because its C-terminus is more extended.

## Tissue Distribution of TASK1

The expression of TASK1 in adult human and mouse tissues was examined by Northern blot analysis. For Northern blot analysis, poly(A)$^+$ RNAs were isolated from adult mouse tissues and blotted onto nylon membranes as previously described (Lesage *et al.,* 1992). The blot was probed under stringent conditions with the $^{32}$P-labelled insert of pBS-mTASK1 in 50% formamide, 5X SSPE (0.9 M sodium chloride, 50 mM sodium phosphate (pH 7.4), 5 mM EDTA), 0.1% SDS, 5X Denhardt's solution, 20 mM potassium phosphate (pH 6.5) and 250 μg denatured salmon sperm DNA at 50°C for 18 h and washed stepwise at 55°C to a final stringency of 0.2XSSC, 0.3% SDS. For hybridization of human multiple tissues Northern blots from Clontech, the procedure was identical except that the probe was derived from pBS-hTASK1. The cDNA insert of pBS-hTASK1 contains different repeat sequences (AluJb, MIR and (CGG)n) in the untranslated regions (UTR) and a *Sma*I/*Apa*I restriction fragment of 1390 bp spanning the coding sequence was chosen as a probe that does not contain these repeats. Three different transcripts were detected in the human tissues with estimated sizes of 6.8, 4.2 and 2.6 kb (Figure 10), the shorter one having the same size that the cloned cDNAs. The two other transcripts (4.2 and 6.8 kb) may result from alternate polyadenylation signals in the 3' non-coding sequence and/or correspond to alternatively spliced or immature forms of the transcript. TASTC1 is expressed in many different tissues but is particularly expressed in pancreas and placenta. Lower levels of expression were found in brain > lung, prostate > heart, kidney > uterus, small intestine and colon. As shown in Figure 11A, the TASK1 probe detected a single transcript in the mouse with an estimated size of 4.2 kb. TASK1 is expressed in heart >lung > brain and kidney. No expression was seen in liver and skeletal muscle.

The TASK1 distribution was further studied in adult mouse brain and heart by *in situ* hybridization. *In situ* hybridization experiments were performed on adult Balb/c mice by using standard procedures (Fink *et al.,* 1996b). An antisense oligonucleotide (48 mer, 5'-CACCAGCAGGTAGGTGAAGGTGCACACGATGAGAGCCAACGTGCGCAC-3') (SEQ ID No. 24) complementary to the mouse cDNA sequence of TASK1 (from nucleotides 7 to 54) was used to detect the expression of TASK1 transcripts in frozen fixed brain sections (10 μm). The probe was 3'-end-labelled with ($\alpha$- $^{33}$P) dATP. Sections were digested with 5 μg/ml of proteinase K for 15 min at 37 °C, acetyled for 10 min in 0.25% acetic anhydre in 0.1 M triethanolamine. Hybridization was carried out overnight at 37 °C in 2X SSC, 50% formamide, 10% dextran sulfate, 1X Denhardt's solution, 5% sarcosyl, 500 μg denatured salmon sperm DNA, 250 mg/ml yeast tRNA, 20 mM dithiothreitol, and 20 mM$NaPO_4$ with 0.2 ng/ml of radio labelled probe (specific activity = $8.10^8$ dpm/μg). Slides were then washed in 1X SSC before dehydratation, drying, and apposition to hyperfilm-βmax (Amersham) for 6 days. The specificity of labelling was verified by *in situ* hybridization using cold displacement of radioactive probe with a 500-fold excess of unlabelled oligonucleotide. A wide and heterogeneous pattern of expression was obtained in the brain (Figures 11B and 11C). TASK1 mRNA was detected throughout the cell layers of the cerebral cortex, in the CAl-CA4 pyramidal cell layer, in the granule cells of the dentate gyrus, in the habenula, in the paraventricular thalamic nuclei, in the amyloid nuclei, in the substantia nigra and in the Purkinje and granular cells of the cerebellum. In the heart, a high level of TASK1 expression was found in the atria (Figure 11D) while ventricular cells did not express this channel.

**<u>Biophysical properties of TASK1 currents in *Xenopus* oocytes.</u>**

For functional studies, TASK1 cRNAs were injected into *Xenopus* oocytes. This was accomplished by subcloning a 2480 bp *Sma*I/*Xho*I fragment from pBS-hT ASK containing 14 bp of 5' UTR, the coding sequence and the entire 3' UTR into the pEXO vector (Lingueglia *et al.,* 1993) to give pEXO-TASK1. Capped-cRNAs were synthesised *in vitro* from the linearized plasmid by using the T7 RNA polymerase (Stratagene). *Xenopus laevis* were purchased from CRBM (Montpellier, France). Preparation and cRNA injection of oocytes has been described elsewhere (Guillemare *et al.,* 1992). Oocytes were used for electrophysiological studies 2 to 4 days following injection (20 ng/oocyte). In a 0.3 ml perfusion chamber, a single oocyte was impaled with two standard microelectrodes (1-2.5 Mn resistance) filled with 3 M KCI and maintained under voltage clamp by using a Dagan TEV 200 amplifier, in standard ND96 solution (96 mM NaC1. 2 mM KCl, 1.8 mM $CaCl_2$, 2 mM $MgCl_2$, 5 mM HEPES, pH 7.4 with NaOH). In some experiments, NaCl was substituted with TMA Cl (Tetra Methyl Ammonium Chloride). Stimulation of the preparation, data acquisition, and analysis were performed using pClamp software (Axon instruments, USA). Drugs were applied externally by addition to the superfusate (flow rate: 3 ml/min) or intracellularly injected by using a pressure microinjector (Inject+Matic, Switzedand). All experiments were performed at room temperature (21-22 °C).

A non-inactivating current, not present in uninjected oocytes (not shown), was measured by two-electrode voltage-clamp (Figure 12A). Activation kinetics of the TASK1 current are almost instantaneous (under 10 ms). The current-voltage (I-V) relationship is outwardly-rectifying and almost no inward currents were recorded in the ND96 external medium containing 2 mM $K^+$ (Figure 12B). However, inward currents were revealed when the external $K^+$ concentration ($[K^+]_{out}$) was gradually increased to 98 mM $K^+$ (Figures 12A and 12B). Figure 12A presents the I-V relationships of the current in $K^+$-rich solutions ranging from 2 mM to 98 mM. The relationship between the reversal potential and $[K^+]_{out}$ was close to the predicted Nemst value (52.1 mV/ decade, n = 4) as expected for highly selective $K^+$ channel (Figure 12C, upper panel). On the other hand, external $K^+$ enhanced the outward currents in a concentration-dependent manner as illustrated in Figure 12C (lower panel). The half maximum activation by $K^+$ was observed at a $K_{0.5}$ of 2.06 mM. The theoretical I-V relationships in various $[K^+]_{out}$ calculated according to the Goldman-Hodgkin-Katz current equation are shown in Figure 12D. These I-V relationships are very close to the I-V relationships corresponding to recorded TASK1 currents (Figure 12A). This strongly suggests that TASK1 currents show no rectification other than that predicted from the constant-field assumptions and that TASK1 lacks intrinsic voltage-sensitivity. The slight deviation between experimental and theoretical points are probably due to small endogenous chloride conductance and/or a $K^+$ loading of the oocytes. We have previously shown that oocytes expressing TWIK-1 or TREK-1 are more polarized that control oocytes, the resting membrane potential ($E_m$) reaching a value close to the $K^+$ equilibrium potential ($E_K$). In oocytes expressing

TASK 1, $E_m$ was -85 ±0.8 mV (n = 23, in standard ND96) instead of - 44 ±2.6 mV (n = 9) in non-injected oocytes. This result demonstrates that TASK1, like other TWIK or TREK channels, is able to drive $E_m$ close to $E_K$. The effect of various pharmacological agents on currents elicited by voltage pulses to +50 mV has been studied in TASK1-expressing oocytes. Less than 20% of TASK1 currents were inhibited in the presence of quinine (100 $\mu$M), quinacrine (100 $\mu$M) or quinidine (100 $\mu$M). The "classical" $K^+$ channels blockers tetraethylammonium (TEA, 1 mM) and 4-aminopyridine (4AP, 1 mM) were also inactive. $Cs^+$ (100 $\mu$M) induced a voltage-dependent block of 31 ±2% (n = 4) of the inward current, recorded at -150 mV, in 50 mM external $K^+$. In the same conditions, $Ba2^+$ (100$\mu$M) was ineffective with a variation of 6 ±1% (n = 4) of the inward current:

**Biophysical properties of TASX1 channel in transfected COS cells,**

The 2480 bp *Sma*I/*Xho*I fragment of pBS-TASKI was subcloned into the pCi plasmid (Promega) under the control of the cytomegatovirus promoter to give pCi- TASK1. COS cells were seeded at a density of 70,000 cells per 35 mm dishes 24 h prior transfection. Cells were then transfected by the classical calcium phosphate precipitation method with 2$\mu$g of pCl-TASK1 and 1 $\mu$g of CD8 plasmids. Transfected cells were visualized 48 h after transfection using the anti-CD8 antiboby coated beads method (Jurman *et al.,* 1994). For electrophysiological recordings, the internal solution contained 150 mM KCl, 3 mM $MgCl_2$, 5 mM EGTA, and 10 mM HEPES at pH 7.2 with KOH, and the external solution 150 mM NaCl, 5 mM KCl, 3 mM $MgCl_2$, 10 mM HEPES at pH 7.4 with NaOH.

Untransfected cells did not express this $K^+$ channel activity (not shown). Figure 12E shows whole cell currents recorded in the mammalian COS cells transiently transfected with TASK1, in external solutions containing 5 raM and 155 mM $K^+$. The current were instantaneous and non-inactivating as in *Xenopus* oocytes. Figure 12F presents the I-V relationships of TASK1 current in various external $K^+$ concentrations. The currents show the same Goldman-Hodgkin-Katz type outward rectification as in oocytes.

**Regulation of the TASK1 channel.**

TASK1 currents were insensitive to internal $Ca^{2+}$ changes obtained by injection of inositol triphosphate (IP3, 1 mM) or EGTA (100 mM), to the activation of adenyl cyclase by perfusion ofIBMX (1 mM) and forskolin (10$\mu$M), or to the activation ofprotein kinase C (PKC) by application of the phorbol ester PMA (40 nM). TASK1 currents were insensitive to the internal acidification or alkalisation obtained by injection of solutions at pH 2 or 8.7 respectively (n = 3). However, their very interesting property is that they are highly sensitive to external pH. The current-potential relationships recorded from a TASK1 -expressing oocyte at pH 6.5,7.4 and 8.4 are presented in Figure 13A. For an external pH of 6.5, a drastic block was observed at all potentials while an activation was recorded at pH 8.4, also at all potentials. The inhibition and activation produced no modification of current kinetics (Figure 13A, inset). The pH-dependence of the TASK1 channel is shown in Figure 13B. For currents recorded at +50 mV, the inhibition by acidic pHs was characterised by an apparent pK of 7.34 ±0.04 units (n = 3) and a Hill coefficient of 1.54 ±0.08 (n = 3). For currents recorded at 0 and .50 mV, the pKs were 7.32 ±0.02 and 7.30 ±0.01 respectively (n = 3) showing that the blocking effect of external protons is not voltage-dependent. The resting membrane potential of TASK1-expressingoocytes was -84 ±1 mV (n = 6) at pH 7.4 and shifted to -47 ±6 mV (n = 6) at pH 6.4 (not shown). Finally, Figures 13C and 13D show that the strong pH sensitivity of TASK1 currents was also observed in transfected COS cells. A large inhibition or activation of the current was recorded, at all potentials, when pH was changed from 7.4 to 6.1 or 7.9 respectively (Figure 13C). The kinetics of the current were unmodified at both pH (Figure 6C, inset). Figure 13D shows that the pH effects were also non voltage-dependent in COS cells. The external pH-dependence of TASK1, at +50 mV, indicates a pK value of 7.29 ±0.03 (n = 5) and a Hill coefficient of 1.57 ±0.07 (n = 5). Currents recorded at 0 and -50 mV presented pKs of 7.29 ±0.04 (n = 5) and 7.32 ±0.05 (n = 4) respectively. 10% of the maximum current was obtained at pH 6.68 ±0.08 (n = 4) and 90% at pH 7.66 ±0.05 (n =4). These results confirm that TASK1 is extremely sensitive to extracellular pH in the physiological range.

**Additional non-mammalian homologs of TWIK-1**

Comparison of the complete sequence of TWIK-1 (SEQ ID No. 1) with the sequences of the Genbank data base allowed identification of at least five genes of *Caenorhabditis elegans,* which had been characterized in the context of the Nematode Sequencing project, which may encode additional structural homologs of TWIK-1. The alignment of two of these homologs (SEQ ID No. 6 and SEQ ID No. 7) with TWIK-1 (SEQ ID No. 2) is shown in Figure 2b. The degree of similarity between the deduced protein sequences of *C. elegans* and TWIK-1 are approximately 55 to 60%. Amino acid sequence identities among the deduced polypeptide sequences range from 25 to 28%. Interestingly, the degree of similarity and amino acid sequence identity of the various *C. elegans* are not greater than what was determined for TWIK- 1. These results indicate that other TWIK-1 related potassium channels maybe present in the *C. elegans* genome and suggest that additional members of the 2P family of potassium channels may exist in mammals.

**Additional mammalian homologs of TWIK-1, the identification of TASK2**

Indeed, the method described above has also led to the identification of a human EST (GenBank accession number HO 1932) that exhibits similarity to the M1P1 extracellular loop of TWIK-1. The 5' end of this EST encoded the P1 and M2 domains of a two P domain channels as expected. We postulated that this EST was a tandem cDNA or was issued from an unspliced mRNA, and we used only the 5' part of the sequence to design oligonucleotides. These oligonucleotides were further used to carry out 3'-RACE PCR experiments on human brain cDNA by standard methods. A DNA fragment was obtained that extended the region homologous to TWIK-1 and previously identified in HOI 932. Two novel oligonucleotides were deduced from the homologous part of this DNA fragment: sense strand, 5'-CACAGAAGCTGCATCTGCTCA-3' (SEQ ID No. 2S); antisense strand, 5'-CCCTCAGTCTCCATGAATAGGA-3' (SEQ ID No. 26). They were used to amplify a 430. bp fragment that was $^{32}P$-labeled and used to screen a human kidney cDNA library as previously described (8). The screening of 3 x $10^5$ phages yielded 37 positive clones. Seven clones were excised from λZAPII XR vector into pBluescriptII SK- (pBS), and were analyzed by restriction analysis and by sequencing of their extremities. The longer cDNA insert (pBS-TASK-2) was completely sequenced on both strands by using the dideoxy nucleotid termination method using an automatic sequencer (Applied Biosystem, model 373A). The full length cDNA of 3.5 kb (SEQ ID No. 27) contains an extended open reading frame that codes for a polypeptide of 499 residues (SEQ ID No. 28) with a calculated MW of 55.2 kDa. The predicted product displays all the hallmarks of the 2P domain $K^+$ channels (Fig. 14A). Analysis of its hydropathy profile indicates the presence of four TMS noted M1 to M4, the M1 and M2 segments flanking a first P domain (P1) and the M3 and M4 flanking a second P domain (P2). An extended M1P1 interdomain which is characteristic of this channel family is also found that is expected to be extracellular as for TWIK-1. This region contains a potential N-linked glycosylation site as well as a cysteine residue (position 51), that is conserved in several other 2P potassium channels. In TWIK-1 (SEQ ID No. 2), this cysteine residue has been shown to be implicated in the formation of an interchain disulfide bond (25). Despite this overall structural conservation, the novel subunit is only distantlyrelated to the other cloned 2P domain $K^+$ channels (between 18 and 22% of amino acid identity). The dendrogram shown in Fig. 14B also suggests that the novel subunit is not more related to TASK than to TWIK-1, TREK-1, or TRAAK. However, it was called TASK-2 (SEQ ID No. 27) to emphasize the fact that it produces $K^+$ currents that are Acid-Sensitive like TASK-1 as shown below. For this reason, TASK is now called TASK-1 (SEQ ID No. 4).

**Tissue distribution of TASK-2**

The tissue distribution of TASK-2 in adult human was analyzed by Northern blot and RT-PCR. Human Multiple Tissue Northern (MTN) blots were purchased from Clontech and hybridized at 65 °C in ExpressHyb solution with 0.6 and 1.2 kb *Sma*I $^{32}P$-labeled fragments from pBS-TASK-2 following the manufacturer's protocol. For RT-PCR experiments, total RNAs were extracted from adult mouse tissues and from mouse embryos with the SNAP total RNA isolation kit (Inrritrogen). After a DNAse treatment, 15 $\mu$g of total RNA were reverse-transcripted according to the manufacturer's instructions (Gibco BRL). 1/40 $\mu$l of each sample were used as template for PCR amplification (Taq DNA polymerase, Gibco BRL) by using TASK 2 (base positions 358-381: 5'.CTGCTCACCTCGGCCATCATCTTC-3' (SEQ ID No. 29) and 901-924: S'-GTAGAGGCCGTCGATGTAGTTCCA-3' (SEQ ID No. 30) and GAPDH (Clontech) primers. PCR conditions were 30 cycles of 30 sec at 94°C, 30 sec at 60°C, and 30 sec at 72 °C. TASK-2 amplified fragments were transferred onto nylon membranes then probed at high stringency (50% aqueous formamide at 50°C) with a $^{32}P$-labeled *Sma*I DNA fragment of pBS-TASK-2 (nt 116 to 1305).

In *situ* hybridization was performed as previously described (21) on 7$\mu$m paraffin sections of human kidneys fixed in 4% paraformaldehyde. A specific anti-sense cRNA probe was generated with T7 RNA polymerase (Promega), by *in vitro* transcription using $^{35}S$-$\alpha$-UTP from a *Eco*RI-linearized plasmid containing a 337 bp *Nco*I/*Cla*I fragment of the 5'-untranslated sequence of TASK-2 cDNA inserted into pBS. The same plasmid was linearized by *Xho*I and T3 RNA polymerase was used for the synthesis of a control sense probe. The probes were hybridized, then slides were covered with NTB2 emulsion (Kodak) and exposed for 32 days at -20°C. Typical stringent hybridization conditions are described above; typical non-stringent conditions contain lower concentrations of formamide (0 - 30% formamide), or a solution comprising 6 X SSC, 5 X Denhardt's solution at 60°C. After development, slides were stained with toluidine blue and photographed. The fragments of human kidney were obtained from surgical ablation of renal cancers (pieces of tissues surrounding the tumor).

Northern blotting (Fig. 15A) showed that a 4 kb transcript is abundantly expressed in the kidney and is present to a lesser extent in the pancreas, the liver, the placenta, and the small intestine. The expression of TASK-2 was also analyzed by RT-PCR from mouse tissues (Fig. 15B). The mouse TASK-2 message was found in the kidney, the liver, and the small intestine, in the same relative abundance as Inhuman. As expected, the RT-PCR method is more sensitive than the Northern blot technique, and faint positive signals were also obtained in mouse brain, heart, skeletal muscle and colon. Surprisingly, TASK-2 expression levels in uterus, lung and pancreas are different between human and mouse.

Figure 15B compares the distribution of TASK-2 to those of the other cloned 2P domain $K^+$ channels, TWTK-1, TREK-1, and TASK-1. As shown previously by Northern blot analyses, the TWIK-1, TREK-1, and TASK-1 channels are widely distributed but with unique patterns. The expression pattern of TASK-2 is also unique. TASK-2 is preferentially expressed in the kidney and the liver, and is quasi absent in the nervous system, unlike TWIK-1, TREK-1, or TASK-1 (Fig. 15B). TRAAK is exclusively expressed in neuronal cells (12).

Both Northern blot and RT-PCR analyses indicate that the tissue that expresses the highest levels of TASK 2 is the kidney. The distribution of TASK-2 in the human kidney cortex was observed at a higher resolution after *in situ* hybridization. Fig. 16 shows that the expression of TASK-2 is restricted to the distal tubules and the collecting ducts. No specific signal was observed in the proximal tubules or over the glomeruli.

**Chromosomal mapping of TASK-2**

The chromosomal assignment of human TASK-2 was carried out by Radiation Hybrid panel analysis. The Genebridge 4 RH DNA panel (Research Genetics) was screened by PCR using primers deduced from the 3'-untranslated part of TASK-2 cDNA (sense primer, 5'-CTTCCTAACCTTCCATCATCC-3' (SEQ ID No. 31) anneals to base positions 1971-1991, and the antisense primer, 5'-CTTGACCTGAGAGAGGGAAC-3'(SEQ ID NO. 32) anneals to bases 2455-2564. PCR conditions were as follows: 40 cycles of 30 sec at 94°C, 30 sec at 50°C, 30 sec at 72°C. PCR products were separated by electrophoresis on agarose and transferred onto charged nylon membranes. Blots were probed at high stringency (e.g., 50% aqueous formamide at 45°C) with a $^{32}$P-labeled DNA probe spanning the amplified region of the TASK-2 gene. The results were analyzed by using RHMAPPER program at the Whitehead Institute (www-gename.wi.mit.edu).

As shown in Fig. 17, the gene encoding TASK-2 lies on chromosome 6p and is 5.45 cR centromeric to the framework marker W1-4142 (LOD score of 21). Although radiation hybrid maps are not anchored to the cytogenic maps, the most likely localization of the TASK-2 gene is 6p21.31-p21.33. TWIK-1 has been previously mapped to chromosome 1 q42-1q43 (26), and TREK and TASK to chromosomes 1q41 and 2p23, respectively (27).

**Biophysical and pharmacological properties of TASK-2**

The sequence coding for TASK-2 was amplified by PCR using a low error rate polymerase (Pwo polymerase, Boehringer Mannheim) and subcloned into the pEXO vector (22) to give pEXO-TASK. Capped-cRNAs were synthesized *in vitro* from the linerarized plasmid by using the T7 RNA polymerase (Stratagene). *Xenopus laevis* were purchased from CRBM (Montpellier, France). Preparation and cRNA injection of oocytes has been described elsewhere (23). Oocytes were used for electrophysiological studies 2 to 4 days following injection (20 ng/oocyte). In a 0.3 ml perfusion chamber, a single oocyte was impaled with two standard microelectrodes (1-2.5 MΩ resistance) filled with 3 M KCl and maintained under voltage clamp by using a Dagan TEV 200 amplifier, in standard ND96 solution (96 mM NaCl, 2 mM KCl, 1.8 mM $CaCl_2$, 2 mM $MgCl_2$,5 mM HEPES, pH 7.4 with NaOH). Stimulation of the preparation, data acquisition, and analysis were performed using pClamp software (Axon instruments, USA). Drugs were applied externally by addition to the superfusate (flow rate: 3 ml/min). All experiments were performed at room temperature (21-22°C).

For patch clamp analyses, a *Not*I/*Eco*RI fragment of 3.2 kb was excised from pBS-TASK 2 and subcloned into the pIRES-CD8 vectorto givepIREScdB-TASK-2. ThepIRES-CD8 vector was obtained by replacing the neo$^R$ gene in the original vector pIRESneo (Clontech) by the coding sequence of the T type lymphocyte's surface marker CD8. COS cells were seeded at a density of 20,000 cells per 35 mm dishes 24 h prior to transfection. Cells were then transiently transfected by the classical DEAE-dextian method with 1 μg of prREScdB-TASK-2 plasmid per 35 mm dish. Transiently-transfected cells were visualized 48 h after transfection using the anti-CD8 antibody coated beads method (24). For whole cell recordings, the internal solution contained 150 mM KCl, 3 mM $MgCl_2$, 5 mM EGTA, and 10 mM HEPES at pH 7.2 with KOH, and he external solution 150 mM NaCl, 5 mM KCl, 3 mM $MgCl_2$ 1 mM $CaCl_2$, 10 mM HEPES at pH 7.4 with NaOH.

TASK-2-transfected COS cells display non-inactivating currents (Fig. 18A) that are not present in control cells (not shown). The activation kinetics of TASK-2 currents are rapid. They are fitted with a single exponential characterized by time constants of 60.9 ± 6ms at +50 mV and 62.6 ± 6 ms at 0mV (n = 8). The current-voltage (I-V) relationship is outwardly-rectifying and almost no inward currents were recorded in an external medium containing 5mM $K^-$ (Fig. 18B). When cells were perfused with a $K^+$ -rich solution (155mM), the TASK-2 currents presented an almost linear I-V relationship and the inward currents recorded at very negative potentials were noisy (Fig. 18A, 18B). The relationship between the reverse potential and $[K^+]_o$ is close to the predicted Nemst value (58.9 ± 4.5 mV / decade, n = 4) as expected for a highly selective $K^+$ channel (Fig. 18C). TASK-2 was also expressed in *Xenopus* oocytes where it shows similar properties (Fig. 18D). Activation kinetics of the TASK currents in oocytes are slightly slower, with time constants of 112.6 ± 16.8 ms at +50 mV and 102.9 ± 7.3 ms at 0 mV (n = 6). As previously shown with TWIK-1, TREK-1, TRAAK and TASK-1 (8, 10-12), the membrane potential of oocytes expressing TASK-2 is strongly polarized, (-78.6 ± 2.7 mV,n=9) compared to control oocytes (-42.2 ± 3.1 mV, n = 6).

Single channel TASK-2 currents were recorded in outside-out patches from transfected COS cells. They are very flickery and show substates (Fig. 18E). In 5 mM external $K^+$, the single channel I-V relationship is almost linear between -80 and +40 mV and presents a saturation at potentials more positive than +40 mV (Fig. 18F). The slope conductance measured between -60 mV and +20 mV is 14.5 ± 1.4 pS (n = 22). In 155 mM external $K^+$, the slope conductance is greatly increased (59.9 ± 3.1 pS, n =19) and saturates both at very negative and positive potentials.

The effects of various pharmacological agents on currents elicited by voltage pulses to +50 mV have been studied in TASK-2-exprcssing. COS cells. The "classical" $K^+$ channels blockers tetraethylammonium (TEA, 1 mM), 4-aminopyridine (4-AP, 100 μM) and $Cs^+$ (1 mM) were inactive on the recorded currents. $Ba^{2+}$ only slightly diminished the current at +50 mV (16.9 ± 1.6% at 1 mM, n = 3). Quinine induced a dose-dependent inhibition of TASK-2 currents characterized by an $IC_{50}$ of 22.4 ± 1.8 μM (n = 9) (not shown) whereas 100 μM quinine induced a 65 ± 3.8% inhibition of the current (n = 4). A strong effect was observed with lidocaine (1 mM) and bupivacaïne (1 mM) with inhibitions of 60.4 ± 1.5% and 80-9 ± 4.5%, respectively (n = 4). Zinc (100 μM) was also tested and induced a slight decrease of the current (15.3 ± 2.2%, n = 5). On the contrary to TRAAK (12), TASK-2 is not sensitive to arachidonic acid (10 μM).

**Regulation of TASK-2 activity by external pH**

TASK-2 currents were insensitive to the activation of adenyl cyclase obtained by increasing intracellular cAMP with a cocktail of IBMX (1 mM) and forskolin (10 μM), or by perfusion of the permanent 8-chloro cAMP (500 μM), or to the activation of PKC obtained by an application of the phrobol ester PMA (70 nM).

Interestingly, TASK-2 currents are highly sensitive to external pH, like TASK-1 (11). The I-V relationships recorded at pH 6.0, 7.4, and 8.6 are presented in Fig. 1*9A. For an external pH of 6.0, a drastic block was observed at all potentials while an activation was recorded at pH 8.6, also at all potentials. The inhibition and activation produced no modification of current kinetics (Fig. 19B). The pH-dependence of the TASK-2 channel is shown in Fig.19C. For currents recorded at -50 mV, 0 mV and +50m V the inhibition by acidic pHs was characterized by pHm values (for 50% of inhibition) of 8.6 ± 0.1, 8.3 ± 0.1 and 7.8 ± 0.1 units, respectively (n = 6). The Hill coefficient at +50 mV is 0.69 ± 0.09 (n = 6). Fig. 19D shows the pH sensitivity of TASK-2 single channel currents in the outside-out configuration. A large inhibition of the current was recorded at pH 6.5 and an increase at pH 9.1. Fig.19E shows that the pH effects are due to a variation in N.Po and not in the single channel conductance (Fig. 19F). 10% of the maximum current was obtained at pH 6.5 ± 0.1 (n = 6) and 90% at pH 8.8 ± 0.1 (n= 6). These results indicate that TASK-2 is very sensitive to extracellular pH in the physiological range.

**Unique structural features of 2P family of potassium transport channels.**

The overall level of amino acid sequence (i.e. primary structure) identity among the various homologs ofTWIK-1, and indeed among all of the known potassium transport channels is very low, approximately 25-28%. However, the presence and organization of three specific domains of TWIK-1 and its homologous sequences TREK-1, TASK1 and TASK2 distinguish these polypeptides from other groups of channels, e.g., the IRK and Shaker proteins. On the basis of this structural similarity, these proteins can be considered to represent a new family of mammalian potassium transport channels, referred to as the 2P family of transport channels. Members of the 2P family are unique in that they possess four transmembrane domains, whereas the IRK and Shaker polypeptides contain 2 and 6 transmembrane domains, respectively. Second, the 2P family of proteins are the only potassium channels that are known to possess two P domains. Indeed, the highest degree of sequence identity observed among the various members ofthe 2P family of channels occurs within these P regions. Thirdly, all members ofthe 2P family are distinguished by the presence of an extended M1P1 interdomain. This peculiar domain has been shown to be extracellular in the case ofTWIK-1 and to be important for the self-association of two TWTIC-1 subunits. The TWIK-1 homodimers are covalent because of the presence of an interchain disulfide bridge between cysteine residues(i.e., amino acid residue 69) located in the M1P1 interdomain (Lesage *et al.,* 1996b). This cysteine residue is conserved in TREK-1 (residue 93) and TASK2 (residue 51), but not in TASK1. This suggests that TASK1 may not form covalent dimers as observed for TWIK 1 (Lesage *et al.,* 1996b), TREK-1 and TASK2.

**Novel Characteristics of TASK-2.**

TASK-2 is a novel member of the emerging family of 2P domain $K^+$ channels. Its cloning extends to five the number of these channels identified to date in mammals. Despite an overall structure conservation, TASK-2 does not share more than 18-22% of amino acid identity with the four other cloned channels and does not seem to be more related to any one of them from a phylogenetic point of view.

Both the *Shaker* and IRK families of $K^+$ channel subunits comprise numerous distinct proteins encoded for by different genes. Within each of these two superfamilies, different subclasses can be distinguished according to their sequence similarities. Moreover, sequence conservation within each family is associated with similar functional properties. For instance, within the IRK family, the Kir3.x subunits share 55-60% of amino acid identity (28). All these Kir3.x proteins form G-protein-activated $K^+$ channels. Kir subunits belonging to the other subgroups are more distant from the point of view of sequences and form inward rectifier channels that are not activated by G-proteins (29-31). On the other hand, the *Shaker*-related voltage-dependent Kvl.x $K^+$ channels (32-34) or the $Ca^{2+}$-dependent SK channels (35) form subsets of proteins sharing 70-85% of amino acid identity. However, in spite of a similar structure with 6 TMS and one P domain, Kv1.x and SK channels have less than 20% of overall amino acid identity. The question then arises to know whether equivalent structural and functional subfamilies can be distinguished in the 2P domain $K^+$ channel family. With a low sequence conservation between the different channels cloned up till now (18-38% of identity), the usual criteria of sequence similarity cannot be used. Nevertheless, our work leads to propose a functional classification. TWIK-1 forms a first functional group because it is the only one to express weakly inward rectifying currents. TREK-1 and TRAAK form a second group of channels that produce outwardly rectifying currents stimulated by arachidonic acid and polyunsaturated fatty acids (10, 12, 19). The fact that both channels share 38% of amino acid identity instead of the 18-22% of identity usually found between the 2P domain $K^+$ channels could signify that they have evolved from a common ancestral gene. However, TRBK-1 and TRAAK probably have different physiological significances, since they have quite different tissue distributions as well as different electrophysiological and regulation properties. The quasi-ubiquitous TREK-1 channel is inhibited by cAMP, but not the neuronal TRAAK channel. TRAAK loses its outward rectification in high external [$K^+$] but not TREK-1. The last functional group of 2P domain $K^+$ channels is composed of TASK-1 and TASK-2. Both channels produce open rectifier $K^+$ currents that are inhibited by a drop of external pH in the physiological range (11, 13). Their pharmacological behaviors are also similar. TASK-2, as TASK 1, is relatively insensitive to classical $K^+$ channel blockers such as $Ba^{2+}$, $Cs^+$, TEA, and 4-AP, and both TASK-1 and TASK-2 are blocked by the local anesthetics lidocaine and bupivacaine. Their sequences are only distantly related and for this reason, it is extremely difficult to know whether they have evolved from a common gene coding for an ancestral pH-sensitive $K^+$ channel. TASK-1 and TASK-2 have different tissue distributions. TASK-1 is widely expressed in excitable as well as non-excitable tissues (11), while TASK-2 seems to be preferentially present in epithelia. They also show significant differences in terms of electrophysiological and regulation properties. Unlike TASK-1 activity, TASK-2 activity is not inhibited by variations of intracellular cAMP (13), and TASK-2 is the sole 2P domain $K^+$ channel cloned to date that displays relatively slow activation kinetics (11, 13, 14). TASK-2 message is poorly expressed or absent in the nervous and muscular systems but is present in epithelial tissues such as lung, colon, intestine, stomach, liver, and particularly in the kidney. In kidney, TASK-2 is more precisely located in the cortical distal tubules and collecting ducts. In these structures, $K^+$-selective currents are postulated to play a major role in the volume regulation and in the control of the negative potential of tubule cells, in $K^+$ recycling across the basolateral membranes in conjunction with the Na-K-ATPase, and in the $K^+$ secretion into the tubular lumen in concert with $Na^+$ influx through amiloride-sensitive $Na^+$ channels (for reviews, see (3, 36)). Principal cells of the collecting ducts express at least two types of apical $K^+$ currents sharing common properties, such as inhibition by $Ba^{2+}$ and ATP, as well as by internal acidification (3). The first one is a $K^+$ channel with a large conductance and a low probability of opening, that is activated by membrane depolarization and internal $Ca^{2+}$, and that is inhibited by TEA (37, 38). The second one is a small conductance (25 pS) $K^+$ channel with a high Po and inward rectification and that is insensitive to TEA (3, 40). A cloned $K^+$ channel that has the same properties is ROMK2 (41). On the other hand, three other $K^+$currents have been described at the basolateral membrane of the collecting duct cells: a small conductance $K^+$ channel (28 pS) up-regulated by protein kinase C, nitric oxide (NO) and cGMP (36,42, 43), am intermediate conductance (85 pS) $K^+$ channel activated by protein kinase A and hyperpolarization (44), and a large conductance (147 pS) $K^+$ channel (42). However, the principal biophysical and pharmacological properties of TASK-2 do not fit those of these native $K^+$ channels. A possibility would be that TASK-2 channels are present in kidney cells but have not yet been recorded, which would not be surprising because of the lack of a specific pharmacology. Anotherpossibility would be that TASK-2 associates with yet unidentified pore-forming subunits or regulatory proteins to produce an active channel in native cells with properties different from those of the cloned channel as it has been observed for some other $K^+$ channels (45-47). The inhibition of newly cloned $K^+$ channels by acidification is consistent with the effect of metabolic acidosis which decreases $K^+$ secretion in distal tubules (36).

New insights into the mechanism of $K^+$ secretion have been recently provided by the cloning of several renal $K^+$ channels and by fine studies of their distribution and cellular localization. The present invention relates to four different areas: (i) identification of the native renal $K^+$ channel with the properties of TASK-2, (ii) identification of potent pharmacology that specifically modulates the activity of the TASK-2 channel, (iii) localizing K+ channels comprising the TASK2 subunit *in vivo,* and (iv) the generation of mice in which the TASK2 gene has been inactivated. In addition, genetic diseases associated with potassium transport channel deficiencies or malfunction are now being discovered with an increasing frequency (48). Therefore, the present invention also relates to diagnostic tests and therapeutic methods to (i) detect and treat human hypertension and diseases that are associated with kidney, pancreas and/or liver dysfunctions that may arise from mutations in the TASK-2 gene, (ii) protect against tissue rejection in kidney, pancreas and liver transplants, and (iii) identify new potential drugs capable of modulating the activity of TASK2.

Thus, the present invention relates to isolated, purified nucleic acid molecules, each of which encodes a protein constituting a potassium channel of the 2P family and/or exhibiting the properties and structure of the type of the TWIK-1 channel described above.

More specifically, the said nucleic acid molecule codes for the TWIK-1 protein, the amino acid sequence of which is represented in the attached sequence list as number SEQ ID NO: 2, TASK1, represented in the attached sequence list as number SEQ ID NO: 4, TASK2 represented by SEQ ID NO: 28, or functionally equivalent derivatives of these sequences that possess the distinguishing structural features of the 2P family of potassium transport proteins. Such derivatives can be obtained by modifying and or suppressing one or more amino acid residues of this sequence, as long as this modification and/or suppression does not modify the functional properties of the TWIK-1 potassium channel of the resultant protein.

The sequences of the DNA molecules coding for the 2P proteins are represented in the attached sequence list as number SEQ ID NO: 1 (TWIK-1), SEQ ID NO: 3 (TASK1), and SEQ ID NO:27 (TASK2).

The invention also relates to a vector containing a molecule of the aforementioned nucleic acid sequences, as well as a procedure for the production or expression in a cellular host of a protein constituting a 2P potassium channel or a channel of the same family as TWIK-1.

A procedure for production of a protein constituting a 2P potassium channel or exhibiting the properties and structure of the type of the TWIK-1 channel consists of:

- transferring a nucleic acid molecule related to the invention or a vector containing the said molecule into a cellular host,
- culturing the cellular host obtained in the preceding step under conditions enabling the production of potassium channels exhibiting the properties of TWIK-1,
- isolating the proteins constituting the potassium channels of the 2P family.

A procedure for the expression of a TWIK-1 potassium channel or a potassium channel of the same family as TWIK-1 consists of:

- transferring a nucleic acid molecule related to the invention or a vector containing the said molecule into a cellular host,
- culturing the cellular host obtained in the preceding step under conditions enabling the expression of potassium channels of the 2P family.

The cellular host employed in the preceding procedures can be selected from among the prokaryotres or the eukaryotes, and notably from among the bacteria, the yeasts, mammal cells, plant cells or insect cells.

The vector used is selected in relation to the host into which it will be transferred; it can be any vector such as a plasmid.

The invention thus also relates to the transferred cells expressing the potassium channels exhibiting the properties and structure of the type ofthe TWIK-1 channel obtained in accordance with the preceding procedures.

The cells expressing TWIK-1 potassium channels or channels exhibiting the properties and structure of the type of the TWTK-1 channels obtained in accordance with the preceding procedures are useful for the screening of substances capable of modulating the activity of the individual members of the TWIK-1 family of potassium channels. This screening is carried out by bringing into contact variable amounts of a substance to be tested with cells expressing the TWIK-1 channel or potassium channels exhibiting the properties and structure of the type of the TWIK-1 channels, then determining the effects of said substance on the currents of the potassium channels of these channels. This screening procedure makes it possible to identify drugs that may be useful in the treatment of diseases of the heart or of the nervous system. Diseases involving the potassium channels and thus likely to involve the channels of the TWIK-1 family are, for example, epilepsy, heart (arrhythmias) and vascular diseases, neurodegenerative diseases, kidney, liver orpancreas diseases, hypertension, and diseases associated with ischemia or anoxia, the endocrine diseases associated with anomalies of hormone secretion, muscle diseases.

An isolated, purified nucleic acid molecule coding for a protein of the 2P family of potassium channel or a vector including this nucleic acid molecule or a cell expressing the potassium channel polypeptide, are also useful for the preparation of transgenetic animals. These can be animals supra-expressing said channels, but especially so-called knock-out animals, i.e., animals presenting a deficiency of these channels; these transgenetic animals are prepared by methods known to the experts in the field, and enable the preparation of live models for studying animal diseases associated with the 2P family of channels.

The nucleic acid molecules related to the invention or the cells transformed by said molecule can be used in genetic therapy strategies to compensate for a deficiency in the potassium channels at the level of one or more tissues of a patient. The invention thus also relates to a medication containing nucleic acid molecules related to the invention or cells transformed by said molecule for the treatment of disease involving the potassium channels.

In addition, the gene of the TW1K-1 channel has been located on chromosome 1 at position q42-q43. The chromosomal localization of this gene constitutes a determinant result for the identification ofgenetic diseases associated with this new family of potassium channels; thus, the knowledge of the structure of the TWIK-1 family of channels is such as to allow performance of a prenatal diagnosis of such diseases. The TASK1 and TASK2 genes have also been localized on the human genome and are useful for diagnosis of genetic diseases involving these loci.

The present invention also relates to a new family of K+ channels, of which TWIK- 1, TASK1 and TASK2 are members, which polypeptides are present in most human tissues, but especially abundant in the brain, kidney or the heart, and which exhibit the properties and structure of the type of those of the TWIK- 1 channels described above. Thus it relates to an isolated, purified protein whose amino acid sequence is represented in the attached sequence list as number SEQ ID NO: 2, SEQ ID NO: 4, or SEQ ID NO:28, or a functionally equivalent derivative of these sequences. '

Such derivatives can be obtained by modifying and/or suppressing one or more amino acid residues of this sequence or by segmenting this sequence, as long as this modification and/or suppression or deletion of a fragment does not modify the functional properties of the TWIK-1 type potassium channel of the resultant protein.

A protein constituting a 2P potassium channel is useful for the manufacture of medications intended for the treatment or prevention of diseases involving dysfunction of the potassium channels.

Polyclonal or monoclonal antibodies directed against a protein constituting a 2P potassium channel can be prepared by the conventional methods described in the literature. These antibodies are useful for investigating the presence of potassium channels ofthe 2P family in different human or animal tissues, but can also be applied for the *in vivo* inhibition or activation of 2P potassium channels.

SEQUENCE LISTING

<110> Duprat, Fabrice
Lesage, Florian
Fink, Michel
Lazdunski, Michel

<120> FAMILY OF MAMMALIAN POTASSIUM CHANNELS, THEIR CLONING AND THEIR USE, ESPECIALLY FOR THE SCREENING OF DRUGS

<130> 989.6705CIP

<140> 09/144,914
<141> 1998-09-01

<150> 08/749,816
<151> 1996-11-15

<150> 60/095,234
<151> 1998-08-04

<150> FR 96/01565
<151> 1996-02-08

<160> 24

<170> PatentIn Ver. 2.0

<210> 1
<211> 1894
<212> DNA
<213> Homo sapiens

<220>

<221> CDS
<222> (183)..(1190)

<220>
<223> TWIK-1

<400> 1

gggcaggaag acggcgctgc ccggaggagc ggggcgggcg ggcgcgcggg ggagcgggcg 60

gcgggcggga gccaggcccg ggcgggggcg ggggcggcgg ggccagaaga ggcggcgggc 120

cgcgctccgg ccggtctgcg gcgttggcct tggctttggc tttggcggcg gcggtggaga 180

ag atg ctg cag tcc ctg gcc ggc agc tcg tgc gtg cgc ctg gtg gag 227
Met Leu Gln Ser Leu Ala Gly Ser Ser Cys Val Arg Leu Val Glu
1 5 10 15

cgg cac cgc tcg gcc tgg tgc ttc ggc ttc ctg gtg ctg ggc tac ttg 275
Arg His Arg Ser Ala Trp Cys Phe Gly Phe Leu Val Leu Gly Tyr Leu
20 25 30

ctc tac ctg gtc ttc ggc gca gtg gtc ttc tcc tcg gtg gag ctg ccc 323
Leu Tyr Leu Val Phe Gly Ala Val Val Phe Ser Ser Val Glu Leu Pro
35 40 45

```
tat gag gac ctg ctg cgc cag gag ctg cgc aag ctg aag cga cgc ttc   371
Tyr Glu Asp Leu Leu Arg Gln Glu Leu Arg Lys Leu Lys Arg Arg Phe
         50                  55                  60

ttg gag gag cac gag tgc ctg tct gag cag cag ctg gag cag ttc ctg   419
Leu Glu Glu His Glu Cys Leu Ser Glu Gln Gln Leu Glu Gln Phe Leu
     65                  70                  75

ggc cgg gtg ctg gag gcc agc aac tac ggc gtg tcg gtg ctc agc aac   467
Gly Arg Val Leu Glu Ala Ser Asn Tyr Gly Val Ser Val Leu Ser Asn
 80                  85                  90                  95

gcc tcg ggc aac tgg aac tgg gac ttc acc tcc gcg ctc ttc ttc gcc   515
Ala Ser Gly Asn Trp Asn Trp Asp Phe Thr Ser Ala Leu Phe Phe Ala
                100                 105                 110

agc acc gtg ctc tcc acc aca ggt tat ggc cac acc gtg ccc ttg tca   563
Ser Thr Val Leu Ser Thr Thr Gly Tyr Gly His Thr Val Pro Leu Ser
            115                 120                 125

gat gga ggt aag gcc ttc tgc atc atc tac tcc gtc att ggc att ccc   611
Asp Gly Gly Lys Ala Phe Cys Ile Ile Tyr Ser Val Ile Gly Ile Pro
        130                 135                 140

ttc acc ctc ctg ttc ctg acg gct gtg gtc cag cgc atc acc gtg cac   659
Phe Thr Leu Leu Phe Leu Thr Ala Val Val Gln Arg Ile Thr Val His
    145                 150                 155

gtc acc cgc agg ccg gtc ctc tac ttc cac atc cgc tgg ggc ttc tcc   707
Val Thr Arg Arg Pro Val Leu Tyr Phe His Ile Arg Trp Gly Phe Ser
160                 165                 170                 175

aag cag gtg gtg gcc atc gtc cat gcc gtg ctc ctt ggg ttt gtc act   755
Lys Gln Val Val Ala Ile Val His Ala Val Leu Leu Gly Phe Val Thr
                180                 185                 190

gtg tcc tgc ttc ttc ttc atc ccg gcc gct gtc ttc tca gtc ctg gag   803
Val Ser Cys Phe Phe Phe Ile Pro Ala Ala Val Phe Ser Val Leu Glu
            195                 200                 205

gat gac tgg aac ttc ctg gaa tcc ttt tat ttt tgt ttt att tcc ctg   851
Asp Asp Trp Asn Phe Leu Glu Ser Phe Tyr Phe Cys Phe Ile Ser Leu
        210                 215                 220

agc acc att ggc ctg ggg gat tat gtg cct ggg gaa ggc tac aat caa   899
Ser Thr Ile Gly Leu Gly Asp Tyr Val Pro Gly Glu Gly Tyr Asn Gln
    225                 230                 235

aaa ttc aga gag ctc tat aag att ggg atc acg tgt tac ctg cta ctt   947
Lys Phe Arg Glu Leu Tyr Lys Ile Gly Ile Thr Cys Tyr Leu Leu Leu
240                 245                 250                 255

ggc ctt att gcc atg ttg gta gtt ctg gaa acc ttc tgt gaa ctc cat   995
Gly Leu Ile Ala Met Leu Val Val Leu Glu Thr Phe Cys Glu Leu His
                260                 265                 270
```

```
gag ctg aaa aaa ttc aga aaa atg ttc tat gtg aag aag gac aag gac   1043
Glu Leu Lys Lys Phe Arg Lys Met Phe Tyr Val Lys Lys Asp Lys Asp
        275                 280                 285

gag gat cag gtg cac atc ata gag cat gac caa ctg tcc ttc tcc tcg   1091
Glu Asp Gln Val His Ile Ile Glu His Asp Gln Leu Ser Phe Ser Ser
    290                 295                 300

atc aca gac cag gca gct ggc atg aaa gag gac cag aag caa aat gag   1139
Ile Thr Asp Gln Ala Ala Gly Met Lys Glu Asp Gln Lys Gln Asn Glu
305                 310                 315

cct ttt gtg gcc acc cag tca tct gcc tgc gtg gat ggc cct gca aac   1187
Pro Phe Val Ala Thr Gln Ser Ser Ala Cys Val Asp Gly Pro Ala Asn
320                 325                 330                 335

cat tgagcgtagg atttgttgca ttatgctaga gcaccagggt cagggtgcaa        1240
His

ggaagaggct taagtatgtt catttttatc agaatgcaaa agcgaaaatt atgtcacttt 1300

aagaaatagc tactgtttgc aatgtcttat taaaaaacaa caaaaaaaga cacatggaac 1360

aaagaagctg tgaccccagc aggatgtcta atatgtgagg aaatgagatg tccacctaaa 1420

attcatatgt gacaaaatta tctcgacctt acataggagg agaatacttg aagcagtatg 1480

ctgctgtggt tagaagcaga ttttatactt ttaactggaa actttggggt ttgcatttag 1540

atcatttagc tgatggctaa atagcaaaat ttatatttag aagcaaaaaa aaaaagcata 1600

gagatctgtt ttataaatag gtttatgtgt actggtttgc atgtacccac ccaaaatgat 1660

tatttttgga gaatctaagt caaactcact atttataatg cataggtaac cattaactat 1720

gtacatataa agtataaata tgtttatatt ctgtacatat ggtttaggtc accagatcct 1780

agtgtagttc tgaaactaag actatagata ttttgtttct tttgatttct ctttatacta 1840

aagaatccag agttgctaca ataaaataag gggaataata aaaaaaaaaa aaaa       1894
```

<210> 2
<211> 336
<212> PRT
<213> Homo sapiens

<220>
<223> TWIK-1

<400> 2

```
Met Leu Gln Ser Leu Ala Gly Ser Ser Cys Val Arg Leu Val Glu Arg
1               5                   10                  15

His Arg Ser Ala Trp Cys Phe Gly Phe Leu Val Leu Gly Tyr Leu Leu
            20                  25                  30
```

Tyr Leu Val Phe Gly Ala Val Val Phe Ser Ser Val Glu Leu Pro Tyr
35 40 45

Glu Asp Leu Leu Arg Gln Glu Leu Arg Lys Leu Lys Arg Arg Phe Leu
50 55 60

Glu Glu His Glu Cys Leu Ser Glu Gln Gln Leu Glu Gln Phe Leu Gly
65 70 75 80

Arg Val Leu Glu Ala Ser Asn Tyr Gly Val Ser Val Leu Ser Asn Ala
85 90 95

Ser Gly Asn Trp Asn Trp Asp Phe Thr Ser Ala Leu Phe Phe Ala Ser
100 105 110

Thr Val Leu Ser Thr Thr Gly Tyr Gly His Thr Val Pro Leu Ser Asp
115 120 125

Gly Gly Lys Ala Phe Cys Ile Ile Tyr Ser Val Ile Gly Ile Pro Phe
130 135 140

Thr Leu Leu Phe Leu Thr Ala Val Val Gln Arg Ile Thr Val His Val
145 150 155 160

Thr Arg Arg Pro Val Leu Tyr Phe His Ile Arg Trp Gly Phe Ser Lys
165 170 175

Gln Val Val Ala Ile Val His Ala Val Leu Leu Gly Phe Val Thr Val
180 185 190

Ser Cys Phe Phe Phe Ile Pro Ala Ala Val Phe Ser Val Leu Glu Asp
195 200 205

Asp Trp Asn Phe Leu Glu Ser Phe Tyr Phe Cys Phe Ile Ser Leu Ser
210 215 220

Thr Ile Gly Leu Gly Asp Tyr Val Pro Gly Glu Gly Tyr Asn Gln Lys
225 230 235 240

Phe Arg Glu Leu Tyr Lys Ile Gly Ile Thr Cys Tyr Leu Leu Leu Gly
245 250 255

Leu Ile Ala Met Leu Val Val Leu Glu Thr Phe Cys Glu Leu His Glu
260 265 270

Leu Lys Lys Phe Arg Lys Met Phe Tyr Val Lys Lys Asp Lys Asp Glu
275 280 285

Asp Gln Val His Ile Ile Glu His Asp Gln Leu Ser Phe Ser Ser Ile
290 295 300

Thr Asp Gln Ala Ala Gly Met Lys Glu Asp Gln Lys Gln Asn Glu Pro
305 310 315 320

Phe Val Ala Thr Gln Ser Ser Ala Cys Val Asp Gly Pro Ala Asn His
325 330 335

<210> 3

<211> 2514
<212> DNA
<213> Homo sapiens

<220>
<221> CDS
<222> (126)..(1307)

<220>
<223> TASK

<400> 3

```
tgccctgcgc ggatagcggc gagcgcagcc atgccccagg ccgcctccgg ggcagcagca 60

gcggcggccg gggccgatgc gcgggccggg ggcgccgggg ggccggcggc ggcccgggcg 120

ggacg atg aag cgg cag aac gtg cgc acg ctg gcg ctc atc gtg tgc acc 170
      Met Lys Arg Gln Asn Val Arg Thr Leu Ala Leu Ile Val Cys Thr
      1               5                   10                  15

ttc acc tac ctg ctg gtg ggc gcc gcg gtc ttc gac gcg ctg gag tcg   218
Phe Thr Tyr Leu Leu Val Gly Ala Ala Val Phe Asp Ala Leu Glu Ser
                20                  25                  30

gag ccc gag ctg atc gag cgg cag cgg ctg gag ctg cgg cag cag gag   266
Glu Pro Glu Leu Ile Glu Arg Gln Arg Leu Glu Leu Arg Gln Gln Glu
            35                  40                  45

ctg cgg gcg cgc tac aac ctc agc cag ggc ggc tac gag gag ctg gag   314
Leu Arg Ala Arg Tyr Asn Leu Ser Gln Gly Gly Tyr Glu Glu Leu Glu
        50                  55                  60

cgc gtc gtg ctg cgc ctc aag ccg cac aag gcc ggc gtg cag tgg cgc   362
Arg Val Val Leu Arg Leu Lys Pro His Lys Ala Gly Val Gln Trp Arg
    65                  70                  75

ttc gcc ggc tcc ttc tac ttc gcc atc acc gtc atc acc acc atc ggc   410
Phe Ala Gly Ser Phe Tyr Phe Ala Ile Thr Val Ile Thr Thr Ile Gly
80                  85                  90                  95

tac ggg cac gcg gca ccc agc acg gat ggc ggc aag gtg ttc tgc atg   458
Tyr Gly His Ala Ala Pro Ser Thr Asp Gly Gly Lys Val Phe Cys Met
                100                 105                 110

ttc tac gcg ctg ctg ggc atc ccg ctc acg ctc gtc atg ttc cag agc   506
Phe Tyr Ala Leu Leu Gly Ile Pro Leu Thr Leu Val Met Phe Gln Ser
            115                 120                 125

ctg ggc gag cgc atc aac acc ctg gtg agg tac ctg ctg cac cgc gcc   554
Leu Gly Glu Arg Ile Asn Thr Leu Val Arg Tyr Leu Leu His Arg Ala
        130                 135                 140

aag aag ggg ctg ggc atg cgg cgc gcc gac gtg tcc atg gcc aac atg   602
Lys Lys Gly Leu Gly Met Arg Arg Ala Asp Val Ser Met Ala Asn Met
    145                 150                 155
```

```
gtg ctc atc ggc ttc ttc tcg tgc atc agc acg ctg tgc atc ggc gcc     650
Val Leu Ile Gly Phe Phe Ser Cys Ile Ser Thr Leu Cys Ile Gly Ala
160                 165                 170                 175

gcc gcc ttc tcc cac tac gag cac tgg acc ttc ttc cag gcc tac tac     698
Ala Ala Phe Ser His Tyr Glu His Trp Thr Phe Phe Gln Ala Tyr Tyr
                180                 185                 190

tac tgc ttc atc acc ctc acc acc atc ggc ttc ggc gac tac gtg gcg     746
Tyr Cys Phe Ile Thr Leu Thr Thr Ile Gly Phe Gly Asp Tyr Val Ala
            195                 200                 205

ctg cag aag gac cag gcc ctg cag acg cag ccg cag tac gtg gcc ttc     794
Leu Gln Lys Asp Gln Ala Leu Gln Thr Gln Pro Gln Tyr Val Ala Phe
        210                 215                 220

agc ttc gtc tac atc ctt acg ggc ctc acg gtc atc ggc gcc ttc ctc     842
Ser Phe Val Tyr Ile Leu Thr Gly Leu Thr Val Ile Gly Ala Phe Leu
    225                 230                 235

aac ctc gtg gtg ctg cgc ttc atg acc atg aac gcc gag gac gag aag     890
Asn Leu Val Val Leu Arg Phe Met Thr Met Asn Ala Glu Asp Glu Lys
240                 245                 250                 255

cgc gac gcc gag cac cgc gcg ctg ctc acg cgc aac ggg cag gcg ggc     938
Arg Asp Ala Glu His Arg Ala Leu Leu Thr Arg Asn Gly Gln Ala Gly
                260                 265                 270

ggc ggc gga ggg ggt ggc agc gcg cac act acg gac acc gcc tca tcc     986
Gly Gly Gly Gly Gly Gly Ser Ala His Thr Thr Asp Thr Ala Ser Ser
            275                 280                 285

acg gcg gca gcg ggc ggc ggc ggc ttc cgc aac gtc tac gcg gag gtg     1034
Thr Ala Ala Ala Gly Gly Gly Gly Phe Arg Asn Val Tyr Ala Glu Val
        290                 295                 300

ctg cac ttc cag tcc atg tgc tcg tgc ctg tgg tac aag agc cgc gag     1082
Leu His Phe Gln Ser Met Cys Ser Cys Leu Trp Tyr Lys Ser Arg Glu
    305                 310                 315

aag ctg cag tac tcc atc ccc atg atc atc ccg cgg gac ctc tcc acg     1130
Lys Leu Gln Tyr Ser Ile Pro Met Ile Ile Pro Arg Asp Leu Ser Thr
320                 325                 330                 335

tcc gac acg tgc gtg gag cag agc cac tcg tcg ccg gga ggg ggc ggc     1178
Ser Asp Thr Cys Val Glu Gln Ser His Ser Ser Pro Gly Gly Gly Gly
                340                 345                 350

cgc tac agc gac acg ccc tcg cga cgc tgc ctg tgc agc ggg gcg cca     1226
Arg Tyr Ser Asp Thr Pro Ser Arg Arg Cys Leu Cys Ser Gly Ala Pro
            355                 360                 365

cgc tcc gcc atc agc tcg gtg tcc acg ggt ctg cac agc ctg tcc acc     1274
Arg Ser Ala Ile Ser Ser Val Ser Thr Gly Leu His Ser Leu Ser Thr
        370                 375                 380
```

```
ttc cgc ggc ctc atg aag cgc agg agc tcc gtg tgactgcccc gagggacctg 1327
Phe Arg Gly Leu Met Lys Arg Arg Ser Ser Val
    385                 390

gagcacctgg gggcgcgggc gggggacccc tgctgggagg ccaggagact gcccctgctg 1387

ccttctgccc agtgggaccc cgcacaacat ccctcaccac tctcccccag cacccccatc 1447

tccgactgtg cctgcttgca ccagccggca ggaggccggg ctctgaggac ccctggggcc 1507

cccatcggag ccctgcaaat tccgagaaat gtgaaacttg gtggggtcag ggaggaaagg 1567

cagaagctgg gagcctccct tccctttgaa aatctaagaa gctcccagtc ctcagagacc 1627

ctgctggtac cacaccccac cttcggaggg gacttcatgt tccgtgtacg tttgcatctc 1687

tatttatacc tctgtcctgc taggtctccc accttccctt ggttccaaaa gccagggtgt 1747

ctatgtccaa gtcaccccta ctcagcccca ctccccttcc tcatccccag ctgtgtctcc 1807

caacctccct tcgtgttgtt ttgcatggct ttgcagttat ggagaaagtg gaaacccagc 1867

agtccctaaa gctggtcccc agaaagcagg acagaaagaa ggagggacag gcaggcagca 1927

ggagggggcga gctgggaggc aggaggcagc ggcctgtcag tctgcagaat ggtcgcactg 1987

gaggttcaag ctaactggcc tccagccaca ttctcatagc aggtaggact tcagccttcc 2047

agacactgcc cttagaatct ggaacagaag acttcagact caccataatt gctgataatt 2107

acccactctt aaatttgtcg agtgattttt agcctctgaa aactctatgc tggccactga 2167

ttccttttgag tctcacaaaa ccctacttag gtcatcaggg caggagttct cactcccatt 2227

ttacagatga acctgtattc ccaacacttt tggaggctga ggttggagga ttgcttgagc 2287

ccaggaattc gagaccagcc taggtgacat agtgagaccc catctctaca aaaaataaaa 2347

aattaaccag gtgtggtggc acgtgcctgg gagtcccagc gacttgggag gctgaggtgg 2407

gaggattgtt tgagcctggg aggtcgaggc tgtagtgagc cctgattgca ccactgtact 2467

ccagcctggg tgacagggca agaccctgtc tcaaaaaaaa aaaaaaa              2514
```

<210> 4
<211> 394
<212> PRT
<213> Homo sapiens

<220>
<223> TASK

<400> 4

```
Met Lys Arg Gln Asn Val Arg Thr Leu Ala Leu Ile Val Cys Thr Phe
1               5                   10                  15
```

Thr Tyr Leu Leu Val Gly Ala Ala Val Phe Asp Ala Leu Glu Ser Glu
20 25 30

Pro Glu Leu Ile Glu Arg Gln Arg Leu Glu Leu Arg Gln Gln Glu Leu
35 40 45

Arg Ala Arg Tyr Asn Leu Ser Gln Gly Gly Tyr Glu Glu Leu Glu Arg
50 55 60

Val Val Leu Arg Leu Lys Pro His Lys Ala Gly Val Gln Trp Arg Phe
65 70 75 80

Ala Gly Ser Phe Tyr Phe Ala Ile Thr Val Ile Thr Thr Ile Gly Tyr
85 90 95

Gly His Ala Ala Pro Ser Thr Asp Gly Gly Lys Val Phe Cys Met Phe
100 105 110

Tyr Ala Leu Leu Gly Ile Pro Leu Thr Leu Val Met Phe Gln Ser Leu
115 120 125

Gly Glu Arg Ile Asn Thr Leu Val Arg Tyr Leu Leu His Arg Ala Lys
130 135 140

Lys Gly Leu Gly Met Arg Arg Ala Asp Val Ser Met Ala Asn Met Val
145 150 155 160

Leu Ile Gly Phe Phe Ser Cys Ile Ser Thr Leu Cys Ile Gly Ala Ala
165 170 175

Ala Phe Ser His Tyr Glu His Trp Thr Phe Phe Gln Ala Tyr Tyr Tyr
180 185 190

Cys Phe Ile Thr Leu Thr Thr Ile Gly Phe Gly Asp Tyr Val Ala Leu
195 200 205

Gln Lys Asp Gln Ala Leu Gln Thr Gln Pro Gln Tyr Val Ala Phe Ser
210 215 220

Phe Val Tyr Ile Leu Thr Gly Leu Thr Val Ile Gly Ala Phe Leu Asn
225 230 235 240

Leu Val Val Leu Arg Phe Met Thr Met Asn Ala Glu Asp Glu Lys Arg
245 250 255

Asp Ala Glu His Arg Ala Leu Leu Thr Arg Asn Gly Gln Ala Gly Gly
260 265 270

Gly Gly Gly Gly Gly Ser Ala His Thr Thr Asp Thr Ala Ser Ser Thr
275 280 285

Ala Ala Ala Gly Gly Gly Gly Phe Arg Asn Val Tyr Ala Glu Val Leu
290 295 300

His Phe Gln Ser Met Cys Ser Cys Leu Trp Tyr Lys Ser Arg Glu Lys
305 310 315 320

Leu Gln Tyr Ser Ile Pro Met Ile Ile Pro Arg Asp Leu Ser Thr Ser
325 330 335

Asp Thr Cys Val Glu Gln Ser His Ser Ser Pro Gly Gly Gly Gly Arg
340 345 350

Tyr Ser Asp Thr Pro Ser Arg Arg Cys Leu Cys Ser Gly Ala Pro Arg
355 360 365

Ser Ala Ile Ser Ser Val Ser Thr Gly Leu His Ser Leu Ser Thr Phe
370 375 380

Arg Gly Leu Met Lys Arg Arg Ser Ser Val
385 390

<210> 5
<211> 405
<212> PRT
<213> Murine

<220>
<223> TASK

<400> 5

Glu Asn Val Arg Thr Leu Ala Leu Ile Val Cys Thr Phe Thr Tyr Leu
1 5 10 15

Leu Val Gly Ala Ala Val Phe Asp Ala Leu Glu Ser Glu Pro Glu Met
20 25 30

Ile Glu Arg Gln Arg Leu Glu Leu Arg Gln Leu Glu Leu Arg Ala Arg
35 40 45

Tyr Asn Leu Ser Glu Gly Gly Tyr Glu Glu Leu Glu Arg Val Val Leu
50 55 60

Arg Leu Lys Pro His Lys Ala Gly Val Gln Trp Arg Phe Ala Gly Ser
65 70 75 80

Phe Tyr Phe Ala Ile Thr Val Ile Thr Thr Ile Gly Tyr Gly His Ala
85 90 95

Ala Pro Ser Thr Asp Gly Gly Lys Val Phe Cys Met Phe Tyr Ala Leu
100 105 110

Leu Gly Ile Pro Leu Thr Leu Ile Met Phe Gln Ser Leu Gly Glu Arg
115 120 125

Ile Asn Thr Phe Val Arg Tyr Leu Leu His Arg Ala Lys Arg Gly Leu
130 135 140

Gly Met Arg His Ala Glu Val Ser Met Ala Asn Met Val Leu Ile Gly
145 150 155 160

Phe Val Ser Cys Ile Ser Thr Leu Cys Ile Gly Ala Ala Ala Phe Ser
165 170 175

Tyr Tyr Glu Arg Trp Thr Phe Phe Gln Ala Tyr Tyr Tyr Cys Phe Ile
180 185 190

Thr Leu Thr Thr Ile Gly Phe Gly Asp Tyr Val Ala Leu Gln Lys Asp
195 200 205

Gln Ala Leu Gln Thr Gln Pro Gln Tyr Val Ala Phe Ser Phe Val Tyr
210 215 220

Ile Leu Thr Gly Leu Thr Val Ile Gly Ala Phe Leu Asn Leu Val Val
225 230 235 240

Leu Arg Phe Met Thr Met Asn Ala Glu Asp Glu Lys Arg Asp Ala Glu
245 250 255

His Arg Ala Leu Leu Thr His Asn Gly Gln Ala Val Gly Leu Gly Gly
260 265 270

Leu Ser Cys Leu Ser Gly Ser Leu Gly Asp Val Arg Pro Arg Asp Pro
275 280 285

Val Thr Cys Ala Ala Ala Ala Gly Gly Val Gly Val Gly Val Gly Gly
290 295 300

Ser Gly Phe Arg Asn Val Tyr Ala Glu Val Leu His Phe Gln Ser Met
305 310 315 320

Cys Ser Cys Leu Trp Tyr Lys Ser Arg Glu Lys Leu Gln Tyr Ser Ile
325 330 335

Pro Met Ile Ile Pro Arg Asp Leu Ser Thr Ser Asp Thr Cys Val Glu
340 345 350

His Ser His Ser Ser Pro Gly Gly Gly Gly Arg Tyr Ser Asp Thr Pro
355 360 365

Ser His Pro Cys Leu Cys Ser Gly Thr Gln Arg Ser Ala Ile Ser Ser
370 375 380

Val Ser Thr Gly Leu His Ser Leu Ala Ala Phe Arg Gly Leu Met Lys
385 390 395 400

Arg Arg Ser Ser Val
405

<210> 6
<211> 347
<212> PRT
<213> Unknown

<220>
<223> Description of Unknown Organism: TWIK-1 homolog

<400> 6

Met Tyr Thr Asp Glu Gly Glu Tyr Ser Gly Asp Thr Asp His Gly Gly
1 5 10 15

```
Ser Thr Met Gln Lys Met Ser Pro Asn Thr Arg Gln Asn Phe Arg Gln
            20                  25                  30

Asn Val Asn Val Val Val Cys Leu Ser Ala Ala Ile Thr Leu Leu Val
        35                  40                  45

Phe Asn Leu Ile Gly Ala Gly Ile Phe Tyr Leu Ala Glu Thr Gln Asn
    50                  55                  60

Ser Ser Glu Ser Leu Asn Glu Asn Ser Glu Val Ser Lys Cys Leu His
65                  70                  75                  80

Asn Leu Pro Ile Gly Gly Lys Ile Thr Ala Glu Met Lys Ser Lys Leu
                85                  90                  95

Gly Lys Cys Leu Thr Lys Ser Ser Arg Ile Asp Gly Phe Gly Lys Ala
            100                 105                 110

Ile Phe Phe Ser Trp Thr Leu Tyr Ser Thr Val Gly Tyr Gly Ser Leu
        115                 120                 125

Tyr Pro His Ser Thr Leu Gly Arg Tyr Leu Thr Ile Phe Tyr Ser Leu
    130                 135                 140

Leu Met Ile Pro Val Phe Ile Ala Phe Lys Phe Glu Phe Gly Thr Phe
145                 150                 155                 160

Leu Ala His Phe Leu Val Val Val Ser Asn Arg Thr Arg Leu Ala Val
                165                 170                 175

Lys Lys Ala Tyr Tyr Lys Leu Ser Gln Asn Pro Glu Asn Ala Glu Thr
            180                 185                 190

Pro Ser Asn Ser Leu Gln His Asp Tyr Leu Ile Phe Leu Ser Ser Leu
        195                 200                 205

Leu Leu Cys Ser Ile Ser Leu Leu Ser Ser Ser Ala Leu Phe Ser Ser
    210                 215                 220

Ile Glu Asn Ile Ser Tyr Leu Ser Ser Val Tyr Phe Gly Ile Ile Thr
225                 230                 235                 240

Met Phe Leu Ile Gly Ile Gly Asp Ile Val Pro Thr Asn Leu Val Trp
                245                 250                 255

Phe Ser Gly Tyr Cys Met Leu Phe Leu Ile Ser Asp Val Leu Ser Asn
            260                 265                 270

Gln Ile Phe Tyr Phe Cys Gln Ala Arg Val Arg Tyr Phe Phe His Ile
        275                 280                 285

Leu Ala Arg Lys Ile Leu Leu Leu Arg Glu Glu Asp Asp Gly Phe Gln
    290                 295                 300

Leu Glu Thr Thr Val Ser Leu Gln His Ile Pro Ile Ile Asn Ser Gln
305                 310                 315                 320
```

Cys Met Pro Ser Leu Val Leu Asp Cys Glu Lys Glu Glu Leu Asp Asn
325 330 335

Asp Glu Lys Leu Ile Ser Ser Leu Thr Ser Thr
340 345

<210> 7
<211> 383
<212> PRT
<213> Unknown

<220>
<223> Description of Unknown Organism: TWIK-1 homolog

<400> 7

Met Thr Val Ser Met Glu Glu Asn Ser Lys Ile Gln Met Leu Ser Ala
1               5                   10                  15

Thr Ser Lys Asp Lys Lys Val Ala Thr Asp Arg Ser Leu Leu Asn Lys
            20                  25                  30

Tyr His Leu Gly Pro Leu Ala Leu His Thr Gly Leu Val Leu Ser Cys
        35                  40                  45

Val Thr Tyr Ala Leu Gly Gly Ala Tyr Leu Phe Leu Ser Ile Glu His
    50                  55                  60

Pro Glu Glu Leu Lys Arg Arg Glu Lys Ala Ile Arg Glu Phe Gln Asp
65                  70                  75                  80

Leu Lys Gln Gln Phe Met Gly Asn Ile Thr Ser Gly Ile Glu Asn Ser
                85                  90                  95

Glu Gln Ser Ile Glu Ile Tyr Thr Lys Lys Leu Ile Leu Met Leu Glu
            100                 105                 110

Asp Ala His Asn Ala His Ala Phe Glu Tyr Phe Phe Leu Asn His Glu
        115                 120                 125

Ile Pro Lys Asp Met Trp Thr Phe Ser Ser Ala Leu Val Phe Thr Thr
    130                 135                 140

Thr Thr Val Ile Pro Val Gly Tyr Gly Tyr Ile Phe Pro Val Ser Ala
145                 150                 155                 160

Tyr Gly Arg Met Cys Leu Ile Ala Tyr Ala Leu Leu Gly Ile Pro Leu
                165                 170                 175

Thr Leu Val Thr Met Ala Asp Thr Gly Lys Phe Ala Ala Gln Leu Val
            180                 185                 190

Thr Arg Trp Phe Gly Asp Asn Asn Met Ala Ile Pro Ala Ala Ile Phe
        195                 200                 205

Val Cys Leu Leu Phe Ala Tyr Pro Leu Val Val Gly Phe Ile Leu Cys
    210                 215                 220

Ser Thr Ser Asn Ile Thr Tyr Leu Asp Ser Val Tyr Phe Ser Leu Thr
225 230 235 240

Ser Ile Phe Thr Ile Gly Phe Gly Asp Leu Thr Pro Asp Met Asn Val
245 250 255

Ile His Met Val Leu Phe Leu Ala Val Gly Val Ile Leu Val Thr Ile
260 265 270

Thr Leu Asp Ile Val Ala Ala Glu Met Ile Asp Arg Val His Tyr Met
275 280 285

Gly Arg His Val Gly Lys Ala Lys Glu Leu Ala Gly Lys Met Phe Gln
290 295 300

Leu Ala Gln Ser Leu Asn Met Lys Gln Gly Leu Val Ser Gly Val Gly
305 310 315 320

Gln Leu His Ala Leu Ala Arg Phe Gly Met Leu Val Gly Arg Glu Glu
325 330 335

Val Asp Lys Thr Gln Glu Asp Gly Ile Ile Ala Phe Ser Pro Asp Val
340 345 350

Met Asp Gly Leu Glu Phe Met Asp Thr Leu Ser Ile Tyr Ser Arg Arg
355 360 365

Ser Arg Arg Ser Ala Glu Asn Ser Ala Arg Asn Leu Phe Leu Ser
370 375 380

<210> 8
<211> 370
<212> PRT
<213> Murine

<220>
<223> TREK-1

<400> 8

Met Ala Ala Pro Asp Leu Leu Asp Pro Lys Ser Ala Ala Gln Asn Ser
1 5 10 15

Lys Pro Arg Leu Ser Phe Ser Ser Lys Pro Thr Val Leu Ala Ser Arg
20 25 30

Val Glu Ser Asp Ser Ala Ile Asn Val Met Lys Trp Lys Thr Val Ser
35 40 45

Thr Ile Phe Leu Val Val Val Leu Tyr Leu Ile Ile Gly Ala Ala Val
50 55 60

Phe Lys Ala Leu Glu Gln Pro Gln Glu Ile Ser Gln Arg Thr Thr Ile
65 70 75 80

Val Ile Gln Lys Gln Thr Phe Ile Ala Gln His Ala Cys Val Asn Ser
85 90 95

Thr Glu Leu Asp Glu Leu Ile Gln Gln Ile Val Ala Ala Ile Asn Ala
100 105 110

Gly Ile Ile Pro Leu Gly Asn Ser Ser Asn Gln Val Ser His Trp Asp
115 120 125

Leu Gly Ser Ser Phe Phe Phe Ala Gly Thr Val Ile Thr Thr Ile Gly
130 135 140

Phe Gly Asn Ile Ser Pro Arg Thr Glu Gly Gly Lys Ile Phe Cys Ile
145 150 155 160

Ile Tyr Ala Leu Leu Gly Ile Pro Leu Glu Gly Phe Leu Leu Ala Gly
165 170 175

Val Gly Asp Gln Leu Gly Thr Ile Phe Gly Lys Gly Ile Ala Lys Val
180 185 190

Glu Asp Thr Phe Ile Lys Trp Asn Val Ser Gln Thr Lys Ile Arg Ile
195 200 205

Ile Ser Thr Ile Ile Phe Ile Leu Phe Gly Cys Val Leu Phe Val Ala
210 215 220

Leu Pro Ala Val Ile Phe Lys His Ile Glu Gly Trp Ser Ala Leu Asp
225 230 235 240

Ala Ile Tyr Phe Val Val Ile Thr Leu Thr Thr Ile Gly Phe Gly Asp
245 250 255

Tyr Val Ala Gly Gly Ser Asp Ile Glu Tyr Leu Asp Phe Tyr Lys Pro
260 265 270

Val Val Trp Phe Trp Ile Leu Val Gly Leu Ala Tyr Phe Ala Ala Val
275 280 285

Leu Ser Met Ile Gly Asp Trp Leu Arg Val Ile Ser Lys Lys Thr Lys
290 295 300

Glu Glu Val Gly Glu Phe Arg Ala His Ala Ala Glu Trp Thr Ala Asn
305 310 315 320

Val Thr Ala Glu Phe Lys Glu Thr Arg Arg Arg Leu Ser Val Glu Ile
325 330 335

Tyr Asp Lys Phe Gln Arg Ala Thr Ser Val Lys Arg Lys Leu Ser Ala
340 345 350

Glu Leu Ala Gly Asn His Asn Gln Glu Leu Thr Pro Cys Met Arg Thr
355 360 365

Cys Leu
370

<210> 9
<211> 27
<212> PRT
<213> Homo sapiens

<220>
<223> TWIK-1 P1

<400> 9

Phe Thr Ser Ala Leu Phe Phe Ala Ser Thr Val Leu Ser Thr Thr Gly
1               5                   10                  15

Tyr Gly His Thr Val Pro Leu Ser Asp Gly Gly
            20                  25

<210> 10
<211> 27
<212> PRT
<213> Homo sapiens

<220>
<223> TWIK-1 P2

<400> 10

Phe Leu Glu Ser Phe Tyr Phe Cys Phe Ile Ser Leu Ser Thr Ile Gly
1               5                   10                  15

Leu Gly Asp Tyr Val Pro Gly Glu Gly Tyr Asn
            20                  25

<210> 11
<211> 27
<212> PUT
<213> Unknown

<220>
<223> Description of Unknown Organism: P domain of representative $K^+$ channel sequence

<220>
<223> TOK-1 P2

<400> 11

Tyr Phe Asn Cys Ile Tyr Phe Cys Phe Leu Cys Leu Leu Thr Ile Gly
1               5                   10                  15

Tyr Gly Asp Tyr Ala Pro Arg Thr Gly Ala Gly
            20                  25

<210> 12
<211> 27
<212> PRT
<213> Unknown

<220>
<223> Description of Unknown Organism: P domain of representative K+channel sequence

<220>
<223> TOK-1 P1

<400> 12

Tyr Gly Asn Ala Leu Tyr Phe Cys Thr Val Ser Leu Leu Thr Val Gly
1 5 10 15

Leu Gly Asp Ile Leu Pro Lys Ser Val Gly Ala
20 25

<210> 13
<211> 27
<212> PRT
<213> Unknown

<220>
<223> Description of unknown Organism: P domain of representative K+ channel sequence

<220>
c223> Slo

<400> 13

Tyr Trp Thr Cys Val Tyr Phe Leu Ile Val Thr Met Ser Thr Val Gly
1 5 10 15

Tyr Gly Asp Val Tyr Cys Glu Thr Val Leu Gly
20 25

<210> 14
<211> 27
<212> PRT
<213> Unknown

<220>
<223> Description of Unknown Organism: P domain of representative K+ channel sequence

<220>
<223> Shaker

<400> 14

Ile Pro Asp Ala Phe Trp Trp Ala Val Val Thr Met Thr Thr Val Gly
1 5 10 15

Tyr Gly Asp Met Thr Pro Val Gly Phe Trp Gly
20 25

<210> 15
<211> 27
<212> PRT
<213> Unknown

<220>

<223> Description of Unknown Organism: P domain of representative K+ channel sequence

<220>
<223> Shab

<400> 15

```
Ile Pro Glu Ala Phe Trp Trp Ala Gly Ile Thr Met Thr Thr Val Gly
 1               5                  10                  15

Tyr Gly Asp Ile Cys Pro Thr Thr Ala Leu Gly
             20                  25
```

<210> 16
<211> 27
<212> PRT
<213> Unknown

<220>
<223> Description of Unknown Organism: P domain of representative K+ channel sequence

<220>
<223> Sha1

<400> 16

```
Ile Pro Ala Ala Phe Trp Tyr Thr Ile Val Thr Met Thr Thr Leu Gly
 1               5                  10                  15

Tyr Gly Asp Met Val Pro Glu Thr Ile Ala Gly
             20                  25
```

<210> 17
<211> 27
<212> PRT
<213> Unknown

<220>
<223> Description of Unknown Organism: P domain of representative K+ channel sequence

<220>
<223> Shaw

<400> 17

```
Ile Pro Leu Gly Leu Trp Trp Ala Leu Val Thr Met Thr Thr Val Gly
 1               5                  10                  15

Tyr Gly Asp Met Ala Pro Lys Thr Tyr Ile Gly
             20                  25
```

<210> 18
<211> 27
<212> PRT
<213> Unknown

<220>
<223> Description of Unknown Organism: P domain of representative k+ channel sequence

<220>
<223> KAT1

<40C> 18

```
Tyr Val Thr Ala Leu Tyr Trp Ser Ile Thr Thr Leu Thr Thr Thr Gly
 1               5                  10                  15

Tyr Gly Asp Phe His Ala Glu Asn Pro Arg Glu
            20                  25
```

<210> 19
<211> 27
<212> PRT
<213> Unknown

<220>
<223> Description of Unknown Organism: P domain of representative K+ channel sequence

<220>
<223> AKT1

<400> 19

```
Tyr Val Thr Ser Met Tyr Trp Ser Ile Thr Thr Leu Thr Thr Val Gly
 1               5                  10                  15

Tyr Gly Asp Ile His Pro Val Asn Thr Lys Glu
            20                  25
```

<210> 20
<211> 27
<212> PRT
<213> Unknown

<220>
<223> Description of Unknown Organism: P domain of representative K+ channel sequence

<220>
<223> eag

<400> 20

```
Tyr Val Thr Ala Leu Tyr Phe Thr Met Thr Cys Met Thr Ser Val Gly
 1               5                  10                  15

Phe Gly Asn Val Ala Ala Glu Thr Asp Asn Glu
            20                  25
```

<210> 21
<211> 27
<212> PRT

<213> Unknown

<220>
<223> Description of Unknown Organism: P domain of representative K+ channel sequence

<220>
<223> ROMK1

<400> 21

```
Met Thr Ser Ala Phe Leu Phe Ser Leu Glu Thr Gln Val Thr Ile Gly
1               5                   10                  15

Tyr Gly Phe Arg Phe Val Thr Glu Gln Cys Ala
            20                  25
```

<210> 22
<211> 27
<212> PRT
<213> Unknown

<220>
<223> Description of Unknown Organism: P domain of representative K+ channel sequence

<220>
<223> IRK1

<400> 22

```
Phe Thr Ala Ala Phe Leu Phe Ser Ile Glu Thr Gln Thr Thr Ile Gly
1               5                   10                  15

Tyr Gly Phe Arg Cys Val Thr Asp Glu Cys Pro
            20                  25
```

<210> 23
<211> 27
<212> PRT
<213> Unknown

<220>
<223> Description of Unknown organism: P domain of representative K+ channel sequence

<220>
<223> GIRK1

<400> 23

```
Phe Pro Ser Ala Phe Leu Phe Phe Ile Glu Thr Glu Ala Thr Ile Gly
1               5                   10                  15

Tyr Gly Tyr Arg Tyr Ile Thr Asp Lys Cys Pro
            20                  25
```

<210> 24
<211> 48
<212> DNA
<213> Artificial Sequence

<220>
<223> Description of Artificial Sequence: antisense oligonucleotide complementary to the partial mouse cDNA sequence of TASK

<400> 24
eaccagcagg taggtgaagg tgcacacgat gagagccaac gtgcgcac 48

SEQ ID No. 25
5'-CACAGAAGCTGCATCTGCTCA-3
SEQ ID No. 26
5'-CCCTCAGTCTCCATGAATAGGA-3'
SEQ ID No. 27
nucleic acid sequence from Fig. 1 Provisional 989.6732P
SEQ ID No. 28
amino acid sequence shown in Fig. 1 of Provisional
SEQ ID NO. 29
5'-CTGCTCACCTCGGCCATCATCTTC-3'
SEQ ID No. 30
5'-GTAGAGGCCCTCGATGTAGTTCCA-3'
SEQ ID NO. 31
5'-CTTCCTAACCTTCCATCATCC-3'
SEQ ID NO. 32
5'-CTTGACCTGAGAGAGGGAAC-3'

## Claims

1. Use of an isolated nucleic acid molecule encoding a mammalian protein which is competent to transport potassium across a membrane, said transport being regulated by external pH, which protein is highly expressed in kidney and comprises two P domains and four transmembrane segments, said protein having the amino acid sequence represented on figure 14a, for the manufacture of a composition for diagnosing or for treating hypertension or dysfunctions of the kidney, liver or pancreas.

2. The use of claim 1 wherein said protein exhibits non-inactivating outwardly rectifying potassium currents.

3. The use of claim 1 wherein said protein is also expressed to a lesser extent in the pancreas, the liver, the placenta, and the small intestine.

4. The use according to any one of claims 1 to 3 wherein the isolated nucleic acid encoding said mammalian protein is represented on figure 14a.

5. The use according to any one of claims 1 to 4 wherein said protein is predominantly expressed in the distal tubules and collecting ducts of the kidney.

6. The use according to any one of claims 1 to 5 wherein said protein is expressed preferentially in epithelia and is poorly expressed in nervous and muscular tissue.

7. The use according to any one of claims 1 to 6 wherein the transport activity of said protein is regulated by external pH in the physiologic range.

8. The use according to any one of claims 1 to 7 wherein said protein exhibits non-inactivating outwardly rectifying potassium currents.

9. The use according to any one of claims 1 to 8 wherein the potassium transport by said protein is 50% inhibited by 22μM quinine.

10. The use according to any one of claims 1 to 9 wherein the potassium transport by said protein is 65% inhibited by 100μM quinidine.

11. The use according to any one of claims 1 to 10 wherein the potassium transport by said protein is not inhibited by 1mM tetraethylammonium, 100μM 4-aminopyridine, 1mM $Cs^+$, or intracellular cAMP.

12. A method for identifying substances which are capable of modulating the potassium transport activity of the protein defined in any one of claims 1 to 11 comprising bringing into contact variable amounts of a substance to be tested with cells expressing said potassium channel and determining the effects of the substance on the potassium transport properties of the channel.

13. A diagnostic test method to detect hypertension or diseases of the kidney, pancreas and/or liver that arise from mutations in the gene comprising the sequence of nucleic acid defined in claim 4, comprising hybridizing said sequence with an unknown sample of human nucleic acid.

14. The diagnostic test of claim 13 wherein the hybridization is carried out under stringent conditions (50% aqueous formamide at 45°C).

15. The diagnostic test of claim 13 wherein the hybridization is carried out under non-stringent conditions (6 X SSC, 5 X Denhardt's solution at 60°C).

16. The diagnostic test of claim 13 where said nucleic acid is comprised of intact, isolated chromosomes.

17. The diagnostic test of claim 16 wherein the gene comprising the sequence is amplified by PCR prior to hybridization with the sequence.

18. A diagnostic test to identify the TASK2 potassium channel in a tissue sample comprising reacting said tissue with an antibody reactive against the protein defined in any one of claims 1 to 11.

**Patentansprüche**

1. Verwendung eines isolierten Nukleinsäuremoleküls, das ein Säugetierprotein codiert, das dazu in der Lage ist, Kalium über eine Membrane zu transportieren, wobei der Transport durch den externen pH reguliert wird, wobei das Protein in der Niere eine hohe Expression aufweist und zwei P-Domänen und vier transmembranöse Abschnitte umfasst, wobei die Aminosäuresequenz des Proteins in Fig. 14a dargestellt ist, zur Herstellung einer Zusammensetzung zur Diagnose oder Behandlung von Bluthochdruck oder Funktionsstörungen der Niere, der Leber oder der Bauchspeicheldrüse.

2. Verwendung nach Anspruch 1, bei der das Protein nicht inaktivierende nach außen rektifizierende Kaliumströme zeigt.

3. Verwendung nach Anspruch 1, bei der das Protein in einem geringeren Umfang auch in der Bauschspeicheldrüse, der Leber, der Plazenta und dem Dünndarm eine Expression aufweist.

4. Verwendung nach einem der Ansprüche 1 bis 3, bei der die isolierte Nukleinsäure, die das Säugetierprotein codiert, in Figur 14a dargestellt ist.

5. Verwendung nach einem der Ansprüche 1 bis 4, bei der das Protein vorwiegend in den distalen Tubuli und den Sammelkanälen der Niere eine Expression aufweist.

6. Verwendung nach einem der Ansprüche 1 bis 5, bei der das Protein vorzugsweise in den Epithelia eine Expression aufweist und im Nerven- und Muskelgewebe eine schwache Expression aufweist.

7. Verwendung nach einem der Ansprüche 1 bis 6, bei der die Transportaktivität des Proteins durch den externen pH im physiologischen Bereich reguliert wird.

**8.** Verwendung nach einem der Ansprüche 1 bis 7, bei der das Protein nicht inaktivierende nach außen rektifizierende Kaliumströme zeigt.

**9.** Verwendung nach einem der Ansprüche 1 bis 8, bei der der Kaliumtransport durch das Protein zu 50% durch 22μM Chinin gehemmt wird.

**10.** Verwendung nach einem der Ansprüche 1 bis 9, bei der der Kaliumtransport durch das Protein zu 65% durch 100μM Chinidin gehemmt wird.

**11.** Verwendung nach einem der Ansprüche 1 bis 10, bei der der Kaliumtransport durch das Protein nicht durch 1 mM Tetraethylammonium, 100μM 4-Aminopyridin, 1 mM Cs+ oder intrazelluläres cAMP gehemmt wird.

**12.** Verfahren zur Identifizierung von Substanzen, die dazu in der Lage sind, die Kaliumtransport-Aktivität des Proteins, definiert in einem der Ansprüche 1 bis 11, zu modulieren, darin eingeschlossen die Kontaktierung variabler Mengen einer Substanz, die getestet werden soll, mit Zellen, die den Kaliumkanal exprimieren, und die Festsetzung der Wirkungen der Substanz auf die Kaliumtransport-Eigenschaften des Kanals.

**13.** Diagnose-Testverfahren, um Bluthochdruck oder Krankheiten der Niere, der Bauchspeicheldrüse und/oder der Leber festzustellen, die sich aus Mutationen im Gen ergeben, umfassend die Nukleinsäuresequenz, definiert in Anspruch 4, umfassend die Hybridisierung der Sequenz mit einer unbekannten Probe menschlicher Nukleinsäure.

**14.** Diagnosetest nach Anspruch 13, bei dem die Hybridisierung unter strengen Bedingungen durchgeführt wird (50% wässriges Formamid bei 45 °C).

**15.** Diagnosetest nach Anspruch 13, bei dem die Hybridisierung unter nicht strengen Bedingungen durchgeführt wird (6 X SSC, 5 X Denhardt's-Lösung bei 60°C) .

**16.** Diagnosetest nach Anspruch 13, bei dem die Nukleinsäure intakte, isolierte Chromosomen umfasst.

**17.** Diagnosetest nach Anspruch 16, bei dem das Gen, das die Sequenz umfasst, vor der Hybridisierung mit der Sequenz durch eine PCR vergrößert wird.

**18.** Diagnosetest, um den TASK2-Kaliumkanal in einer Gewebeprobe zu identifizieren, umfassend das Zur-Reaktion-Bringen des Gewebes mit einem Antikörper, der gegen das Protein, das in einem der Ansprüche 1 bis 11 definiert wird, reaktiv ist.

## Revendications

**1.** Utilisation d'une molécule d'acide nucléique isolé codant pour une protéine mammifère qui est compétente pour transporter le potassium à travers une membrane, ledit transport étant régulé par le pH externe, laquelle protéine est grandement exprimée dans les reins et comprend deux domaines P et quatre segments transmembranaires, ladite protéine ayant la séquence d'acides aminés représentée sur la figure 14a, pour la fabrication d'une composition destinée au diagnostic ou au traitement de l'hypertension ou de dysfonctions rénales, hépatiques ou pancréatiques.

**2.** Utilisation selon la revendication 1, dans laquelle ladite protéine présente des courants potassiques rectifiants sortants non-inactivants.

**3.** Utilisation selon la revendication 1, dans laquelle ladite protéine est également exprimée à un moindre degré dans le pancréas, le foie, le placenta, et l'intestin grêle.

**4.** Utilisation selon l'une quelconque des revendications 1 à 3, dans laquelle l'acide nucléique isolé codant pour ladite protéine de mammifère est représenté sur la figure 14a.

**5.** Utilisation selon l'une quelconque des revendications 1 à 4, dans laquelle ladite protéine est principalement exprimée dans les tubules distaux et les canaux collecteurs des reins.

**6.** Utilisation selon l'une quelconque des revendications 1 à 5, dans laquelle ladite protéine est préférentiellement exprimée dans l'épithélium et est faiblement exprimée dans les tissus nerveux et musculaires.

**7.** Utilisation selon l'une quelconque des revendications 1 à 6, dans laquelle l'activité de transport de ladite protéine est régulée par le pH externe dans la plage physiologique.

**8.** Utilisation selon l'une quelconque des revendications 1 à 7, dans laquelle ladite protéine présente des courants potassiques rectifiants sortants non-inactivants.

**9.** Utilisation selon l'une quelconque des revendications 1 à 8, dans laquelle le transport de potassium par ladite protéine est inhibé à 50 % par de la quinine 22 μM.

**10.** Utilisation selon l'une quelconque des revendications 1 à 9, dans laquelle le transport de potassium par ladite protéine est inhibé à 65 % par de la quinine 100 μM.

**11.** Utilisation selon l'une quelconque des revendications 1 à 10, dans laquelle le transport de potassium par ladite protéine n'est pas inhibé par du tétraéthylammonium 1 mM, de la 4-aminopyridine 100 μM, du $Cs^+$ 1 mM, ou l'AMPc intracellulaire.

**12.** Procédé d'identification de substances qui sont capables de moduler l'activité de transport du potassium de la protéine définie dans l'une quelconque des revendications 1 à 11, comprenant la mise en contact de quantités variables d'une substance à tester avec des cellules exprimant ledit canal potassique et la détermination des effets de la substance sur les propriétés de transport du potassium du canal.

**13.** Test diagnostique destiné à détecter l'hypertension ou des maladies rénales, pancréatiques et/ou hépatiques qui sont dues à des mutations dans le gène comprenant la séquence d'acide nucléique définie dans la revendication 4, comprenant l'hybridation de ladite séquence avec un échantillon inconnu d'acide nucléique humain.

**14.** Test diagnostique selon la revendication 13, dans lequel l'hybridation est réalisée dans des conditions stringentes (formamide aqueux à 50 % à 45°C).

**15.** Test diagnostique selon la revendication 13, dans lequel l'hybridation est réalisée dans des conditions non stringentes (SSC 6 X, solution de Denhardt 5 X à 60°C).

**16.** Test diagnostique selon la revendication 13, dans lequel l'acide nucléique est composé de chromosomes isolés intacts.

**17.** Test diagnostique selon la revendication 16, dans lequel le gène comprenant la séquence est amplifié par PCR avant l'hybridation avec la séquence.

**18.** Test diagnostique destiné à identifier le canal potassique TASK2 dans un échantillon de tissu, comprenant la mise en réaction dudit tissu avec un anticorps réactif contre la protéine définie dans l'une quelconque des revendications 1 à 11.

Fig.1 b

gggcaggaagacggcgctgcccggaggagc -153
ggggcgggcgggcgcgcgggggagcgggcggcgggcgggagccaggcccgggcgggggcgggggcggcggggccag -77
aagaggcggcgggccgcgctccggccggtctgcggcgttggccttggctttggctttggcggcggcggtggagaag -1

ATG CTG CAG TCC CTG GCC GGC AGC TCG TGC GTG CGC CTG GTG GAG CGG CAC CGC TCG 57
M L Q S L A G S S C V R L V E R H R S 19

GCC TGG TGC TTC GGC TTC CTG GTG CTG GGC TAC TTG CTC TAC CTG GTC TTC GGC GCA 114
A W C F G F L V L G Y L L Y L V F G A 38

GTG GTC TTC TCC TCG GTG GAG CTG CCC TAT GAG GAC CTG CTG CGC CAG GAG CTG CGC 171
V V F S S V E L P Y E D L L R Q E L R 57

AAG CTG AAG CGA CGC TTC TTG GAG GAG CAC GAG TGC CTG TCT GAG CAG CAG CTG GAG 228
K L K R R F L E E H E C L S E Q Q L E 76

CAG TTC CTG GGC CGG GTG CTG GAG GCC AGC AAC TAC GGC GTG TCG GTG CTC AGC AAC 285
Q F L G R V L E A S N Y G V S V L S N 95

GCC TCG GGC AAC TGG AAC TGG GAC TTC ACC TCC GCG CTC TTC TTC GCC AGC ACC GTG 342
A S G N W N W D F T S A L F F A S T V 114

CTC TCC ACC ACA GGT TAT GGC CAC ACC GTG CCC TTG TCA GAT GGA GGT AAG GCC TTC 399
L S T T G Y G H T V P L S D G G K A F 133

TGC ATC ATC TAC TCC GTC ATT GGC ATT CCC TTC ACC CTC CTG TTC CTG ACG GCT GTG 456
C I I Y S V I G I P F T L L F L T A V 152

GTC CAG CGC ATC ACC GTG CAC GTC ACC CGC AGG CCG GTC CTC TAC TTC CAC ATC CGC 513
V Q R I T V H V T R R P V L Y F H I R 171

TGG GGC TTC TCC AAG CAG GTG GTG GCC ATC GTC CAT GCC GTG CTC CTT GGG TTT GTC 570
W G F S K Q V V A I V H A V L L G F V 190

ACT GTG TCC TGC TTC TTC TTC ATC CCG GCC GCT GTC TTC TCA GTC CTG GAG GAT GAC 627
T V S C F F F I P A A V F S V L E D D 209

TGG AAC TTC CTG GAA TCC TTT TAT TTT TGT TTT ATT TCC CTG AGC ACC ATT GGC CTG 684
W N F L E S F Y F C F I S L S T I G L 228

GGG GAT TAT GTG CCT GGG GAA GGC TAC AAT CAA AAA TTC AGA GAG CTC TAT AAG ATT 741
G D Y V P G E G Y N Q K F R E L Y K I 247

GGG ATC ACG TGT TAC CTG CTA CTT GGC CTT ATT GCC ATG TTG GTA GTT CTG GAA ACC 798
G I T C Y L L L G L I A M L V V L E T 266

TTC TGT GAA CTC CAT GAG CTG AAA AAA TTC AGA AAA ATG TTC TAT GTG AAG AAG GAC 855
F C E L H E L K K F R K M F Y V K K D 285

AAG GAC GAG GAT CAG GTG CAC ATC ATA GAG CAT GAC CAA CTG TCC TTC TCC TCG ATC 912
K D E D Q V H I I E H D Q L S F S S I 304

ACA GAC CAG GCA GCT GGC ATG AAA GAG GAC CAG AAG CAA AAT GAG CCT TTT GTG GCC 969
T D Q A A G M K E D Q K Q N E P F V A 323

ACC CAG TCA TCT GCC TGC GTG GAT GGC CCT GCA AAC CAT TGA gcgtaggatttgttgcatt 1030
T Q S S A C V D G P A N H * 337
atgctagagcaccagggtcagggtgcaaggaagaggcttaagtatgttcattttatcagaatgcaaaagcgaaaa 1106
ttatgtcactttaagaaatagctactgtttgcaatgtcttattaaaaacaacaaaaaagacacatggaacaaag 1182
aagctgtgaccccagcaggatgtctaatatgtgaggaaatgagatgtccacctaaaattcatatgtgacaaatta 1258
tctcgaccttacataggaggagaatacttgaagcagtatgctgctgtggttagaagcagattttatacttttaact 1334
ggaaactttggggtttgcatttagatcatttagctgatggctaaatagcaaaatttatatttagaagcaaaaaaa 1410
aaagcatagagatgtgttttataaataggtttatgtgtactggtttgcatgtacccacccaaaatgattattttg 1486
gagaatctaagtcaaactcactatttataatgcataggtaaccattaactatgtacatataaagtataaatatgtt 1562
tatattctgtacatatggtttaggtcaccagatcctagtgtagttctgaaactaagactatagatatttgtttct 1638
tttgatttctctttatactaaagaatccagagttgctacaataaaataaggggaataataaaaaaaaaaaaaa 1712

Fig.1a

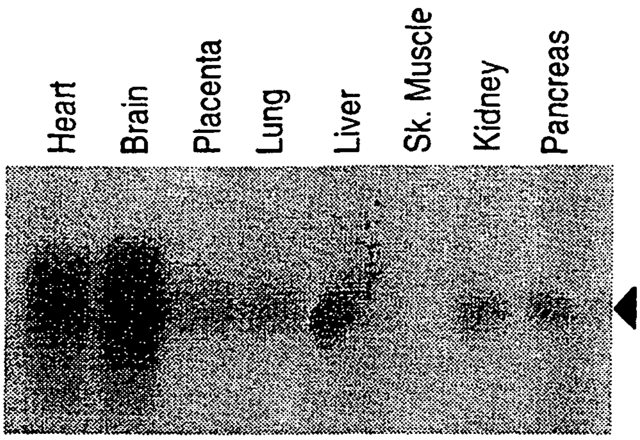
Heart
Brain
Placenta
Lung
Liver
Sk. Muscle
Kidney
Pancreas

Fig.1 c

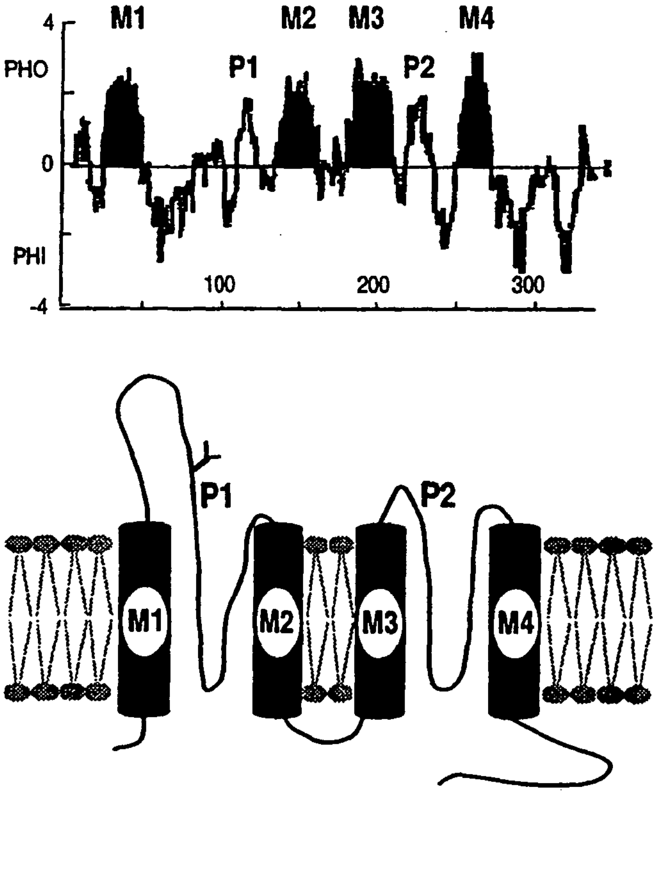
M1
M2
M3
M4
P1
P2
PHO
PHI
4
0
-4
100
200
300
M1
M2
M3
M4
P1
P2

Fig.2 a

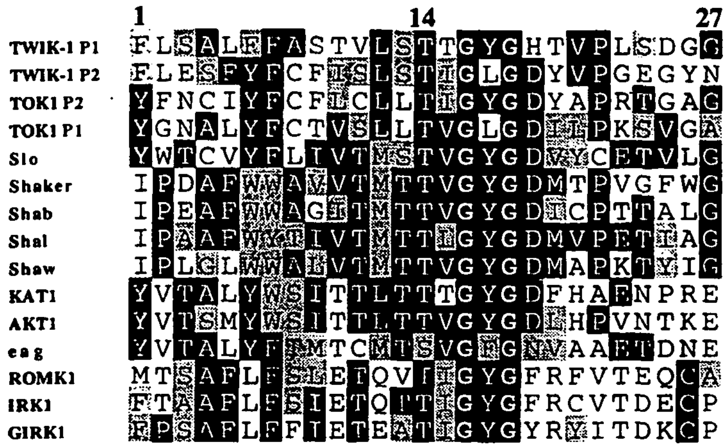
1
14
27
TWIK-1 P1
TWIK-1 P2
TOK1 P2
TOK1 P1
Slo
Shaker
Shab
Shal
Shaw
KAT1
AKT1
eag
ROMK1
IRK1
GIRK1

Fig.2 b

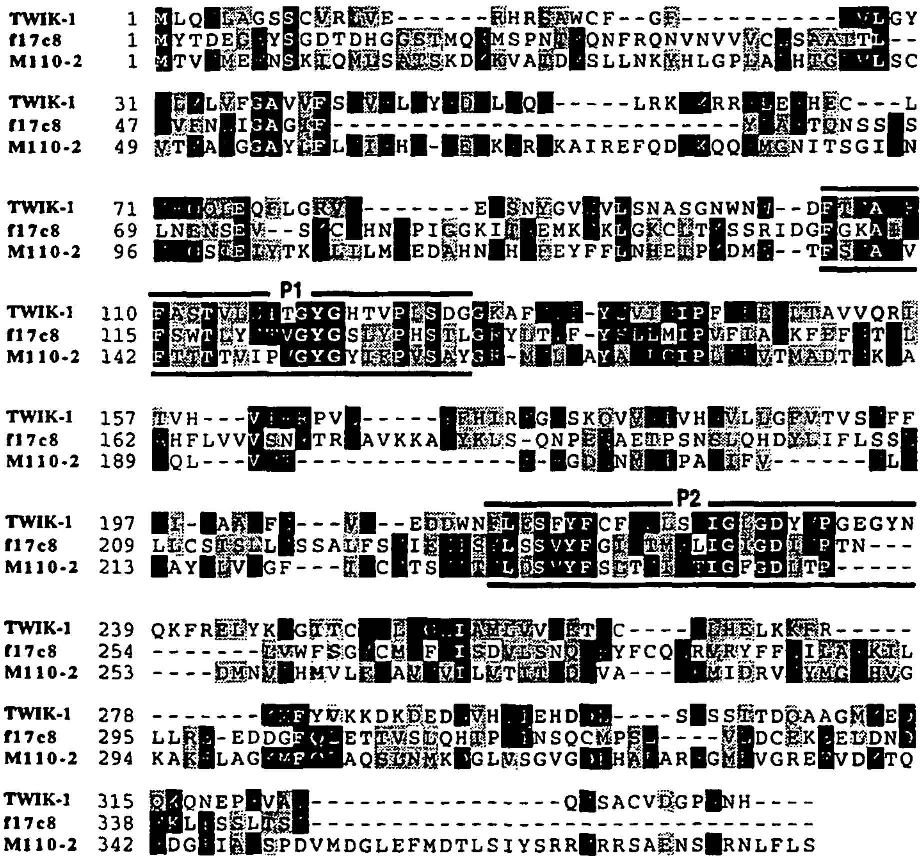
TWIK-1
f17c8
M110-2
P1
P2

Fig.3 a

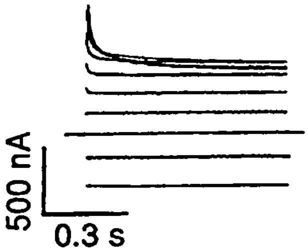


Fig.3 b

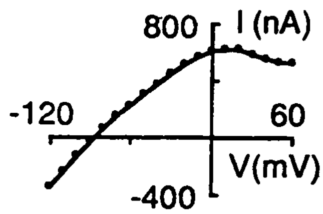


Fig.3 c

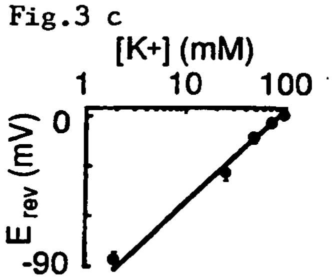


Fig.3 d

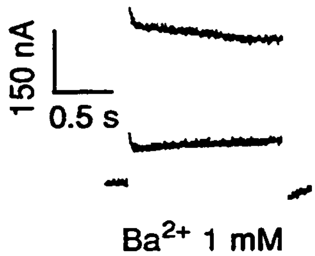


Fig.3 e

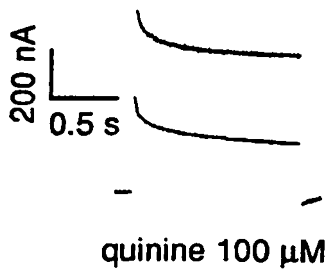


Fig.3 f

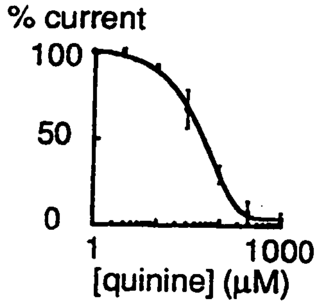

Fig. 4 a

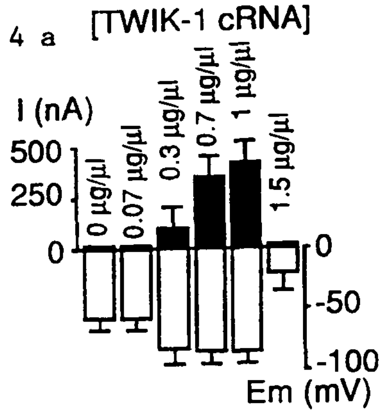


Fig.4 b

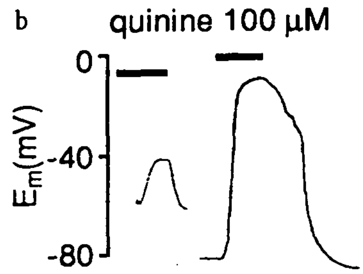


Fig.4 c

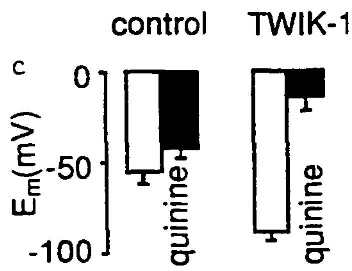

Fig.5 a

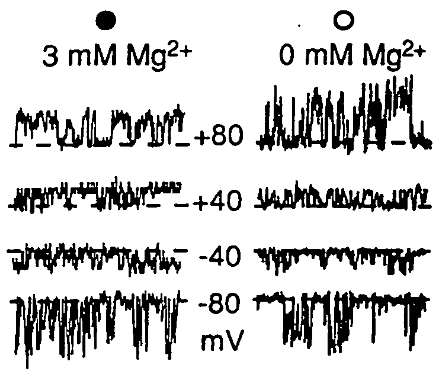


Fig.5 b

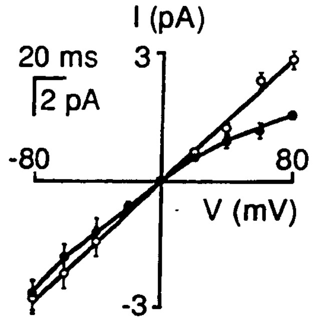


Fig.5 c

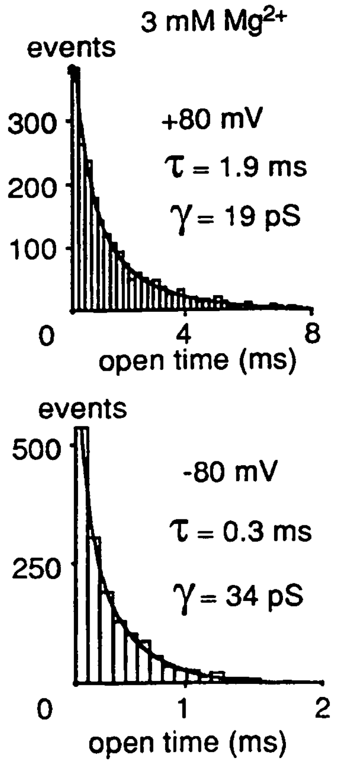


Fig.5 d

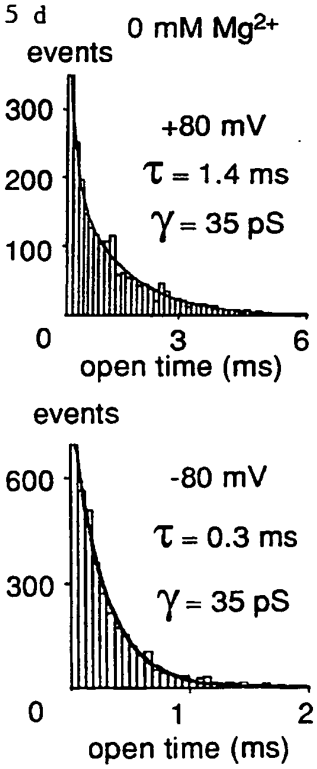

Fig.6 a

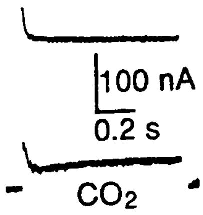


Fig.6 b

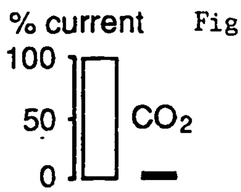


Fig.6 c

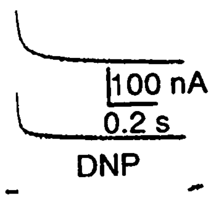


Fig.6 d

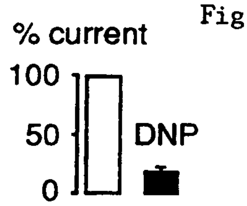


Fig.6 e

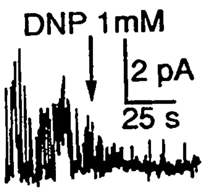


Fig.6 f

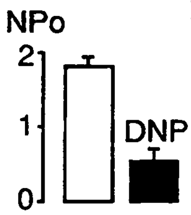


Fig.6 g

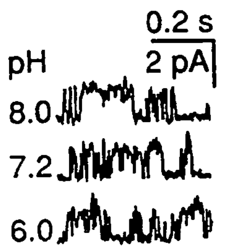


Fig.6 h

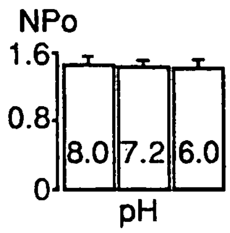

Fig.7 a

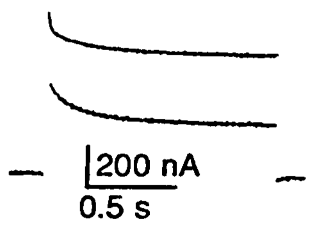


Fig.7 b

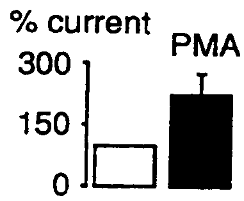


Fig.7 c

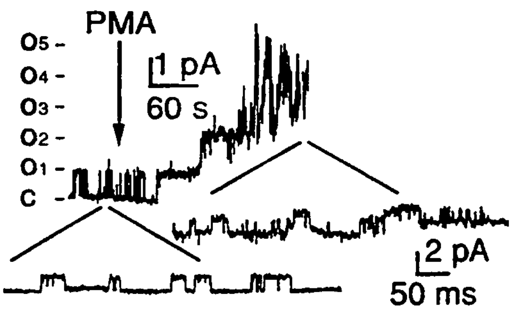


Fig. 7 d

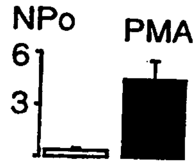

Fig.8 a

```
                                 tgccctgcgcggatagcggcgagcgcagccatgccccaggccgcctccg  -77
gggcagcagcagcggcggccggggccgatgcgcggccgggggcgccgggggccggcggcggcccgggcgggacg  -1

ATG AAG CGG CAG AAC GTG CGC ACG CTG GCG CTC ATC GTG TGC ACC TTC ACC TAC CTG   57
 M   K   R   Q   N   V   R   T   L   A   L   I   V   C   T   F   T   Y   L   19
             E   N   V   R   T   L   A   L   I   V   C   T   F   T   Y   L

CTG GTG GGC GCC GCG GTC TTc GAC GCG CTG GAG TCG GAG CCC GAG CTG ATC GAG CGG  114
 L   V   G   A   A   V   F   D   A   L   E   S   E   P   E   L   I   E   R   38
 L   V   G   A   A   V   F   D   A   L   E   S   E   P   E   M   I   E   R

CAG CGG CTG GAG CTG CGG CAG CAG GAG CTG CGG GCG CGC TAC AAC CTC AGC CAG GGC  171
 Q   R   L   E   L   R   Q   Q   E   L   R   A   R   Y   N   L   S   Q   G   57
 Q   R   L   E   L   R   Q   L   E   L   R   A   R   Y   N   L   S   E   G
                                                         *
GGC TAC GAG GAG CTG GAG CGC GTC GTG CTG CGC CTC AAG CCG CAC AAG GCC GGC GTG  228
 G   Y   E   E   L   E   R   V   V   L   R   L   K   P   H   K   A   G   V   76
 G   Y   E   E   L   E   R   V   V   L   R   L   K   P   H   K   A   G   V

CAG TGG CGC TTC GCC GGC TCC TTC TAC TTC GCC ATC ACC GTC ATC ACC ACC ATC GGC  285
 Q   W   R   F   A   G   S   F   Y   F   A   I   T   V   I   T   T   I   G   95
 Q   W   R   F   A   G   S   F   Y   F   A   I   T   V   I   T   T   I   G

TAC GGG CAC GCG GCA CCC AGC ACG GAT GGC GGC AAG GTG TTC TGC ATG TTC TAC GCG  342
 Y   G   H   A   A   P   S   T   D   G   G   K   V   F   C   M   F   Y   A   114
 Y   G   H   A   A   P   S   T   D   G   G   K   V   F   C   M   F   Y   A

CTG CTG GGC ATC CCG CTC ACG CTC GTC ATG TTC CAG AGC CTG GGC GAG CGC ATC AAC  399
 L   L   G   I   P   L   T   L   V   M   F   Q   S   L   G   E   R   I   N   133
 L   L   G   I   P   L   T   L   I   M   F   Q   S   L   G   E   R   I   N

ACC TTG GTG AGG TAC CTG CTG CAC CGC GCC AAG AAG GGG CTG GGC ATG CGG CGC GCC  456
 T   L   V   R   Y   L   L   H   R   A   K   K   G   L   G   M   R   R   A   152
 T   F   V   R   Y   L   L   H   R   A   K   R   G   L   G   M   R   H   A

GAC GTG TCC ATG GCC AAC ATG GTG CTC ATC GGC TTC TTC TCG TGC ATC AGC ACG CTG  513
 D   V   S   M   A   N   M   V   L   I   G   F   F   S   C   I   S   T   L   171
 E   V   S   M   A   N   M   V   L   I   G   F   V   S   C   I   S   T   L

TGC ATC GGC GCC GCC GCC TTC TCC CAC TAC GAG CAC TGG ACC TTC TTC CAG GCC TAC  570
 C   I   G   A   A   A   F   S   H   Y   E   H   W   T   F   F   Q   A   Y   190
 C   I   G   A   A   A   F   S   Y   Y   E   R   W   T   F   F   Q   A   Y

TAC TAC TGC TTC ATC ACC CTC ACC ACC ATC GGC TTC GGC GAC TAC GTG GCG CTG CAG  627
 Y   Y   C   F   I   T   L   T   T   I   G   F   G   D   Y   V   A   L   Q   209
 Y   Y   C   F   I   T   L   T   T   I   G   F   G   D   Y   V   A   L   Q

AAG GAC CAG GCC CTG CAG ACG CAG CCG CAG TAC GTG GCC TTC AGC TTC GTC TAC ATC  684
 K   D   Q   A   L   Q   T   Q   P   Q   Y   V   A   F   S   F   V   Y   I   228
 K   D   Q   A   L   Q   T   Q   P   Q   Y   V   A   F   S   F   V   Y   I

CTT ACG GGC CTC ACG GTC ATC GGC GCC TTC CTC AAC CTC GTG GTG CTG CGC TTC ATG  741
 L   T   G   L   T   V   I   G   A   F   L   N   L   V   V   L   R   F   M   247
 L   T   G   L   T   V   I   G   A   F   L   N   L   V   V   L   R   F   M
```

Fig.8 a continued

```
ACC ATG AAC GCC GAG GAC GAG AAG CGC GAC GCC GAG CAC CGC GCG CTG CTC ACG CGC   798
 T   M   N   A   E   D   E   K   R   D   A   E   H   R   A   L   L   T   R    266
 T   M   N   A   E   D   E   K   R   D   A   E   H   R   A   L   L   T   H

AAC GGG CAG GCG GGC GGC GGC GGA GGG GGT GGC AGC GCG CAC ACT ACG GAC ACC GCC   855
 N   G   Q   A   G   G   G   G   G   G   G   S   A   H   T   T   D   T   A    285
 N   G   Q   A   V   G   L   G   G   L   S   C   L   S   G   S   L   G   D

TCA TCC ACG GCG GCA GCG GGC GGC GGC GGC TTC CGC AAC GTC TAC GCG GAG GTG CTG   912
 S   S   T   A   A   A   G   G   G   G   F   R   N   V   Y   A   E   V   L    304
VRPRDPV TC AA A  A   G GVGVGVGGS G   F   R   N   V   Y   A   E   V   L

CAC TTC CAG TCC ATG TGC TCG TGC CTG TGG TAC AAG AGC CGC GAG AAG CTG CAG TAC   969
 H   F   Q   S   M   C   S   C   L   W   Y   K   S   R   E   K   L   Q   Y    323
 H   F   Q   S   M   C   S   C   L   W   Y   K   S   R   E   K   L   Q   Y
                                                                         ●

TCC ATC CCC ATG ATC ATC CCG CGG GAC CTC TCC ACG TCC GAC ACG TGC GTG GAG CAG  1026
 S   I   P   M   I   I   P   R   D   L   S   T   S   D   T   C   V   E   Q    342
 S   I   P   M   I   I   P   R   D   L   S   T   S   D   T   C   V   E   H

AGC CAC TCG TCG CCG GGA GGG GGC GGC CGC TAC AGC GAC ACG CCC TCG CGA CGC TGC  1083
 S   H   S   S   P   G   G   G   G   R   Y   S   D   T   P   S   R   R   C    361
 S   H   S   S   P   G   G   G   G   R   Y   S   D   T   P   S   H   P   C
                                                             ■

CTG TGC AGC GGG GCG CCA CGC TCC GCC ATC AGC TCG GTG TCC ACG GGT CTG CAC AGC  1140
 L   C   S   G   A   P   R   S   A   I   S   S   V   S   T   G   L   H   S    380
 L   C   S   G   T   Q   R   S   A   I   S   S   V   S   T   G   L   H   S

CTG TCC ACC TTC CGC GGC CTC ATG AAG CGC AGG AGC TCC GTG TGA  ctgccccgagggacc 1200
 L   S   T   F   R   G   L   M   K   R   R   S   S   V   *                    395
 L   A   A   F   R   G   L   M   K   R   R   S   S   V
         ■                               ▲   ▲

tggagcacctgggggcgcgggcgggggacccctgctgggaggccaggagactgcccctgctgccttctgcccagtg 1276
ggaccccgcacaacatccctcaccactctcccccagcacccccatctccgactgtgcctgcttgcaccagccggca 1352
ggaggccgggctctgaggacccctgggcccccatcggagccctgcaaattccgagaaatgtgaaacttggtgggg 1428
tcagggaggaaaggcagaagctgggagcctcccttccctttgaaaatctaagaagctcccagtcctcagagaccct 1504
gctggtaccacaccccaccttcggaggggacttcatgttccgtgtacgtttgcatctctatttatacctctgtcct 1580
gctaggtctcccaccttcccttggttccaaaagccagggtgtctatgtccaagtcacccctactcagccccactcc 1656
ccttcctcatccccagctgtgtctcccaacctcccttcgtgttgttttgcatggctttgcagttatggagaaagtg 1732
gaaacccagcagtccctaaagctggtccccagaaagcaggacagaaagaaggagggacaggcaggcagcaggaggg 1808
gcgagctgggaggcaggaggcagcggcctgtcagtctgcagaatggtcgcactggaggttcaagctaactggcctc 1884
cagccacattctcatagcaggtaggacttcagccttccagacactgcccttagaatctggaacagaagacttcaga 1960
ctcaccataattgctgataattacccactcttaaatttgtcgagtgattttagcctctgaaaactctatgctggc 2036
cactgattcctttgagtctcacaaaccctacttaggtcatcagggcaggagttctcactcccatttacagatga 2112
gaatactgaggcctggacaggtgaagtgaccagagagcaaaaggcaaaggggtgggggctgggtgcagtggctcac 2188
acctgtattcccaacactttggaggctgaggttggaggattgcttgagcccaggaattcgagaccagcctaggtg 2264
acatagtgagaccccatctctacaaaaaataaaaaattaaccaggtgtggtggcacgtgcctgggagtcccagcga 2340
cttgggaggctgaggtgggaggattgtttgagcctgggaggtcgaggctgtagtgagccctgattgcaccactgta 2416
ctccagcctgggtgacagggcaagaccctgtctcaaaaaaaaaaaaaa                            2465
```

Fig. 8 b

tgccctgcgcggatagcggcgagcgcagccatgccccaggccgcctccg -77
gggcagcagcagcggcggccggggccgatgcgcgggccgggggcgccggggggccggcggcggcccgggcgggacg -1

ATG AAG CGG CAG AAC GTG CGC ACG CTG GCG CTC ATC GTG TGC ACC TTC ACC TAC CTG 57
M K R Q N V R T L A L I V C T F T Y L 19
E N V R T L A L I V C T F T Y L

CTG GTG GGC GCC GCG GTC TTc GAC GCG CTG GAG TCG GAG CCC GAG CTG ATC GAG CGG 114
L V G A A V F D A L E S E P E L I E R 38
L V G A A V F D A L E S E P E M I E R

CAG CGG CTG GAG CTG CGG CAG CAG GAG CTG CGG GCG CGC TAC AAC CTC AGC CAG GGC 171
Q R L E L R Q Q E L R A R Y N L S Q G 57
Q R L E L R Q L E L R A R Y N L S E G
*

GGC TAC GAG GAG CTG GAG CGC GTC GTG CTG CGC CTC AAG CCG CAC AAG GCC GGC GTG 228
G Y E E L E R V V L R L K P H K A G V 76
G Y E E L E R V V L R L K P H K A G V

CAG TGG CGC TTC GCC GGC TCC TTC TAC TTC GCC ATC ACC GTC ATC ACC ACC ATC GGC 285
Q W R F A G S F Y F A I T V I T T I G 95
Q W R F A G S F Y F A I T V I T T I G

TAC GGG CAC GCG GCA CCC AGC ACG GAT GGC GGC AAG GTG TTC TGC ATG TTC TAC GCG 342
Y G H A A P S T D G G K V F C M F Y A 114
Y G H A A P S T D G G K V F C M F Y A

CTG CTG GGC ATC CCG CTC ACG CTC GTC ATG TTC CAG AGC CTG GGC GAG CGC ATC AAC 399
L L G I P L T L V M F Q S L G E R I N 133
L L G I P L T L I M F Q S L G E R I N

ACC TTG GTG AGG TAC CTG CTG CAC CGC GCC AAG AAG GGG CTG GGC ATG CGG CGC GCC 456
T L V R Y L L H R A K K G L G M R R A 152
T F V R Y L L H R A K R G L G M R H A

GAC GTG TCC ATG GCC AAC ATG GTG CTC ATC GGC TTC TTC TCG TGC ATC AGC ACG CTG 513
D V S M A N M V L I G F F S C I S T L 171
E V S M A N M V L I G F V S C I S T L

TGC ATC GGC GCC GCC GCC TTC TCC CAC TAC GAG CAC TGG ACC TTC TTC CAG GCC TAC 570
C I G A A A F S H Y E H W T F F Q A Y 190
C I G A A A F S Y Y E R W T F F Q A Y

TAC TAC TGC TTC ATC ACC CTC ACC ACC ATC GGC TTC GGC GAC TAC GTG GCG CTG CAG 627
Y Y C F I T L T T I G F G D Y V A L Q 209
Y Y C F I T L T T I G F G D Y V A L Q

AAG GAC CAG GCC CTG CAG ACG CAG CCG CAG TAC GTG GCC TTC AGC TTC GTC TAC ATC 684
K D Q A L Q T Q P Q Y V A F S F V Y I 228
K D Q A L Q T Q P Q Y V A F S F V Y I

CTT ACG GGC CTC ACG GTC ATC GGC GCC TTC CTC AAC CTC GTG GTG CTG CGC TTC ATG 741
L T G L T V I G A F L N L V V L R F M 247
L T G L T V I G A F L N L V V L R F M

Fig.8 b continued

ACC ATG AAC GCC GAG GAC GAG AAG CGC GAC GCC GAG CAC CGC GCG CTG CTC ACG CGC 798
T M N A E D E K R D A E H R A L L T R 266
T M N A E D E K R D A E H R A L L T H

AAC GGG CAG GCG GGC GGC GGC GGA GGG GGT GGC AGC GCG CAC ACT ACG GAC ACC GCC 855
N G Q A G G G G G G G S A H T T D T A 285
N G Q A V G L G G L S C L S G S L G D

TCA TCC ACG GCG GCA GCG GGC GGC GGC GGC TTC CGC AAC GTC TAC GCG GAG GTG CTG 912
S S T A A A G G G G F R N V Y A E V L 304
VRPRDPV TC AA A A G GVGVGVGGS G F R N V Y A E V L

CAC TTC CAG TCC ATG TGC TCG TGC CTG TGG TAC AAG AGC CGC GAG AAG CTG CAG TAC 969
H F Q S M C S C L W Y K S R E K L Q Y 323
H F Q S M C S C L W Y K S R E K L Q Y
●

TCC ATC CCC ATG ATC ATC CCG CGG GAC CTC TCC ACG TCC GAC ACG TGC GTG GAG CAG 1026
S I P M I I P R D L S T S D T C V E Q 342
S I P M I I P R D L S T S D T C V E H

AGC CAC TCG TCG CCG GGA GGG GGC GGC CGC TAC AGC GAC ACG CCC TCG CGA CGC TGC 1083
S H S S P G G G G R Y S D T P S R R C 361
S H S S P G G G G R Y S D T P S H P C
■

CTG TGC AGC GGG GCG CCA CGC TCC GCC ATC AGC TCG GTG TCC ACG GGT CTG CAC AGC 1140
L C S G A P R S A I S S V S T G L H S 380
L C S G T Q R S A I S S V S T G L H S

CTG TCC ACC TTC CGC GGC CTC ATG AAG CGC AGG AGC TCC GTG TGA ctgccccgagggacc 1200
L S T F R G L M K R R S S V * 395
L A A F R G L M K R R S S V
■ ▲ ▲

tggagcacctgggggcgcgggcgggggacccctgctgggaggccaggagactgcccctgctgccttctgcccagtg 1276
ggaccccgcacaacatccctcaccactctcccccagcacccccatctccgactgtgcctgcttgcaccagccggca 1352
ggaggccgggctctgaggacccctggggcccccatcggagccctgcaaattccgagaaatgtgaaacttggtgggg 1428
tcagggaggaaaggcagaagctgggagcctcccttccctttgaaaatctaagaagctcccagtcctcagagaccct 1504
gctggtaccacaccccaccttcggaggggacttcatgttccgtgtacgtttgcatctctatttatacctctgtcct 1580
gctaggtctcccaccttcccttggttccaaaagccagggtgtctatgtccaagtcacccctactcagccccactcc 1656
ccttcctcatccccagctgtgtctcccaacctcccttcgtgttgttttgcatggctttgcagttatggagaaagtg 1732
gaaacccagcagtccctaaagctggtccccagaaagcaggacagaaagaaggagggacaggcaggcagcaggaggg 1808
gcgagctgggaggcaggaggcagcggcctgtcagtctgcagaatggtcgcactggaggttcaagctaactggcctc 1884
cagccacattctcatagcaggtaggacttcagccttccagacactgcccttagaatctggaacagaagacttcaga 1960
ctcaccataattgctgataattacccactcttaaatttgtcgagtgattttagcctctgaaaactctatgctggc 2036
cactgattcctttgagtctcacaaaaccctacttaggtcatcagggcaggagttctcactcccattttacagatga 2112
gaatactgaggcctggacaggtgaagtgaccagagagcaaaaggcaaaggggtgggggctgggtgcagtggctcac 2188
acctgtattcccaacactttggaggctgaggttggaggattgcttgagcccaggaattcgagaccagcctaggtg 2264
acatagtgagaccccatctctacaaaaaataaaaaattaaccaggtgtggtggcacgtgcctgggagtcccagcga 2340
cttgggaggctgaggtgggaggattgtttgagcctgggaggtcgaggctgtagtgagccctgattgcaccactgta 2416
ctccagcctgggtgacagggcaagaccctgtctcaaaaaaaaaaaaaa 2465

Fig.9 a

```
            1 -----MLQSLAGSSCVR-----------LVERHRS---
            1 MAAPDLLDPKSAAQNSKPRLSFSSKPTVLASRVESDSA
            1 -----MKR----Q-NVR-------------------
                                    M1
TWIK-1     20 ----AWCFG-FLVLGYLLYLVFGAVVFSSVELPYEDLL
TREK-1     39 INVMKWKTVSTIFLVVVLYLIIGAAVFKALEQPQEISQ
TASK        8 --------TLALIVCTFTYLLVGAAVFDALESEPELIE

TWIK-1     53 RQELRKLKRRFLEEHECLSEQQLEQFLGRVLEASNYGV
TREK-1     77 RTTIVIQKQTFIAQHACVNSTELDELIQQIVAAINAGI
TASK       38 RQRLELRQQELRARYNLSQGG-YEELERVVLRLKPHKA
                                              P1
TWIK-1     91 SVLSNASG-NWNWDFTSALFFASTVLSTTGYGHTVPLS
TREK-1    115 IPLGNSSNQVSHWDLGSSFFFAGTVITTIGFGNISPRT
TASK       75 G--------VQ-WRFAGSFYFAITVITTIGYGHAAPST
                         M2
TWIK-1    128 DGGKAFCIIYSVIGIPFTLLFLTAVVQRITVHVTR--R
TREK-1    153 EGGKIFCIIYALLGIPLFGFLLAGVGDQLGTIFGKGIA
TASK      104 DGGKVFCMFYALLGIPLTLVMFQSLGERINTLVRY---
                                        M3
TWIK-1    164 PVLYFHIRWGFSKQVVAIVHAVLLGFVTVSCFFFIPAA
TREK-1    191 KVEDTFIKWNVSQTKIRIISTIIFILFGCVLFVALPAV
TASK      139 LLHRAKKGLGMRRADVSMANMVLIGFFSCISTLCIGAA
                                   P2
TWIK-1    202 VFSVLEDDWNFLESFYFCFISLSTIGLGDYVPGE-GYN
TREK-1    229 IFKHIEG-WSALDAIYFVVITLTTIGFGDYVAG--GSD
TASK      177 AFSHYEH-WTFFQAYYYCFITLTTIGFGDYVALQKDQA
                           M4
TWIK-1    239 QKFRELYKIGITCYLLLGLIAMLVVLETFCELHELKKF
TREK-1    264 IEYLDFYKPVVWFWILVGLAYFAAVLSMIGDWLRVISK
TASK      214 LQTQPQYVAFSFVYILTGLTVIGAFLNLVVLRFMTMNA

TWIK-1    277 RKMFYVKKDKD---------------------------
TREK-1    302 KTKEEVGEFR----------------------------
TASK      252 EDEKRDAEHRALLTRNGQAGGGGGGGSAHTTDTASSTA

TWIK-1    288 -----------EDQVHIIEHDQLSFSSITDQAAGMK--
TREK-1    312 -----------AHAAEWTANVTAEFKETRRRLSVEI--
TASK      290 AAGGGGFRNVYAEVLHFQSMCSCLWYKSREKLQYSIPM

TWIK-1    313 ---EDQKQNEPFVATQSSACVDGPANH-----------
TREK-1    337 ---YDKFQRATSVKRKLSAELAGNHNQELTPCMRTCL-
TASK      328 IIPRDLSTSDTCVEQSHSSPGGGGRYSDTPSRRCLCSG

TWIK-1    337 ----------------------------
TREK-1    371 ----------------------------
TASK      366 APRSAISSVSTGLHSLSTFRGLMKRRSSV
```

Fig.9 b

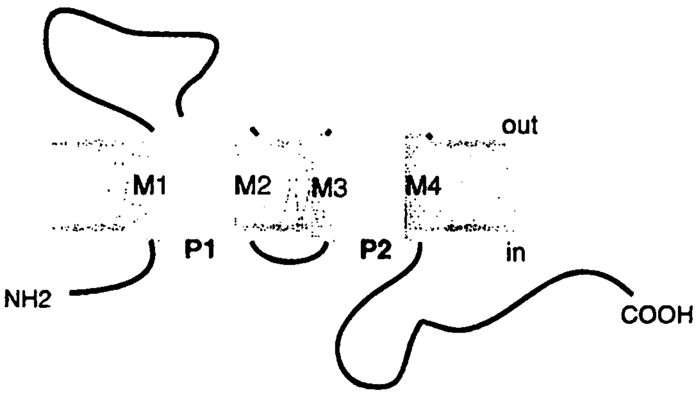

Fig.10

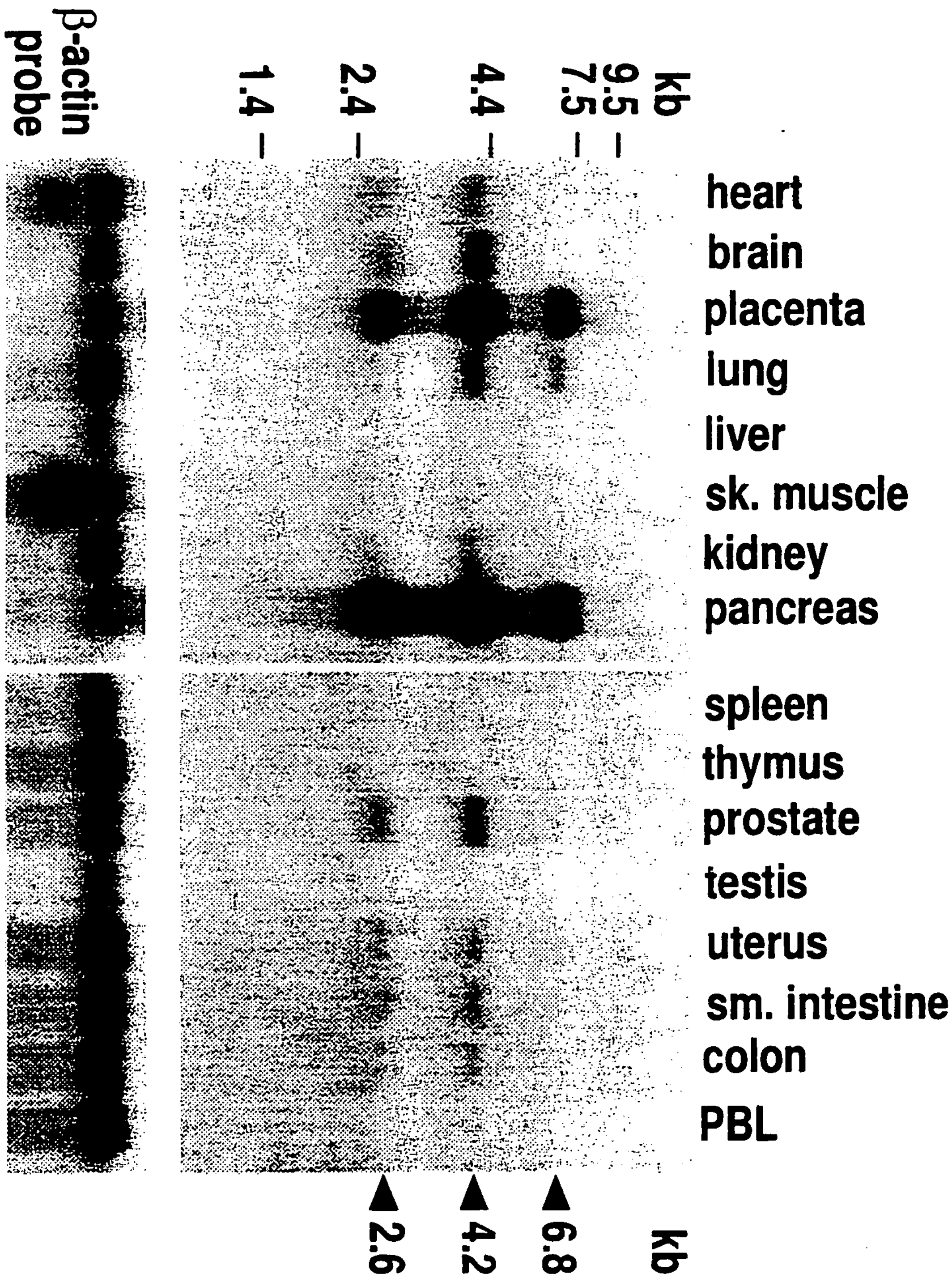
β-actin probe
1.4
2.4
4.4
7.5
9.5
kb
heart
brain
placenta
lung
liver
sk. muscle
kidney
pancreas
spleen
thymus
prostate
testis
uterus
sm. intestine
colon
PBL
2.6
4.2
6.8
kb

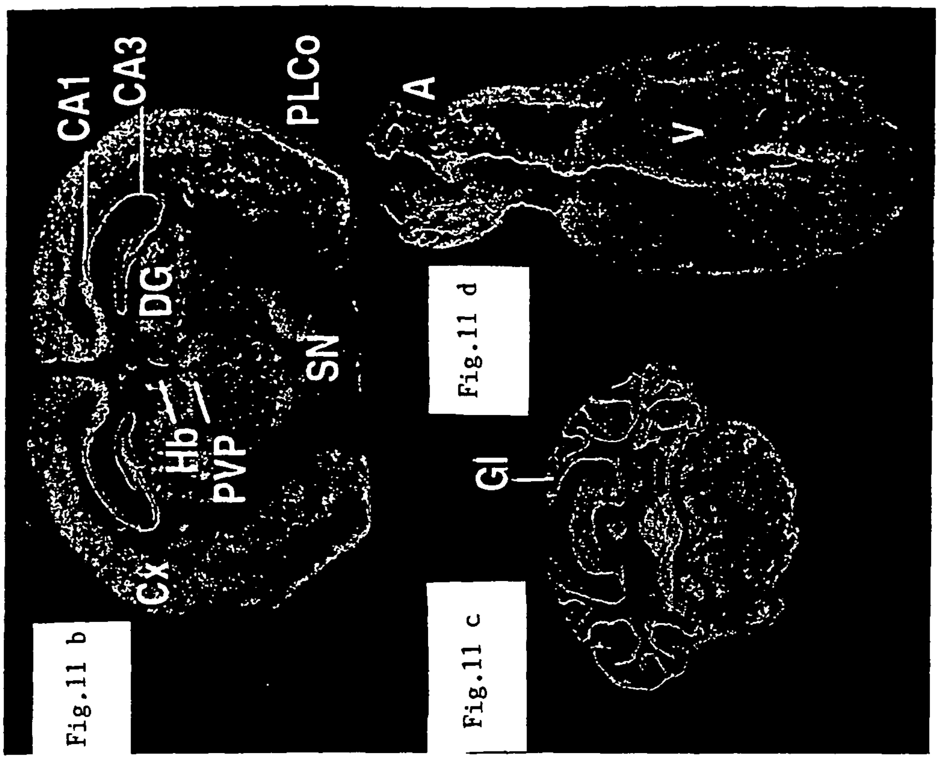

Fig.11 b

Fig.11 c

Fig.11 d

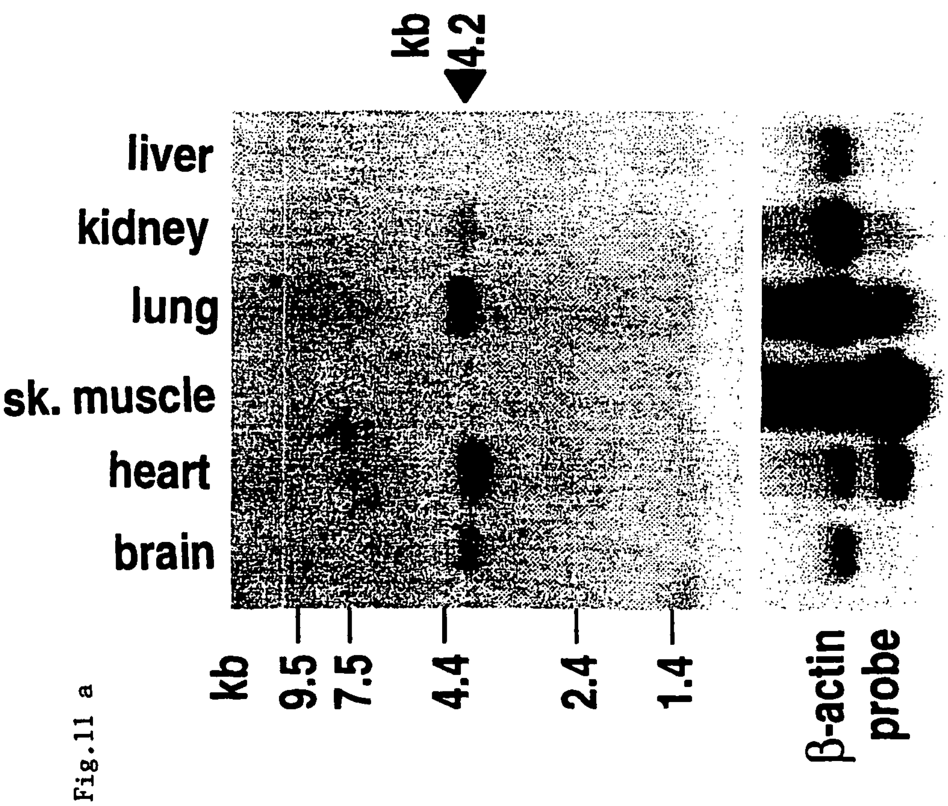

Fig.11 a

Fig.12 a

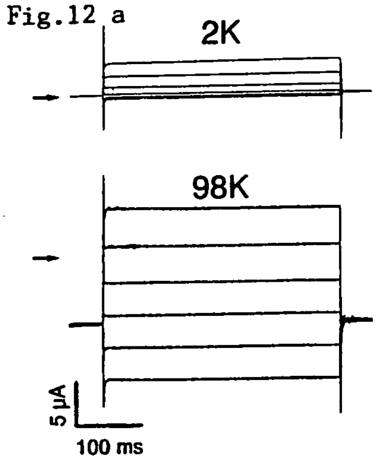


Fig.12 b

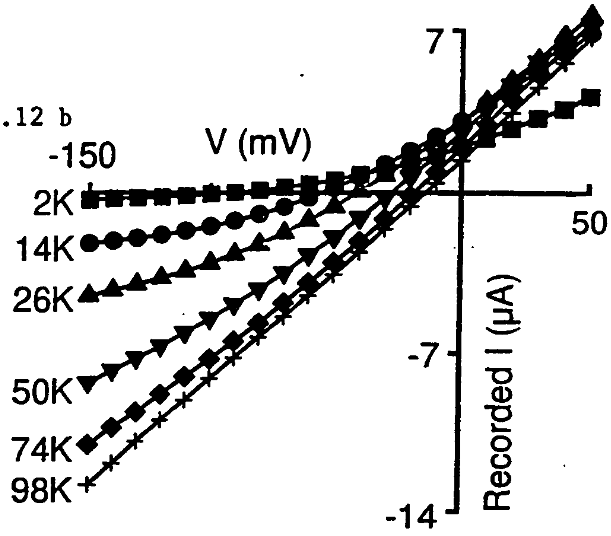


Fig.12 c

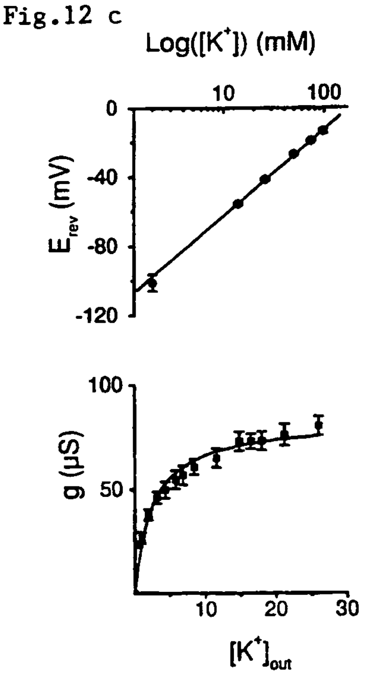


Fig.12 d

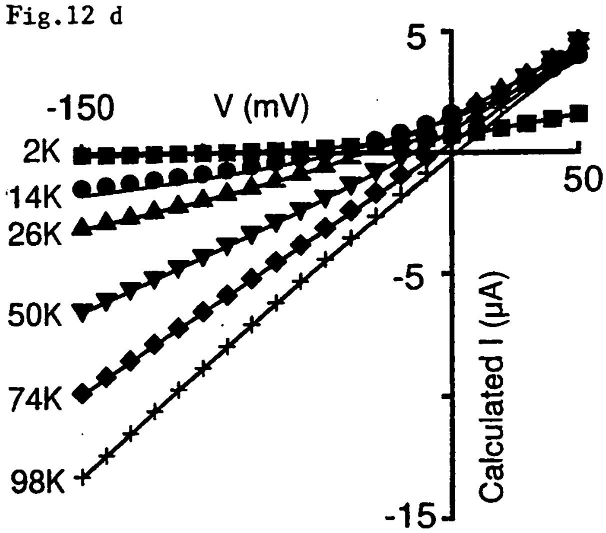

Fig.12 e

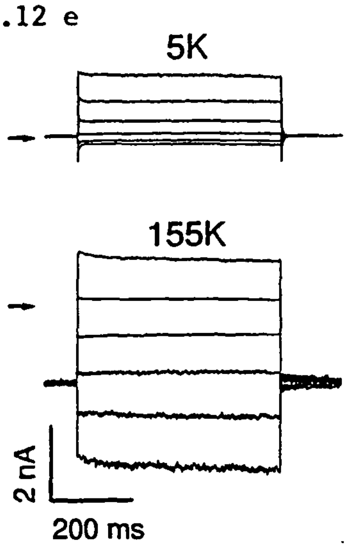


Fig.12 f

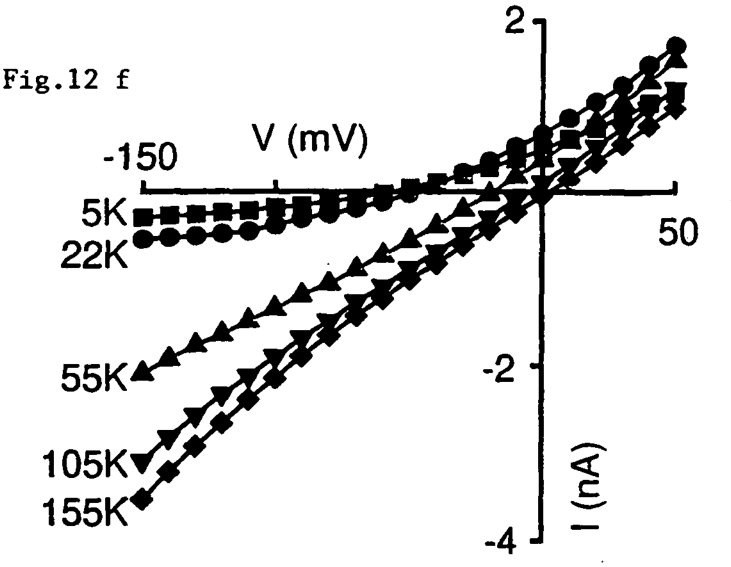

Fig.13 a

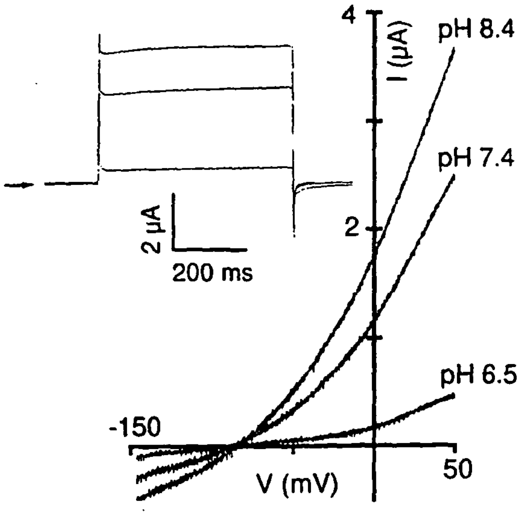


Fig.13 b

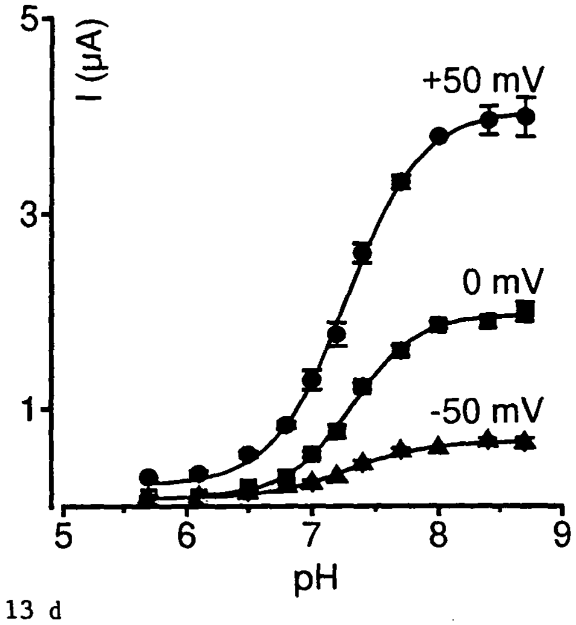


Fig.13 c

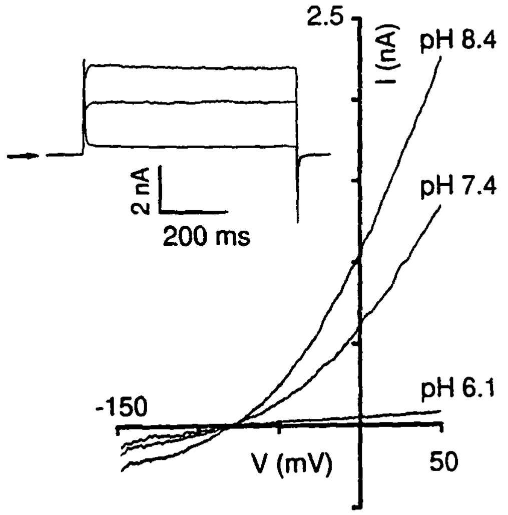


Fig.13 d

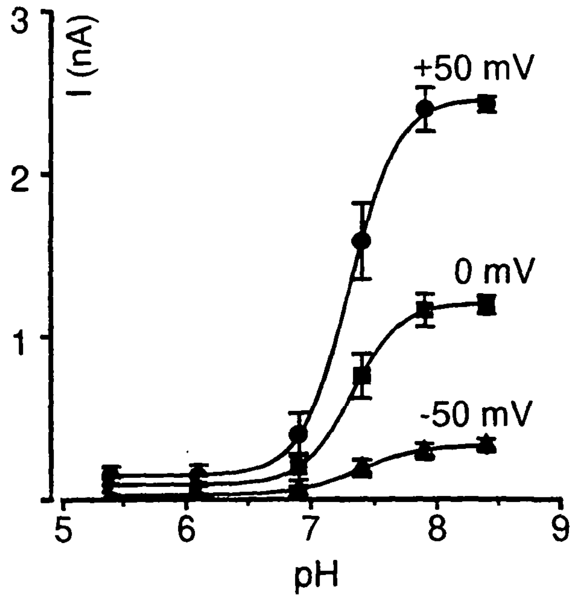

Fig.14 a

```
1    agcgacgcgtggagaagcggcccacgtgtctgcccagagtcaagtcctgtg
52   ttcttcccgctccttacgcatccgcggtccagggcgccctttcagccccgc
103  tggtgttcgcccaccccggccgcgtgagtggggccccacgcagctccccg
154  cactccgtgggccaacttggccaagcaactctgtccggggagcggtgcttg
205  cggggggtgagtaccgggcactgcgcatgcggagctccaaattcaaacagc
256  tgttttcagaggctggagggcgggcggactggtagcagctggggctaggag

307  aggctttctctaggaggcggccgctcgagagccATGGTGGACCGGGGCCCT
1                                     M  V  D  R  G  P

358  CTGCTCACCTCGGCCATCATCTTCTACCTGGCCATCGGGGCGGCGATCTTC
7     L  L  T  S  A  I  I  F  Y  L  A  I  G  A  A  I  F

409  GAAGTGCTGGAGGAGCCACACTGGAAGGAGGCCAAGAAAAACTACTACACA
24    E  V  L  E  E  P  H  W  K  E  A  K  K  N  Y  Y  T

460  CAGAAGCTGCATCTGCTCAAGGAGTTCCCGTGCCTGGGTCAGGAGGGCCTG
41    Q  K  L  H  L  L  K  E  F  P  C  L  G  Q  E  G  L

511  GACAAGATCCTAGAGGTGGTATCTGATGCTGCAGGACAGGGTGTGGCCATC
58    D  K  I  L  E  V  V  S  D  A  A  G  Q  G  V  A  I

562  ACAGGGAACCAGACCTTCAACAACTGGAACTGGCCCAATGCAATGATTTTT
75    T  G  N  Q  T  F  N  N  W  N  W  P  N  A  M  I  F
            *

613  GCAGCGACCGTCATTACCACCATTGGATATGGCAATGTGGCTCCCAAGACC
92    A  A  T  V  I  T  T  I  G  Y  G  N  V  A  P  K  T

664  CCCGCCGGTCGCCTCTTCTGTGTTTTCTATGGTCTCTTCGGGGTGCCGCTC
109   P  A  G  R  L  F  C  V  F  Y  G  L  F  G  V  P  L

715  TGCCTGACGTGGATCAGTGCCCTGGGCAAGTTCTTCGGGGGACGTGCCAAG
126   C  L  T  W  I  S  A  L  G  K  F  F  G  G  R  A  K

766  AGACTAGGGCAGTTCCTTACCAAGAGAGGTGTGAGTCTGCGGAAGGCGCAG
143   R  L  G  Q  F  L  T  K  R  G  V  S  L  R  K  A  Q

817  ATCACGTGCACAGTCATCTTCATCGTGTGGGGCGTCCTAGTCCACCTGGTG
160   I  T  C  T  V  I  F  I  V  W  G  V  L  V  H  L  V

868  ATCCCACCCTTCGTATTCATGGTGACTGAGGGGTGGAACTACATCGAGGGC
177   I  P  P  F  V  F  M  V  T  E  G  W  N  Y  I  E  G

919  CTCTACTACTCCTTCATCACCATCTCCACCATCGGCTTCGGTGACTTTGTG
194   L  Y  Y  S  F  I  T  I  S  T  I  G  F  G  D  F  V

970  GCCGGTGTGAACCCCAGCGCCAACTACCACGCCCTGTACCGCTACTTCGTG
211   A  G  V  N  P  S  A  N  Y  H  A  L  Y  R  Y  F  V

1021 GAGCTCTGGATCTACTTGGGGCTGGCCTGGCTGTCCCTTTTTGTCAACTGG
228   E  L  W  I  Y  L  G  L  A  W  L  S  L  F  V  N  W

1072 AAGGTGAGCATGTTTGTGGAAGTCCACAAAGCCATTAAGAAGCGGCGGCGG
245   K  V  S  M  F  V  E  V  H  K  A  I  K  K  R  R  R

1123 CGACGGAAGGAGTCCTTTGAGAGCTCCCCACACTCCCGGAAGGCCCTGCAG
262   R  R  K  E  S  F  E  S  S  P  H  S  R  K  A  L  Q

1174 GTGAAGGGGAGCACAGCCTCCAAGGACGTCAACATCTTCAGCTTTCTTTCC
279   V  K  G  S  T  A  S  K  D  V  N  I  F  S  F  L  S

1225 AAGAAGGAAGAGACCTACAACGACCTCATCAAGCAGATCGGGAAGAAGGCC
296   K  K  E  E  T  Y  N  D  L  I  K  Q  I  G  K  K  A

1276 ATGAAGACAAGCGGGGGTGGGGAGACGGGCCCGGGCCCAGGGCTGGGGCCT
313   M  K  T  S  G  G  G  E  T  G  P  G  P  G  L  G  P
```

Fig.14 a Continued

```
1327 CAAGGCGGTGGGCTCCCAGCACTGCCCCCTTCCCTGGTGCCCCTGGTAGTC
 330  Q  G  G  G  L  P  A  L  P  P  S  L  V  P  L  V  V

1378 TACTCCAAGAACCGGGTGCCCACCTTGGAAGAGGTGTCACAGACACTGAGG
 347  Y  S  K  N  R  V  P  T  L  E  E  V  S  Q  T  L  R

1429 AGCAAAGGCCACGTATCAAGGTCCCCAGATGAGGAGGCTGTGGCACGGGCC
 364  S  K  G  H  V  S  R  S  P  D  E  E  A  V  A  R  A

1480 CCTGAAGACAGCTCCCCTGCCCCCGAGGTGTTCATGAACCAGCTGGACCGC
 381  P  E  D  S  S  P  A  P  E  V  F  M  N  Q  L  D  R

1531 ATCAGCGAGGAATGCGAGCCATGGGACGCCCAGGACTACCACCCACTCATC
 398  I  S  E  E  C  E  P  W  D  A  Q  D  Y  H  P  L  I

1582 TTCCAGGACGCCAGCATCACCTTCGTGAACACGGAGGCTGGCCTCTCAGAC
 415  F  Q  D  A  S  I  T  F  V  N  T  E  A  G  L  S  D

1633 GAGGAGACCTCCAAGTCCTCGCTAGAGGACAACTTGGCAGGGGAGGAGAGC
 432  E  E  T  S  K  S  S  L  E  D  N  L  A  G  E  E  S

1684 CCCCAGCAGGGGGCTGAAGCCAAGGCGCCCCTGAACATGGGCGAGTTCCCC
 449  P  Q  Q  G  A  E  A  K  A  P  L  N  M  G  E  F  P

1735 TCCTCCTCCGAGTCCACCTTCACCAGCACTGAGTCTGAGCTCTCTGTGCCT
 466  S  S  S  E  S  T  F  T  S  T  E  S  E  L  S  V  P

1786 TACGAACAGCTGATGAATGAGTACAACAAGGCTAACAGCCCCAAGGGCACA
 483  Y  E  Q  L  M  N  E  Y  N  K  A  N  S  P  K  G  T

1837 TGAggcagggccggctccccaccccacctttgatggcctcttccccctca
 500  *

1888 ccctagggtgtcccgagatgaccgggacgcctggcccctggtgggggggc
1939 agcctcggaactgggagtgggggccaggggccttcctaaccttccatcat
1990 cctcagctagatgtatgcccgggacagggcctctgttctccagctgaacca
2041 taccctggctgtgggggcatctgtcctgagcttggctggtgtatctcacaa
2092 tgcaaagacatgctggctggcgggacaggtgggcaggactgaccctgagga
2143 ggccttgcctgcagggtctttgtctcaccatttggtggagtatcacacggt
2194 tctctgaggtctggggcctcagctgtttaagtttaccggtattactgagct
2245 cggcatttggagagggagctctgaagtgtctggggaggtaccgctgtgcgt
2296 ggggtcaggtgtttccgtaccacagcaggagcagggcccgcccgcatccca
2347 gctgtgggcctgccggtcaggtcgggcacctactacaaaccgtagtggggt
2398 ggaggctgctggaggtgggagtgaggagatgagggcagggtctcaaacagt
2449 cctgactcacagggcctggaaacaagtcctatgtgggcctggggcctgggg
2500 tcctcatcctccttgttggtctactcaggcccagcccagagctgtgttccc
2551 tgtctcaggtcaagcagtggcagacgcaaggctttctgtgggcccccaagt
2602 ggtaggagggagagtagcagagcatgggttactggaagccgggactgctag
2653 ggctggtggccagggagctgcaagagtgaggctcagctctggctggttctg
2704 cccttacccctcctgcccgcctgagaactgcacaccctgcccgctggcccc
2755 aggacctgcactcccaatcctgctgtcttctccttccctgtgccctgaaca
2806 aggacctcactgcccgccttcccctcccaccagcccccctggggctggccca
2857 ctgtgtcctgaatgtttttgttatttttttgttttatttttttaaacaaactg
2908 ctgtttttatatacctggaatctgttgttggcttcagagccagtggttaaa
2959 gagcagggtcccaaggattgggagatctagtgtctgccctcctgccctgca
3010 actcaattgggcctttttcggtgacctcatccaaggccatgatgtcaaggg
3061 ccatgtccccaagcagaggtggagaaggggacactgaggtgagcaaaagca
3112 ggaaggggcatccactgcgggtgactggaggccgggcaggaagcaagtcat
3163 cagagccgctcagctccgttcactctctgccttctgccccactactgtggg
3214 gcagtggggccagagcccacctccccaacatgtgaagacagtgatgggcac
3265 gtgcccacacccccacttctctagccgtttgcagaggccgccacccagcac
3316 gggcctgaaaaggagcagcctcgtatttttctgtgaaatgttttaatgaac
3367 catgttgttgctggttgtcctggcatcccgcacactgtatgtacatactgg
3418 caacgatgtcaaatgtaatttattttaacatttttacaataaaacatgagg
3469 tggacaggcaaaaaaaaaaaaaaaaaaaaaaaaaaaaaaaaaaaaa
```

Fig.14 b

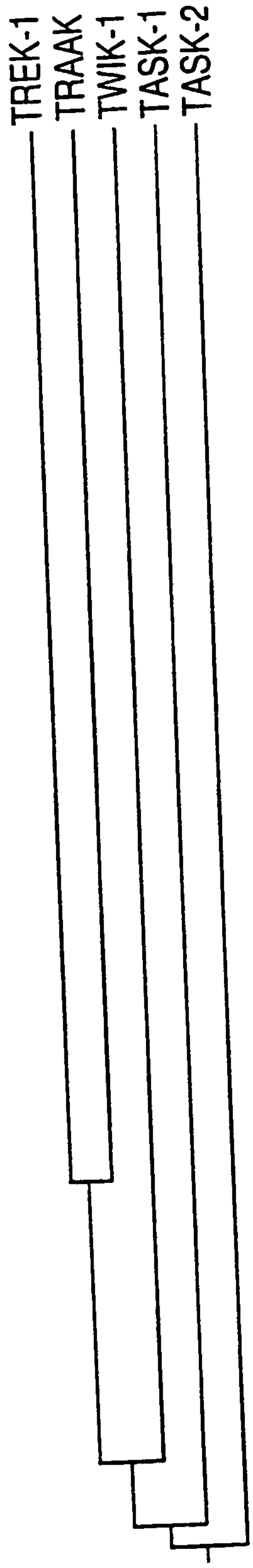

Fig.15 a

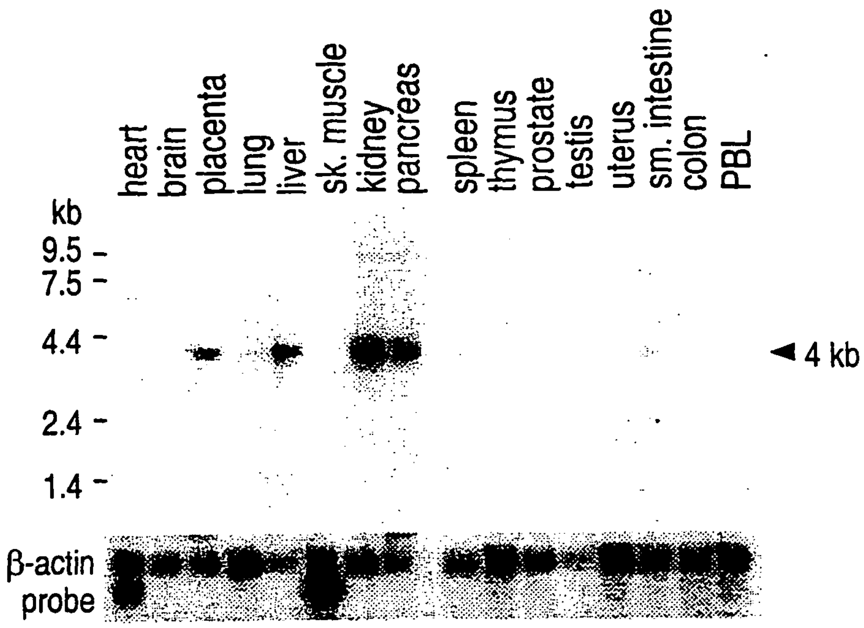


Fig.15 b

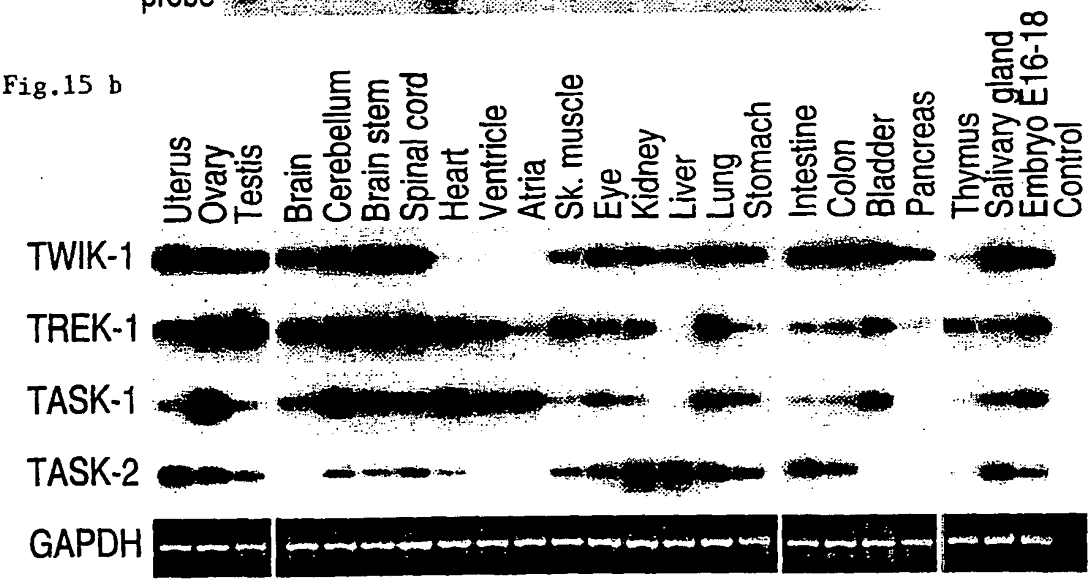

Fig.16

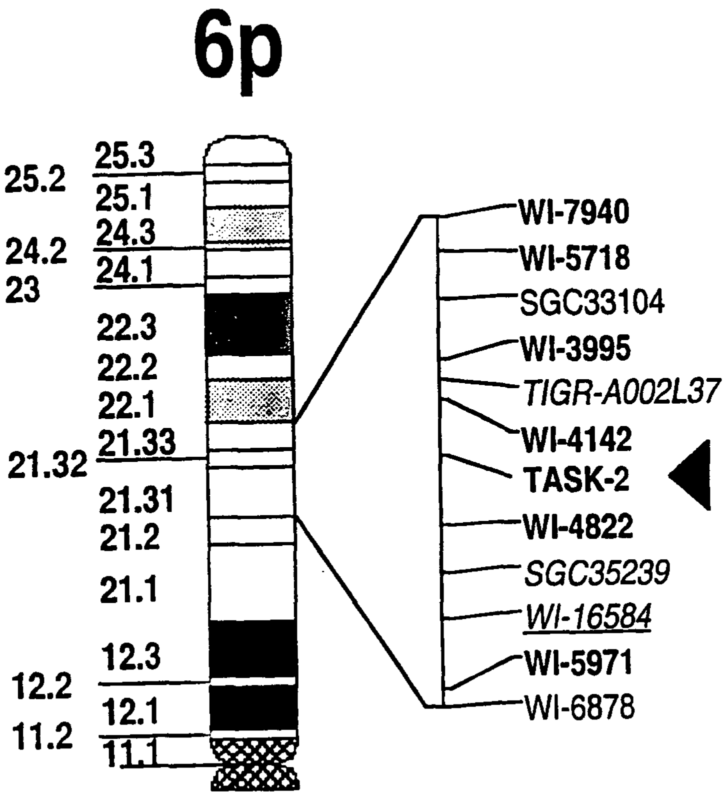
6p
25.2
25.3
25.1
24.3
24.2
24.1
23
22.3
22.2
22.1
21.33
21.32
21.31
21.2
21.1
12.3
12.2
12.1
11.2
11.1
WI-7940
WI-5718
SGC33104
WI-3995
TIGR-A002L37
WI-4142
TASK-2
WI-4822
SGC35239
WI-16584
WI-5971
WI-6878

Fig.17 a

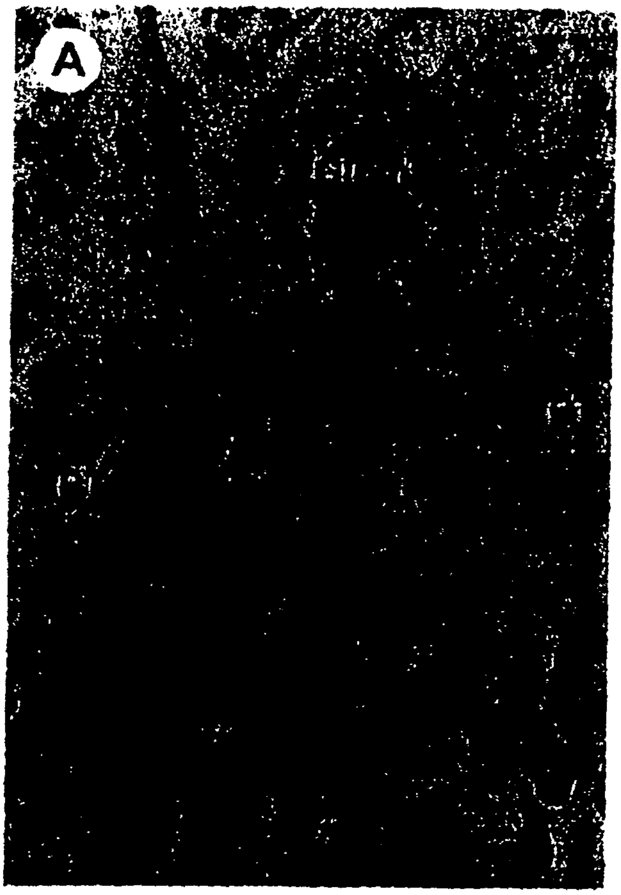


Fig.17 b

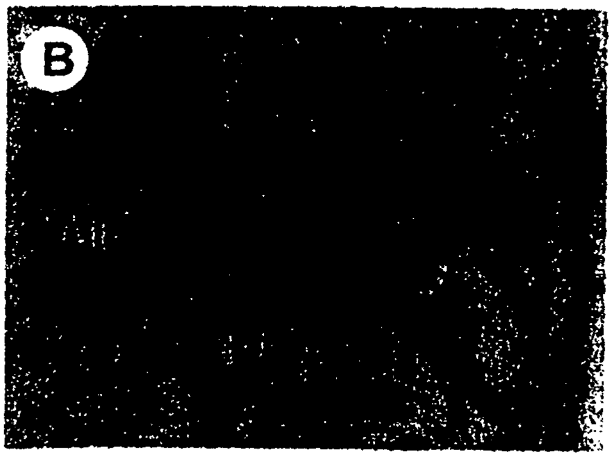

Fig.18 a

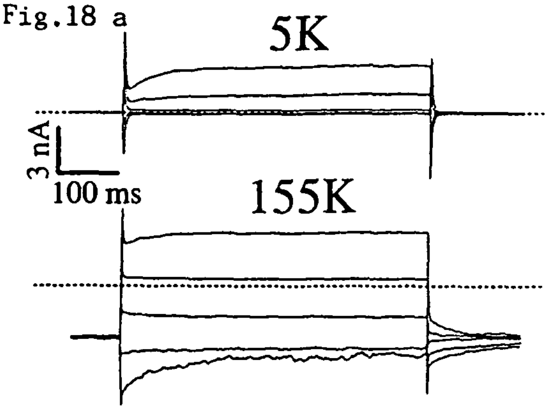


Fig.18 b

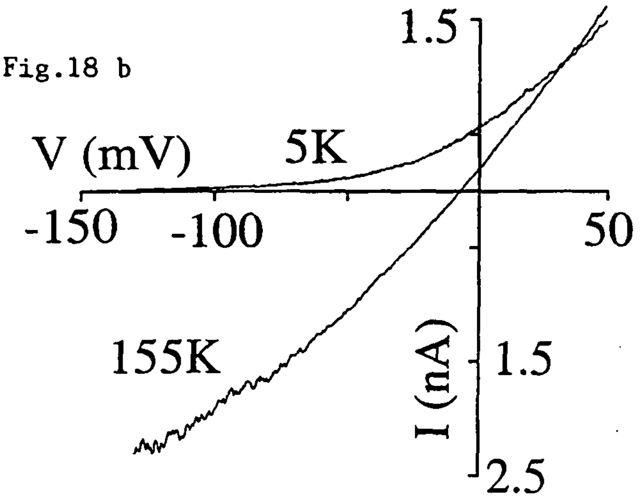


Fig.18 c

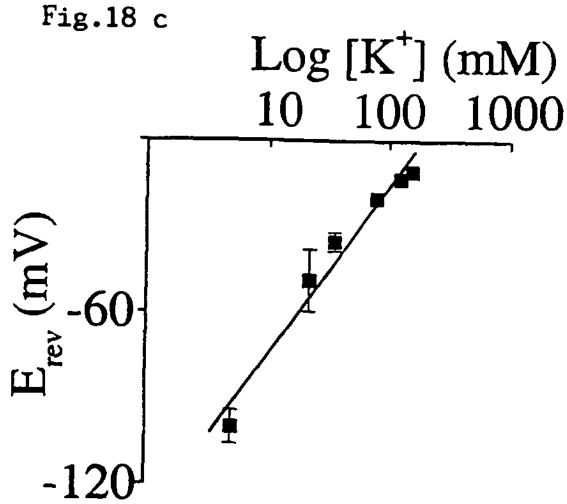

Fig.18 d

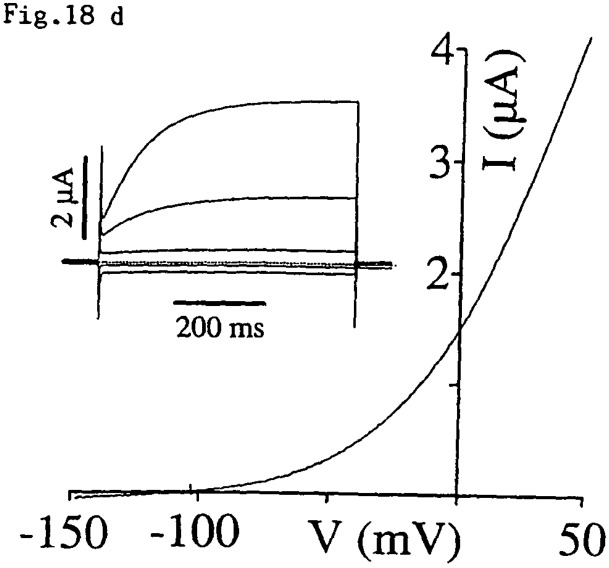


Fig.18 e

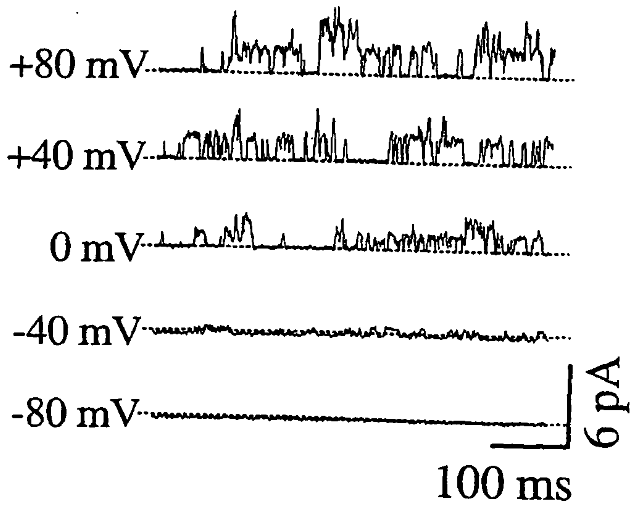


Fig.18 f

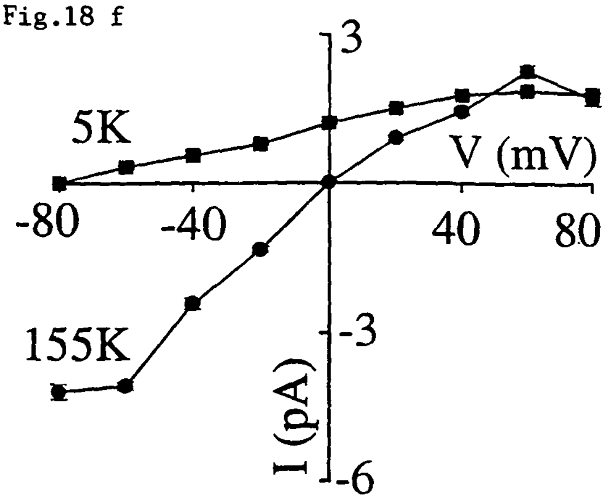

Fig.19 a

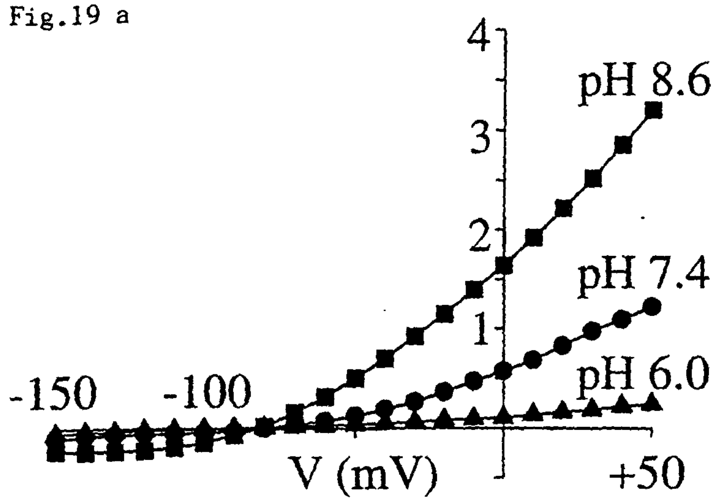


Fig.19 b

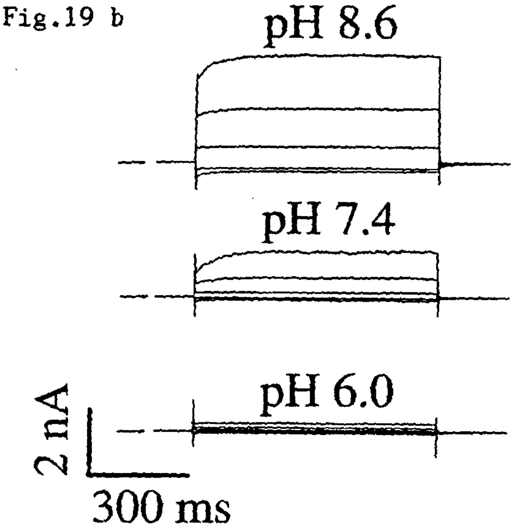


Fig.19 c

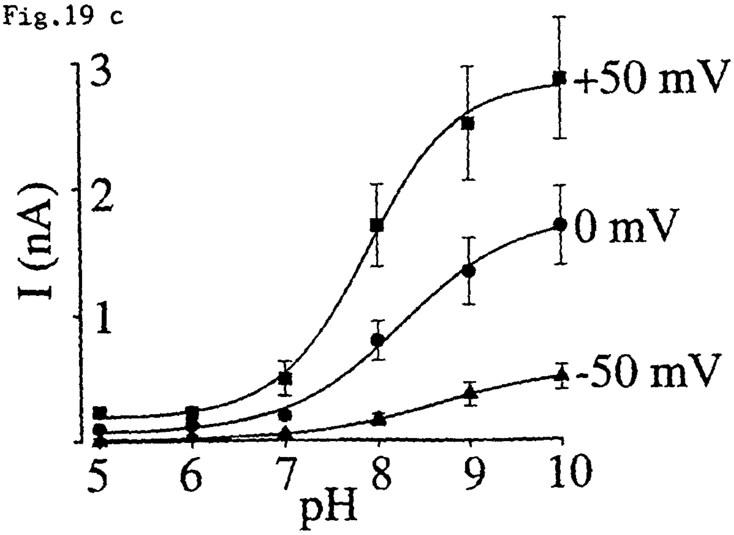

Fig. 19 d

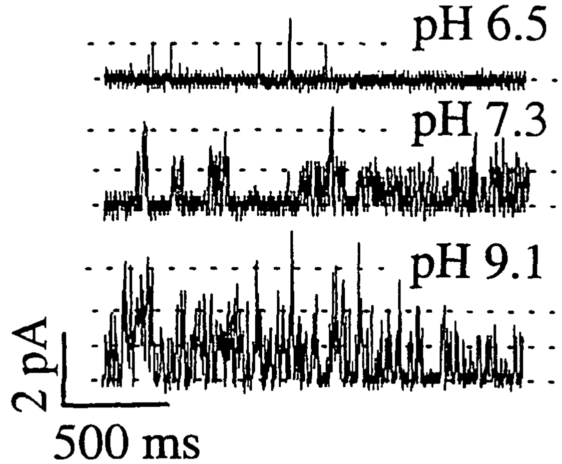


Fig.19 e

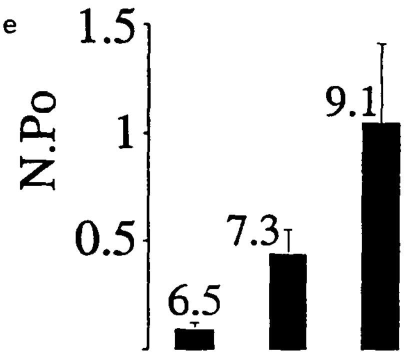


Fig.19 f

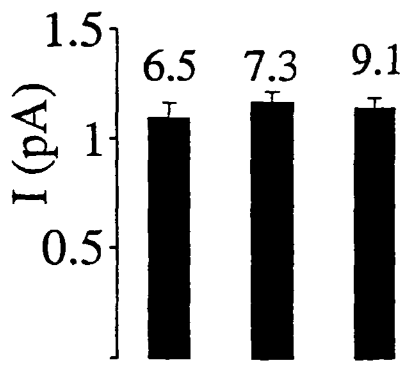

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 08749816 B
- US 09523498 P
- US 09144914 B
- US 60107692 B
- WO 0005367 A
- WO 09144914 A
- WO 08749816 A
- WO 60095234 A
- FR 9601565


### Non-patent literature cited in the description


- **RUDY, B.** *Neurosciences,* 1988, vol. 25, 729-749
- **HILLE, B.** Ionic Channels of Excitable Membrane. 1992
- **BARREL, B. A. et al.** *Annul Rev. Neurosci.,* 1990, vol. 13, 441-474
- **BETZ, H.** *Biochemistry,* 1990, vol. 29, 3591-3599
- **PONGS, 0.** *Physiol. Rev.,* 1992, vol. 72, S69 88
- **SALLCOFF, L. et al.** *Trends Neurosci.,* 1992, vol. 15, 161-166
- **JAN. L. Y. ; Y. N. JAN.** *Nature,* 1994, vol. 371, 199-122
- **DOUPNIK. C. A. et al.** *Curr. Opin. Neurobiol.,* 1995, vol. 5, 268-277
- **ALDRICH, R. W.** *Curr. Biol.,* 1994, vol. 4, 839-840
- **ISOM, L. L. et al.** *Neuron,* 1994, vol. 12, 1183-1194
- **RETTIG, J. et al.** *Nature,* 1994, vol. 369, 289-294
- **ATTALI, B.** *Proc. Nafl. Acad. Sci. USA,* 1995, vol. 92, 6092-6096
- **LOGOTHETIS, D. E. et al.** *Neuron,* 1992, vol. 8, 531-540
- **BEZANILLA, F. ; STEFANI, E.** *Annul Rev. Biophys. Biomol. Struct.,* 1994, vol. 23, 819-846
- **MATSUDA, H.** *Annul Rev. Physiol.,* 1991, vol. 53, 289-298
- **LU, Z. ; MACKINNON, R.** *Nature,* 1994, vol. 371, 243 246
- **NICHOLS, C. G. et al.** *J. Physiol. London,* 1994, vol. 476, 399-409
- **PONGS, O.** *J.Membrane Biol.,* 1993, vol. 136, 1-8
- **HEGINBOTHAM, L. et al.** *Biophys. J.,* 1994, vol. 66, 1061-1067
- **MACKINNON, R.** *Neuron,* 1995, vol. 14, 889-892
- **PASCUAL, J. M. et al.** *Neuron,* 1995, vol. 14, 1055-1063
- **DUPRAT, F et al.** TASK, a human background K+ channel to sense external pH variation near physiological pH. *EMBO JOURNAL,* 1997, vol. 16/17, 5464-5471
- TWIK-1, a ubiquitous human weakly inward rectifying K+ channel with a novel structure. **LESAGE F et al.** EMBO JOURNAL. OXFORD UNIVERSITY PRESS, 1996, vol. 15, 1004-1011
- Cloning, functional expression and brain localization of a novel unconventional outward rectifier K+ channel. **FINK M et al.** EMBO JOURNAL. OXFORD UNIVERSITY PRESS, 16 December 1996, vol. 15, 6854-6862
- **ALTSCHUL, S. F. et al.** *J. Mol. Biol.,* 1990, vol. 215, 403-410
- **GUILLEMARE, E. et al.** *Biochemistry,* 1992, vol. 31, 1246312468
- **KETCHUM, K. A. et al.** *Nature,* 1995, vol. 376, 690-695
- **LINGUEGLIA, E. et al.** *J. Biol. Chem.,* 1993, vol. 269, 13736-13739
- **DUPRAT, F. et al.** *Biochem. Biophys. Res. Commun.,* 1995, vol. 212, 657-663
- **BEAR, C. E. et al.** *Biochim. Biophys. Acta,* 1988, vol. 944, 113-120
- **GUILLEMARE, E. et al.** *Mol. Pharmacol.,* 1995, vol. 47, 588-594
- **PEDERSEN, P. L. ; CARAFOLI, E.** *Trends Biol. Sci.,* 1997, vol. 12, 146-189
- **LEVITAN, I. B.** *Annul Rev. Physiol.,* 1994, vol. 56, 193-212